# FASCICULE DE BREVET EUROPEEN

(11) **EP 2 879 698 B1**
(45) Date de publication et mention de la délivrance du brevet: **17.01.2018**
(21) Numéro de dépôt: 13826491.6
(22) Date de dépôt: 02.08.2013
(51) Int. Cl.: A61K 39/012, A61K 39/39

(54) **UTILISATION DE SOUCHES ATTENUEES DE TOXOPLASMA POUR LA PREVENTION OU LE TRAITEMENT DE LA CRYPTOSPORIDIOSE**
VERWENDUNG VON ATTENUIERTEN STÄMMEN VON TOXOPLASMA ZUR PRÄVENTION ODER BEHANDLUNG VON CRYPTOSPORIDIOSE
USE OF ATTENUATED STRAINS OF TOXOPLASMA FOR THE PREVENTION OR TREATMENT OF CRYPTOSPORIDIOSIS

(30) Priorité: 02.08.2012 FR 1257547
(43) Date de publication de la demande: 10.06.2015
(73) Titulaire: Vitamfero, 37200 Tours (FR); Institut National de la Recherche Agronomique (INRA), 75338 Paris Cedex 07 (FR); Université François Rabelais de Tours, 37041 Tours Cedex 1 (FR)
(72) Inventeur: GNAHOUI-DAVID,, Audrey, F-37041 Tours Cedex 01 (FR); LAURENT, Fabrice, 31 avenue Monge F-37200 Tours (FR); MEVELEC, Marie-Noëlle, F-37200 Tours (FR); SECHE, Edouard, F-37200 Tours (FR)
(74) Mandataire: Grosset-Fournier, Chantal Catherine
(86) Numéro de dépôt international: PCT/FR2013/051876
(87) Numéro de publication internationale: WO 2014/020290

(56) Documents cités:
- WO-A2-2011/011725
- PENARETE-VARGAS DIANA MARCELA ET AL: "Protection against Lethal Neospora caninum Infection in Mice Induced by Heterologous Vaccination with a mic1 mic3 Knockout Toxoplasma gondii Strain", INFECTION AND IMMUNITY, vol. 78, no. 2, février 2010 (2010-02), pages 651-660, XP002696799,

### SEQUENCE LISTING

<110> VitamFero
   Institut National de la Recherche Agronomique
   Université François-Rabelais de Tours
<120> UTILISATION DE SOUCHES ATTENUEES DE PARASITES POUR LA PREVENTION OU LE TRAITEMENT DE PATHOLOGIES ASSOCIEES A UN APICOMPLEXE
<130> WOB 11 DE VIT CRYP
<160> 38
<170> PatentIn version 3.5
<210> 1
   <211> 34
   <212> DNA
   <213> Artificial Sequence
<220>
   <223> 5 HR NCmic3 F KpnI
<400> 1
   cgcggtaccc atgtgaatat gctttaaccg tgac 34
<210> 2
   <211> 34
   <212> DNA
   <213> Artificial Sequence
<220>
   <223> 5 HR NCmic3 R ClaI
<400> 2
   cgcatcgatg agctataacc cttggaaatg actc 34
<210> 3
   <211> 31
   <212> DNA
   <213> Artificial Sequence
<220>
   <223> 3 HR NCmic3 F XbaI
<400> 3
   cgctctagac atgctgatga agaagggaag t 31
<210> 4
   <211> 34
   <212> DNA
   <213> Artificial Sequence
<220>
   <223> 3 HR NCmic3 R NotI
<400> 4
   cgcgcggccg ctctctcctg aagtcttcga gacc 34
<210> 5
   <211> 25
   <212> DNA
   <213> Artificial Sequence
<220>
   <223> HR NCmic3 F
<400> 5
   gtcatcgacc gccggaacta gtagt 25
<210> 6
   <211> 26
   <212> DNA
   <213> Artificial Sequence
<220>
   <223> HR NCmic3 R
<400> 6
   gcagaggttc tgcgtatcta acacgg 26
<210> 7
   <211> 21
   <212> DNA
   <213> Artificial Sequence
<220>
   <223> ORF NCmic3 F
<400> 7
   tttcccttct aaacacagtc g 21
<210> 8
   <211> 22
   <212> DNA
   <213> Artificial Sequence
<220>
   <223> ORF NCmic3 R
<400> 8
   ccttcagtgg ttctccatga gt 22
<210> 9
   <211> 20
   <212> DNA
   <213> Artificial Sequence
<220>
   <223> ORF DHFR F
<400> 9
   ccttctcaga caacggggta 20
<210> 10
   <211> 20
   <212> DNA
   <213> Artificial Sequence
<220>
   <223> ORF DHFR R
<400> 10
   agatcttcac gcccttctca 20
<210> 11
   <211> 24
   <212> DNA
   <213> Artificial Sequence
<220>
   <223> Integ NCmic3 F
<400> 11
   gaaagtgtca gtggtagaga ctgc 24
<210> 12
   <211> 27
   <212> DNA
   <213> Artificial Sequence
<220>
   <223> ORF NCmic3 R2
<400> 12
   ccttcactcg agatcgcgca aatgagc 27
<210> 13
   <211> 23
   <212> DNA
   <213> Artificial Sequence
<220>
   <223> ORF DHFR R2
<400> 13
   ggacctctgt acgagacatg ccg 23
<210> 14
   <211> 24
   <212> DNA
   <213> Artificial Sequence
<220>
   <223> Integ NCmic3 R
<400> 14
   tgtttacagg tgatccagaa aagg 24
<210> 15
   <211> 20
   <212> DNA
   <213> Artificial Sequence
<220>
   <223> ORF NCmic3 F2
<400> 15
   gaattttggg acaggggaat 20
<210> 16
   <211> 25
   <212> DNA
   <213> Artificial Sequence
<220>
   <223> ORF DHFR F2
<400> 16
   gtctctcgtt ttcctctctt ttcgg 25
<210> 17
   <211> 33
   <212> DNA
   <213> Artificial Sequence
<220>
   <223> 3 HR NCmic1 F KpnI
<400> 17
   cgcggtacca ggcagaagta aagaaggttc ctc 33
<210> 18
   <211> 30
   <212> DNA
   <213> Artificial Sequence
<220>
   <223> 3 HR NCmic1 R HindIII
<400> 18
   cgcaagcttt gatcacgcaa gaaaagaagc 30
<210> 19
   <211> 32
   <212> DNA
   <213> Artificial Sequence
<220>
   <223> 5 HR NCmic1 F BamHI
<400> 19
   cgcggatccc atttgtagat acggttgcac ac 32
<210> 20
   <211> 33
   <212> DNA
   <213> Artificial Sequence
<220>
   <223> 5 HR NCmic1 R NotI
<400> 20
   cgcgcggccg cacattcaga cggcagaact ctg 33
<210> 21
   <211> 20
   <212> DNA
   <213> Artificial Sequence
<220>
   <223> Integ NCmic1 F
<400> 21
   ccgagcaagt tagcaagtcc 20
<210> 22
   <211> 20
   <212> DNA
   <213> Artificial Sequence
<220>
   <223> ORF CATGFP R
<400> 22
   ccgtttggtg gatgtcttct 20
<210> 23
   <211> 20
   <212> DNA
   <213> Artificial Sequence
<220>
   <223> ORF CATGFP F
<400> 23
   gcatcgactt caaggaggac 20
<210> 24
   <211> 20
   <212> DNA
   <213> Artificial Sequence
<220>
   <223> Integ NCmic1 R
<400> 24
   cttgtccgtc acatcgtttg 20
<210> 25
   <211> 20
   <212> DNA
   <213> Artificial Sequence
<220>
   <223> ORF NCmic1 R
<400> 25
   ttctccaggc actcacctct 20
<210> 26
   <211> 20
   <212> DNA
   <213> Artificial Sequence
<220>
   <223> ORF NCmic1 F
<400> 26
   agcttccaac aacgagagga 20
<210> 27
   <211> 20
   <212> DNA
   <213> Artificial Sequence
<220>
   <223> ORF NCmic1 F2
<400> 27
   cccaggatat cgtttgttgc 20
<210> 28
   <211> 20
   <212> DNA
   <213> Artificial Sequence
<220>
   <223> ORF NCmic1 R2
<400> 28
   cttctgatgc acggaactga 20
<210> 29
   <211> 20
   <212> DNA
   <213> Artificial Sequence
<220>
   <223> ORF CATGFP F2
<400> 29
   cctgaagttc atctgcacca 20
<210> 30
   <211> 20
   <212> DNA
   <213> Artificial Sequence
<220>
   <223> ORFCATGFP R2
<400> 30
   gtagtggttg tcgggcagca 20
<210> 31
   <211> 21
   <212> DNA
   <213> Artificial Sequence
<220>
   <223> IL12 F
<400> 31
   ctcacatctg ctgctccaca a 21
<210> 32
   <211> 21
   <212> DNA
   <213> Artificial Sequence
<220>
   <223> IL12 R
<400> 32
   gacgccattc cacatgtcac t 21
<210> 33
   <211> 22
   <212> DNA
   <213> Artificial Sequence
<220>
   <223> IFN gamma F
<400> 33
   tcttcttgga tatctggagg aa 22
<210> 34
   <211> 23
   <212> DNA
   <213> Artificial Sequence
<220>
   <223> IFN gamma R
<400> 34
   agctcattga atgcttggcg ctg 23
<210> 35
   <211> 22
   <212> DNA
   <213> Artificial Sequence
<220>
   <223> HPRT F
<400> 35
   ggatacaggc cagactttgt tg 22
<210> 36
   <211> 20
   <212> DNA
   <213> Artificial Sequence
<220>
   <223> HPRT R
<400> 36
   gagggtaggc tggcctatag 20
<210> 37
   <211> 25
   <212> DNA
   <213> Artificial Sequence
<220>
   <223> SAG-1 F
<400> 37
   ctgcaccact tcattatttc ttctg 25
<210> 38
   <211> 19
   <212> DNA
   <213> Artificial Sequence
<220>
   <223> SAG-1 R
<400> 38
   actcacgcga cacaagctg 19

## Description

La présente invention concerne l'utilisation de souches atténuées de parasites pour la prévention ou le traitement de pathologies associées à un apicomplexe.

Les apicomplexes sont des parasites majoritairement intracellulaires obligatoires qui possèdent un cycle de vie pouvant faire intervenir plusieurs hôtes. Le phylum de ces parasites se subdivise en plusieurs familles.

*Toxoplasma gondii* (*T. gondii*) appartient à la famille des *Sarcocystidae.* Ce protozoaire existe sous trois formes infectieuses qui varient en fonction de l'hôte et du stade infectieux :
- le tachyzoïte : forme proliférative et infectieuse qui se multiplie de manière asexuée dans les cellules des hôtes intermédiaires (*i.e.* l'ensemble des homéothermes) et définitif (*i.e.* les félidés et le chat en particulier),
- le bradyzoïte : forme à division lente et à bas niveau de métabolisme du parasite contenu dans des kystes,
- le sporozoïte : forme contenue dans les oocystes, qui résulte de la multiplication sexuée du parasite dans l'intestin des hôtes définitifs (*i.e.* chat et autres félidés).
Le chat, hôte définitif du parasite, s'infecte en ingérant des proies parasitées contenant des kystes (ou des tachyzoïtes si l'animal est en phase aigue de toxoplasmose), ou par l'ingestion d'oocystes. Après une multiplication sexuée dans l'intestin des félidés, via des gamétocytes, les oocystes sont disséminés dans l'environnement. Ces oocystes sporulent dans le milieu extérieur et leur pathogénicité persiste au moins un an. Après ingestion d'oocystes, les sporozoïtes libérés infectent les entérocytes de l'hôte définitif ou intermédiaire et se transforment en tachyzoïtes qui se disséminent dans l'organisme. Sous la pression du système immunitaire, les tachyzoïtes s'enkystent avec un tropisme préférentiel pour le système nerveux central, la rétine ou les muscles. L'ingestion de tissus enkystés est la deuxième cause de contamination des hôtes définitifs et intermédiaires. Après ingestion de kystes, les bradyzoïtes sont libérés et infectent les cellules entéroépithéliales et se transforment en tachyzoïtes, qui se disséminent dans l'hôte.

Les souches de *T. gondii* sont classées en fonction de leur degré de virulence *in vivo* : les souches de type I (*i.e.* la souche RH) sont hautement virulentes alors que les souches de types II (*i.e.* les souches ME49, 76K ou Pru) et III (*i.e.* les souches CEP ou M7741) sont moins virulentes et établissent généralement des infections chroniques. Par ailleurs, de nombreuses souches atypiques non rattachables aux trois premiers types sont identifiées notamment en Afrique et en Amérique du Sud (Howe DK et al., 1995, J. Infect. Dis., 171, 1561-1566 ; Rajendran C et al., 2011, Infect. Genet. Evol., 12, 359-368 ; Mercier A et al., 2010, PLoS Negl. Trop. Dis., 4, e876).

Récemment, une souche vivante atténuée de *Toxoplasma gondii,* le parasite responsable de la toxoplasmose a été mise au point par invalidation de deux gènes codant pour les protéines TgMIC1 et TgMIC3 (EP 1 703 914 B1 et US 7, 964, 185 B2 / Cérède et al., 2005, J. Exp. Med., 201:453-63). Cette souche, dénommée Toxo *mic1-3* KO a généré une réponse immunitaire forte et spécifique contre *Toxoplasma gondii* et permet de prévenir les effets d'une infection ultérieure chez la souris (Ismaël et al., 2005, J. Infect. Dis., 194: 1176-1183) et également chez la brebis (Mevelec et al., 2010, Vet.Res., 41 : 49). Il a également été démontré que la virulence *in vivo* n'est que très peu affectée par l'inactivation isolée de TgMIC1 ou de TgMIC3 ; en revanche, elle est fortement diminuée par l'inactivation simultanée des deux protéines, mettant en évidence le rôle synergique des deux protéines (Cérède et al., 2005 J. Exp. Med., 201:453-63).

*Neospora caninum* est un parasite intracellulaire, responsable de la néosporose. Il appartient également à la famille des *Sarcocystidae.* Le cycle de vie de *Neospora caninum* est très proche de celui de *T. gondii* avec deux phases distinctes : une phase sexuée chez l'hôte final (*i.e.* les canidés et le chien en particulier) qui conduit à la production d'oocystes, contenant des sporozoïtes, éliminés dans les fèces et une phase asexuée chez un hôte intermédiaire (*i.e.* les ovins, les caprins, les bovins, les équidés, etc...) qui conduit à la production de tachyzoïtes puis de kystes contenant les bradyzoïtes.

Plus récemment, il a été obtenu une souche vivante atténuée de *Neospora caninum,* la souche Neo *ncmicl-3* KO, qui a été invalidée pour les gènes *ncmic1* et *ncmic3* par recombinaison homologue. Dans cette souche, le gène *ncmic3* est remplacé par une cassette DHFR qui permet la résistance à la pyriméthamine et le gène *ncmic1* est remplacé par une cassette CAT-GFP qui confère au parasite la résistance au chloramphénicol et rend le parasite fluorescent. Le parasite n'exprime plus les protéines NcMIC1 et NcMIC3. Il a été montré que cette souche mutante possède des propriétés infectieuses et immunogènes conférant aux mammifères une protection vaccinale vis-à-vis des effets délétères de la néosporose.

*Toxoplasma gondii* et *Neospora caninum* ont en commun un processus spécifique d'invasion des cellules hôtes en plusieurs étapes conduisant à la formation d'une vacuole parasitophore dans laquelle le parasite se multiplie et se développe.

Les *Cryptosporidiidae* constituent une autre famille appartenant au phylum des apicomplexes et sont responsables de la cryptosporidiose, maladie extrêmement fréquente qui affecte notamment l'Homme et de nombreuses espèces animales dont des animaux de rente (ovin, caprin, bovin, etc...) par ingestion de parasites présents dans leur alimentation. Le parasite se multiplie alors dans les intestins, dans un premier temps selon un mode asexué puis, dans un second temps, selon un mode sexué. Les individus contaminés excrètent et disséminent de nouveaux parasites contaminant ainsi leur environnement (*i.e.* pâturages, eau, etc).

Plusieurs espèces de *Cryptosporidiidae* ont été identifiées parmi lesquelles *Cryptosporidium parvum* est l'un des plus fréquents et des plus virulents.

Chez l'Homme, la cryptosporidiose est une maladie généralement bénigne. Cependant, les conséquences de cette maladie dont l'incidence croît chaque année, notamment aux Etats-Unis, peuvent être extrêmement graves chez les jeunes enfants et les personnes immunodéprimées, en particulier les patients infectés par le virus VIH. Ainsi, au sein de ces populations, la cryptosporidiose est responsable de diarrhées sévères pouvant générer d'importantes déshydratations, voire, sans traitement approprié, la mort de l'individu.

Chez les animaux adultes immunocompétents, la cryptosporidiose est également bénigne. A *contrario,* la cryptosporidiose a généralement de graves conséquences chez les très jeunes animaux, âgés de quelques jours à quelques semaines et dont le système immunitaire est immature. Ainsi, *Cryptosporidum spp,* et notamment *C. parvum,* s'avère être l'un des agents étiologiques les plus fréquents des diarrhées néonatales qui génèrent des retards de croissance importants et qui, sans traitement approprié, peuvent être fatales à l'animal.

Les diarrhées néonatales constituent une menace constante pour les éleveurs de bovins, d'ovins et de caprins, et représentent une perte économique considérable due au manque à gagner consécutif au retard de croissance, à la mortalité des nouveau-nés, à l'intervention des vétérinaires et aux coûts des traitements curatifs et de réhydratation. L'étiologie des diarrhées néonatales est souvent multiple, plusieurs agents infectieux étant, en effet, responsables de ces symptômes, notamment, les rotavirus, les coronavirus, le virus BVDV (Bovine Viral Diarrhea Virus), *Escherichia coli* et, bien entendu, *C*. *parvum.* Le rôle de ces différents agents infectieux dans les diarrhées néonatales des ruminants est difficile à estimer car les diagnostics sont rarement effectués et il y a régulièrement des infections multiples. Cependant, *C. parvum* est actuellement considéré comme le facteur principal des diarrhées néonatales (de Graaf et al., 1999, Int. J. Parasitol., 29:1269-1287). Ainsi, une étude réalisée sur des veaux souffrant de diarrhées néonatales a mis en évidence que 40% d'entre eux étaient infectés par *C*. *parvum* et parmi lesquels les ¾ ne présentaient aucun autre agent infectieux (données du « Veterinary and Agrochemical Research Centre » à Bruxelles).

Pour réduire l'intensité et la durée des symptômes, de nombreuses molécules ont été évaluées dans différents modèles, mais aucune n'a donné des résultats satisfaisants. Ainsi, chez les ruminants domestiques, seul le lactate d'halofuginone (HaloCur®) et la paromycine ont donné des résultats intéressants mais ne permettent pas un contrôle total du parasite (Chartier, 2002, Le Point vétérinaire Pathologie ovine et caprine, 112-117). Chez l'homme, et en particulier pour le traitement des sidéens, le traitement usuel est basé sur l'utilisation de paromomycine (Humatin) ou de nitazoxanide. Les deux molécules ont une efficacité similaire mais la nitazoxanide est jugée moins toxique. Par ailleurs, des inhibiteurs spécifiques des protéines kinases dépendantes du calcium de *T. gondii* et de *C. parvum* ont récemment été mis au point (WO 2011/094628 A1). Il s'agit notamment de composés appartenant aux classes des pyrazolopyrimidines et des imidazo[1,5-a]pyrazines. Ces inhibiteurs affectent la capacité d'invasion et la prolifération des parasites sans perturber les activités biologiques de la cellule hôte.

En conclusion, que ce soit chez l'homme ou l'animal, il n'existe à l'heure actuelle aucun traitement spécifique permettant de lutter efficacement contre la cryptosporidiose et seule la prévention, basée sur des règles d'hygiène strictes, permet de réduire l'ingestion d'eau ou d'aliments souillés par *C. parvum.*

Dans la demande WO 2011/011725, les auteurs ont montré qu'un extrait soluble d'antigènes (appelé STAg) de tachyzoïtes de *Toxoplasma gondii* pouvait être utilisé chez des souris adultes pour le traitement d'une infection causée par *Plasmodium berghei,* parasite appartenant à la famille de Plasmodiidae, notamment responsable du paludisme.

Il existe par conséquent un réel besoin pour des agents de prévention et de traitement de la cryptosporidiose chez les mammifères, notamment les mammifères possédant un système immunitaire immature ou déficient. En effet, l'infection pouvant avoir lieu au cours des premiers jours, les nouveau-nés ont un système immunitaire en cours de développement rendant impossible un schéma de vaccination classique chez le jeune. Ainsi, des essais de prévention menés avec des parasites tués sur des veaux nouveau-nés dans une région à forte endémie n'ont démontré aucune protection significative (Harp et al., 1996, Am.J.Vet.Res., 57:1586-1588 ; Harp et al., 1998, J.Dairy.Sci. 81: 289-294).

Il a cependant été démontré que la réponse immunitaire contre *C. parvum* conduit au développement d'une réponse protectrice de type Th1 avec une production importante des cytokines IL-12 et IFN-γ. Il a également été démontré que ces cytokines sont capables de diminuer l'impact de l'infection (Lacroix et al., 2001, Infect. Immun., 69 : 1635-42).

L'un des buts de l'invention est de fournir un immunostimulant capable d'induire chez le nouveau-né une réponse immunitaire non spécifique avec production d'IL-12 et d'IFNγ.

Un autre but de l'invention est de fournir un immunostimulant capable de limiter les effets de l'infection de mammifères, humains ou animaux, par *Cryptosporidium parvum.*

Encore un autre but de l'invention est de fournir un traitement prophylactique, et notamment un vaccin, contre la cryptosporidiose. Souches de Toxoplasma gondii possédant les deux adhésines MIC-1 et MIC-3 inactivées par une modification génique portant sur les deux gènes mic-1 et mic-3 pour leur utilisation dans la prévention chez un mammifère nouveau-né, d'une pathologie associée à un apicomplexe de la famille des Cryptosporidiidae, ladite pathologie étant la cryptosporidiose, et ledit apicomplexe de la famille des Cryptosporidiidae responsable de la cryptosporidiose étant Cryptosporidium parvum. Souches de Toxoplasma gondii possédant les deux adhésines MIC-1 et MIC-3 inactivées par une modification génique portant sur les deux gènes mic-1 et mic-3 pour leur utilisation selon la revendication 1, dans laquelle ledit mammifère est un être humain ou un animal. Souches de Toxoplasma gondii possédant les deux adhésines MIC-1 et MIC-3 inactivées par une modification génique portant sur les deux gènes mic-1 et mic-3 pour leur utilisation selon la revendication 2, dans laquelle ledit animal appartient au groupe comprenant ou constitué des ovins, des caprins, des porcins, des bovins, des équidés, des camélidés, des canidés ou des félidés. Souches de Toxoplasma gondii possédant les deux adhésines MIC-1 et MIC-3 inactivées par une modification génique portant sur les deux gènes mic-1 et mic-3 pour leur utilisation selon l'une des revendications 1 à 3, dans laquelle lesdites souches de Toxoplasma gondii sont administrées au mammifère à raison de 20 à 109 tachyzoïtes. Souches de Toxoplasma gondii possédant les deux adhésines MIC-1 et MIC-3 inactivées par une modification génique portant sur les deux gènes mic-1 et mic-3 pour leur utilisation selon l'une quelconque des revendications 1 à 4, dans laquelle lesdites souches se trouvent sous une forme galénique choisie parmi le groupe comprenant ou constitué par des suspensions liquides, des dispersions solides ou liquides, des poudres, des pâtes ou des lyophilisats. Souches de Toxoplasma gondii possédant les deux adhésines MIC-1 et MIC-3 inactivées par une modification génique portant sur les deux gènes mic-1 et mic-3 pour leur utilisation selon l'une quelconque des revendications 1 à 5, dans laquelle lesdites souches sont associées à au moins un autre antigène, ou au moins un adjuvant, ou au moins un stabilisant, ou au moins un conservateur ou un mélange d'au moins deux desdits produits permettant d'accroitre la réponse immune dudit mammifère. Par « immunostimulant », on désigne la capacité d'une souche de *Toxoplasma spp* ou *Neospora spp* à induire une activation précoce du système immunitaire de l'hôte. Cette activation met en jeu des composants du système immunitaire tels que les interférons, les cytokines, les cellules phagocytaires, les cellules NK (Natural killer), les cellules dendritiques et le système de complément qui vont agir de façon non spécifique sur le pathogène ciblé. Cette immunostimulation n'est pas basée sur la mise en place d'une immunité adaptative.

Par « prévention », on désigne la prophylaxie ayant pour but d'empêcher l'apparition ou la propagation d'une maladie. Il s'agit notamment de protéger un individu prédisposé à la contraction et au développement d'une pathologie liée à un apicomplexe de la famille des *Cryptosporidiidae.* De tels individus sont notamment des nouveaux nés possédant un système immunitaire immature ou des individus présentant un dysfonctionnement du système immunitaire. Il s'agit également de protéger un mammifère exposé à un risque de contamination par son environnement.

Par « traitement », on désigne non seulement l'inhibition de la progression de la pathologie mais également l'atténuation des symptômes liés à cette pathologie. Le traitement a pour but de réduire l'amplitude des symptômes jusqu'à leur disparition complète permettant à l'individu de retrouver un état physiologique normal.

Par « mammifère », on désigne les êtres humains, certains animaux de rentes ou d'élevage et certains animaux de compagnie.

Par « souches de *Toxoplasma spp* ou *Neospora spp* atténuées », on désigne des souches de *Toxoplasma spp* ou *Neospora spp* qui possèdent une virulence atténuée, inférieure à la virulence des souches de *T. gondii* ou de *N. caninum* sauvages capables d'induire une pathologie, mais qui conservent néanmoins un pouvoir immunogène afin de pouvoir s'en servir dans la prévention ou le traitement d'une pathologie associée à un apicomplexe de la famille des *Cryptosporidiidae.* L'atténuation de la virulence peut résulter soit d'un processus naturel d'évolution de l'espèce soit être induit notamment par des techniques de biologie moléculaire bien connues de l'homme de l'art. Qu'elle soit d'origine naturelle ou bien consécutive de la main de l'homme, l'atténuation de la virulence est due à l'absence d'expression de facteurs de virulence ou à l'expression d'un ou plusieurs facteurs de virulence non fonctionnels ou ayant une fonction altérée. La modification *in vitro* du patrimoine génétique de *Toxoplasma spp* ou *Neospora spp* confère à la souche un caractère de mutant, par opposition à la souche sauvage dont elle dérive. Les souches sauvages de parasites ont non seulement un potentiel immunogène mais sont également virulentes, c'est-à-dire qu'elles sont capables d'induire une pathologie associée à *Toxoplasma spp* ou *Neospora spp* (*i.e.* respectivement la toxoplasmose et la néosporose), rendant leur utilisation impropre dans le cadre de la présente invention. La virulence des souches de *Toxoplasma spp* ou de *Neospora spp* peut être notamment évaluée par des essais d'infectivité cellulaire *in vitro* ou par des essais d'infectivité chez l'animal.

Les tests d'infectivité cellulaire sont réalisés en déposant des tachyzoites sur des cellules confluentes par exemple des cellules HFF (Human Foreskin Fibroblast) ou des cellules Vero, lignées cellulaires fréquemment utilisées pour la production de tachyzoïtes de *T. gondii* ou de *N. caninum.* Le nombre de vacuoles formées et le nombre de parasites dans chaque vacuole est déterminé par observation microscopique. Pour les essais d'infectivité chez l'animal, les différentes souches sont injectées aux animaux et la survie de ces animaux est suivie au cours du temps (Cérède et al, 2005).

Par « pathologies associées à un apicomplexe de la famille des *Cryptosporidiidae* », on désigne les maladies résultant d'une infection par un protozoaire appartenant au phylum des apicomplexes, et en particulier des parasites appartenant à la famille des *Cryptosporidiidae* qui comporte le genre *cryptosporidium.* Plusieurs dizaines d'espèces de *cryptosporidium* sont référencées (Fayer R, 2010, Exp. Parasitol., 124, 90-7). Ces parasites sont capables d'envahir les épithéliums muqueux.

Selon un mode de réalisation particulier, dans l'utilisation selon la présente invention des souches de *Sarcocystidae* choisies parmi *Toxoplasma spp* ou *Neospora spp* isolées de leur milieu naturel et possédant un effet immunostimulant, ledit mammifère est un nouveau-né.

La présente invention a pour objet des souches de *Sarcocystidae* choisies parmi *Toxoplasma spp* ou *Neospora spp,* isolées de leur milieu naturel et possédant un effet immunostimulant pour leur utilisation dans la prévention ou le traitement, chez un mammifère nouveau-né, d'une pathologie associée à un apicomplexe de la famille des *Cryptosporidiidae.*

Par « nouveau-né », on désigne un mammifère du moment de sa naissance jusqu'à son sevrage, c'est-à-dire jusqu'au moment où le mammifère devient capable de s'alimenter par lui-même et ne dépend plus du lait maternel. Le principal avantage de l'utilisation de la souche mutante de *Toxoplasma spp* ou *Neospora spp* pour la prévention et/ou le traitement de pathologies associées à un apicomplexe de la famille des *Cryptosporidiidae* chez le nouveau-né est de stimuler son système immunitaire immature pour induire une réponse immunitaire aspécifique par le biais de la synthèse de molécules inhibant la croissance des apicomplexes.

Selon un mode de réalisation particulier, dans l'utilisation selon la présente invention des souches de *Sarcocystidae* choisies parmi *Toxoplasma spp* ou *Neospora spp* isolées de leur milieu naturel et possédant un effet immunostimulant, ledit mammifère est un être humain ou un animal.

Les animaux visés par la présente invention sont principalement des animaux de rentes ou d'élevage, lesquels présentent un intérêt pour les industries agroalimentaires mais également certains animaux de compagnie.

Selon un mode de réalisation plus particulier, dans l'utilisation selon la présente invention des souches de *Sarcocystidae* choisies parmi *Toxoplasma spp* ou *Neospora spp* isolées de leur milieu naturel et possédant un effet immunostimulant, ledit animal appartient au groupe comprenant ou constitué des ovins, des caprins, des porcins, des bovins, des équidés, des camélidés, des canidés ou des félidés (Fayer, 2004, Vet. Parasitol., 126, 37-59).

Selon un autre mode de réalisation, dans l'utilisation selon la présente invention des souches de *Sarcocystidae* choisies parmi *Toxoplasma spp* ou *Neospora spp* isolées de leur milieu naturel et possédant un effet immunostimulant, lesdites souches de *Toxoplasma spp* ou de *Neospora spp* possèdent au moins une adhésine MIC1 et/ou une adhésine MIC3 inactivée par une modification génique portant sur l'un au moins des gènes *mic1* et/ou *mic3.*

Par « une adhésine MIC1 et/ou une adhésine MIC3 », on désigne les protéines des micronèmes, également appelées adhésines, MIC1 et/ou MIC3 qui jouent un rôle dans la mobilité, la migration ou l'invasion des parasites du phylum des apicomplexes chez son hôte. Ces protéines possèdent des modules de liaisons qui leur permettent de se fixer sur les cellules cibles de l'hôte.

Par « une adhésine inactivée », on désigne une adhésine dont la fonction ne peut plus être assurée au sein de la cellule. Une adhésine est inactivée lorsqu'elle n'est pas produite ou lorsqu'elle est produite mais ne possède pas d'activité fonctionnelle ou possède une activité fonctionnelle réduite. L'inactivation concerne également une adhésine qui ne peut plus se lier à d'autres protéines pour former un complexe.

Par « modification génique », on désigne toute mutation réalisée dans la séquence nucléique d'un gène aboutissant à l'absence d'expression de la protéine codée par ce gène ou aboutissant à l'expression d'une forme non ou moins fonctionnelle de la protéine codée par ce gène. Cette opération nécessite l'intervention de la main de l'homme lorsqu'elle est opérée *in vitro.* Cette mutation peut consister en la délétion de tout ou partie du gène, ou de sa région codante, ou de sa région promotrice, en l'insertion ou la substitution de nucléotides dans la séquence nucléotidique du gène.

Par « gène *mic*1 », on désigne le gène codant pour la protéine des micronèmes MIC1, également appelée adhésine MIC1. Cette protéine contient plusieurs modules dont des domaines de liaison qui lient spécifiquement le lactose. La protéine MIC1 est également capable de se lier à la surface des cellules hôtes.

La construction détaillée de la souche Toxo *mic1* KO est décrite dans les documents US 7, 946, 185 B2 et EP 1 703 914 B1. La construction détaillée de la souche Neo *ncmic1* KO est décrite dans la présente demande.

Par « gène *mic3* », on désigne le gène codant pour la protéine des micronèmes MIC-3, également appelée adhésine MIC3. Cette protéine s'homodimérise pour former un complexe de 90 kDa. MIC3 comporte des domaines de type EGF et un domaine de type lectine. La protéine MIC3 est également capable de se lier à la surface des cellules hôtes.

La construction détaillée de la souche Toxo *mic3* KO est décrite dans les documents US 7, 946, 185 B2 et EP 1 703 914 B1. La construction détaillée de la souche Neo *ncmic3* KO est décrite dans la présente demande.

Selon un autre mode de réalisation particulier, dans l'utilisation selon la présente invention des souches de *Sarcocystidae* choisies parmi *Toxoplasma spp* ou *Neospora spp* isolées de leur milieu naturel et possédant un effet immunostimulant, lesdites souches de *Toxoplasma spp* ou de *Neospora spp* possèdent les deux adhésines MIC1 et MIC3 inactivées par une modification génique portant sur les deux gènes *mic1* et *mic3.*

Par « modification génique portant sur les deux gènes *mic1* et *mic3* », on désigne la mutation réalisée dans la séquence nucléique du gène *mic1* et dans celle du gène *mic3.* Cette double mutation aboutit à l'absence d'expression des protéines MIC1 et MIC3 ou aboutit à l'expression d'une forme non ou moins fonctionnelle des protéines MIC1 et MIC3. Ces souches mutantes de *Toxoplasma spp* ou de *Neospora spp* sont respectivement appelées Toxo *micl-3* KO ou Neo *ncmicl-3* KO et possèdent une virulence très atténuée en comparaison avec les souches sauvages de *T. gondii* de type RH ou de *N. caninum* de type NC1 dont elles dérivent. Les souches Toxo *mic1-3* KO ou Neo *ncmic1-3* KO conservent toutefois un fort pouvoir immunogène. La construction détaillée de la souche Toxo *micl-3* KO est décrite dans les documents US 7, 946, 185 B2 et EP 1 703 914 B1. La construction détaillée de la souche Neo *ncmicl-3* KO est décrite dans la présente demande.

Les souches Toxo *micl-3* KO et Neo *ncmicl-3* KO ont conservé leur capacité à coloniser les tissus cibles sans qu'il y ait développement de phénomène pathogène consécutif de l'administration desdites souches à un mammifère. L'invalidation des gènes *mic1* et *mic3* n'altère pas ou peu le potentiel immunogène de ces souches, mais réduit considérablement leur virulence par rapport à une souche virulente, c'est-à-dire une souche ayant une virulence substantiellement identique à la virulence de la souche à partir de laquelle la souche de virulence atténuée a été obtenue.

L'inoculation de la souche Toxo *micl-3* KO à des souriceaux nouveau-nés entraine une production de cytokines IL-12 et IFN-γ et protège efficacement les souriceaux d'une infection ultérieure à *Cryptosporidium parvum.*

La souche mutante Toxo *mic1-3* KO stimule efficacement la production d'IL-12 et d'IFN-γ à partir de cellules des ganglions mésentériques et des splénocytes d'agneaux confirmant la possibilité d'utiliser cette souche mutante comme immunostimulant chez cette espèce animale cible.

Selon un mode de réalisation particulier, dans l'utilisation selon la présente invention des souches de *Sarcocystidae* choisies parmi *Toxoplasma spp* ou *Neospora spp* isolées de leur milieu naturel et possédant un effet immunostimulant, lesdites souches de *Toxoplasma spp* ou de *Neospora spp* sont respectivement *Toxoplasma gondii ou Neospora caninum.*

Selon un autre mode de réalisation, dans l'utilisation selon la présente invention des souches de *Sarcocystidae* choisies parmi *Toxoplasma spp* ou *Neospora spp* isolées de leur milieu naturel et possédant un effet immunostimulant, ledit effet immunostimulant desdites souches conduit à la sécrétion d'interleukine-12 (IL-12) puis d'interféron-γ (IFN-γ).

Par « interleukine-12 (IL-12) » on désigne la cytokine synthétisée par des cellules de l'immunité telles que les monocytes, les cellules dendritiques et les macrophages. La cytokine est sécrétée précocement et va ainsi activer les cellules cibles (cellules T et Natural Killer) afin que celles-ci sécrètent à leur tour de l'IFN-γ.

Par «interféron-γ (IFN-γ) », on désigne la cytokine synthétisée par les lymphocytes T CD4+, CD8+ et les cellules Natural Killer (NK) activés par l'IL-12. La sécrétion d'IFN-γ par les cellules de l'organisme permettra de produire une réponse innée protectrice contre *C. parvum.* Cette cytokine aura une activité pléiotropique de part la diversité des lignées cellulaires ciblées.

On entend par cytokines toutes les molécules impliquées dans le développement et la régulation du système immunitaire. Les cytokines sont des protéines glycosylées ou non qui peuvent être classées en fonction de leur activité biologique :
- pro-inflammatoires : on y retrouve des interleukines (IL) et les facteurs nécrosant les tumeurs (TNF),
- immuno-régulatrices : on y retrouve les interleukines (IL),
- effectrices : on y retrouve les interférons (IFN), les facteurs nécrosant les tumeurs (TNF) et les chimiokines.

Selon un autre mode de réalisation plus particulier, dans l'utilisation selon la présente invention des souches de *Sarcocystidae* choisies parmi *Toxoplasma spp* ou *Neospora spp* isolées de leur milieu naturel et possédant un effet immunostimulant, ladite sécrétion d'interleukine-12 (IL-12) et d'interféron-γ (IFN-γ) débute entre 3 et 9 jours après l'utilisation desdites souches de *Toxoplasma spp* ou de *Neospora spp* comme immunostimulant.

Selon un mode de réalisation plus particulier, dans l'utilisation selon la présente invention des souches de *Sarcocystidae* choisies parmi *Toxoplasma spp* ou *Neospora spp* isolées de leur milieu naturel et possédant un effet immunostimulant, ladite pathologie associée à un apicomplexe de la famille des *Cryptosporidiidae* est la cryptosporidiose.

Par « cryptosporidiose », on désigne la pathologie caractérisée par des symptômes de type crampes, fièvre, fatigue, nausées et surtout des diarrhées pouvant conduire à une déshydratation du mammifère infecté par *Cryptosporidium parvum* ou par une autre espèce de *Cryptosporidium.* La cryptosporidiose affecte principalement les intestins des mammifères mais peut également infecter les voies biliaires et pancréatiques et le tractus respiratoire chez l'individu immunodéprimé. L'infestation s'opère par ingestion d'oocystes présents dans les excréments des animaux parasités qui ont souillé l'environnement (l'eau, la terre ou des aliments crus). Les symptômes apparaissent deux à dix jours après que l'infection ait eu lieu et persistent pendant plus de deux semaines. La sévérité de cette pathologie varie en fonction de l'âge de l'hôte infecté mais plus particulièrement de l'état de son système immunitaire. Chez un hôte possédant un système immunitaire mature et fonctionnel, la cryptosporidiose est sans conséquence sur la santé de son hôte. A l'inverse, un hôte très jeune, très vieux ou présentant un système immunitaire immature ou déficient, peut développer des formes très sévères de la cryptoporidiose.

Par « système immunitaire immature ou déficient », on désigne le système immunitaire pour lequel un ou plusieurs lignages cellulaires sont soit absents, soit déficients. L'immaturité du système immunitaire est un trait commun chez les mammifères nouveaux-nés qui les rend particulièrement vulnérables aux infections parasitaires. L'absorption des anticorps maternels via le colostrum confère un certain degré de protection au nouveau-né le temps que son système immunitaire se mette en place. L'immaturité ou la déficience du système immunitaire peut également résulter de pathologies d'origine génétique, notamment chez l'Homme, comme le syndrome de Wiskott-Aldrich, syndrome lymphoprolifératif lié à l'X. Cette déficience peut également être consécutive d'une infection par le virus de l'immunodéficience humaine (VIH) ou être consécutive à un traitement thérapeutique associé à une greffe d'organe ou à la prise en charge médicale de certains cancers par chimio- ou radiothérapie. Les mammifères possédant un système immunitaire immature ou déficient sont rendus particulièrement vulnérables aux infections à apicomplexes.

Selon un mode de réalisation encore plus particulier, dans l'utilisation selon la présente invention des souches de *Sarcocystidae* choisies parmi *Toxoplasma spp* ou *Neospora spp* isolées de leur milieu naturel et possédant un effet immunostimulant, ledit apicomplexe de la famille des *Cryptosporidiidae* responsable de la cryptosporidiose est au moins un apicomplexe choisi parmi le groupe constitué de *Cryptosporidium parvum, Cryptosporidium bovis, Cryptosporidium andersoni, Cryptosporidium ryanae, Cryptosporidium muris, Cryptosporidium ubiquitum, Cryptosporidium hominis, Cyrptosporidium canis, Cyrptosporidium felis, Cyrptosporidium baileyi, Cyrptosporidium meleagridis ou Cryptosporidium xiaoi.*

Par « *Cryptosporidium parvum, Cryptosporidium bovis, Cryptosporidium andersoni, Cryptosporidium ryanae, Cryptosporidium muris, Cryptosporidium ubiquitum, Cryptosporidium hominis, Cyrptosporidium canis, Cyrptosporidium felis, Cyrptosporidium baileyi, Cyrptosporidium meleagridis ou Cryptosporidium xiaoi* », on désigne les protozoaires du phylum des apicomplexes capables de provoquer des désordres intestinaux de nature hydrique chez la plupart des mammifères. Ces parasites sont notamment capables de provoquer la cryptosporidiose chez les ovins, les caprins, les porcins, les bovins, les équidés, les chameaux, les camélidés, les canidés, les félidés ou les humains (Fayer, 2004, Vet. Parasitol., 126, 37-59) possédant un système immunitaire immature ou déficient.

La présente invention concerne également les souches de *Toxoplasma gondii* ou de *Neospora caninum* qui possèdent les deux adhésines MIC1 et MIC3 inactivées par une modification génique portant sur les deux gènes *mic1* et *mic3* pour leur utilisation dans la prévention ou le traitement, chez un mammifère, d'une pathologie associée à un apicomplexe de la famille des *Cryptosporidiidae,* ladite pathologie associée à un apicomplexe de la famille des *Cryptosporidiidae* étant la cryptosporidiose.

La présente invention concerne également les souches de *Toxoplasma gondii* ou de *Neospora caninum* isolées de leur milieu naturel et possédant un effet immunostimulant pour leur utilisation dans la prévention ou le traitement, chez un mammifère, d'une pathologie associée à un apicomplexe de la famille des *Cryptosporidiidae,* lesdites souches de *Toxoplasma gondii* ou de *Neospora caninum* possédant les deux adhésines MIC-1 et MIC-3 inactivées par une modification génique portant sur les deux gènes *mic-1* et *mic-3,* et ladite pathologie associée à un apicomplexe de la famille des *Cryptosporidiidae* est la cryptosporidiose.

L'invention concerne également les souches de *Toxoplasma gondii* ou de *Neospora caninum* isolées de leur milieu naturel et possédant un effet immunostimulant, lesdites souches de *Toxoplasma gondii* ou de *Neospora caninum* possèdant les deux adhésines MIC1 et MIC3 inactivées par une modification génique portant sur les deux gènes *mic1* et *mic3* pour leur utilisation dans la prévention ou le traitement, chez un mammifère, d'une pathologie associée à un apicomplexe de la famille des *Cryptosporidiidae,* lesdites souches de *Toxoplasma gondii* ou de *Neospora caninum* étant administrées audit mammifère à raison de 20 à 10⁹ tachyzoïtes.

Par « tachyzoïte », on désigne la forme de réplication rapide de *Toxoplasma gondii* ou de *Neospora caninum.* Le tachyzoïte possède une forme de croissant de taille variant de 5-8 x 2-3µm. La partie apicale du parasite comporte des conoïdes qui participent à la pénétration du parasite dans la cellule hôte. Les micronèmes, les rhoptries et les granules denses constituent les trois organelles majeures du tachyzoïte qui comporte également un noyau, un apicoplaste, un appareil de Golgi, un réticulum endoplasmique et un organite proche de la mitochondrie.

La détermination d'une dose efficace de tachyzoïtes en vue du traitement prophylactique des mammifères permet de limiter l'infection ou la transmission de l'agent pathogène responsable de la cryptosporidiose. Un tel traitement pourra être adapté et/ou répété autant de fois que nécessaire par l'homme de l'art, en fonction de l'âge et du statut immunologique du mammifère.

La mise en contact des tachyzoïtes avec le mammifère est réalisable non seulement sur un mammifère présentant les symptômes inhérents à la cryptosporidiose mais également sur un mammifère ne présentant aucun des symptômes de la cryptosporidiose mais qui est au contact d'autres mammifères infectés par *Cryptosporidium parvum* ou par une autre espèce de *Cryptosporidium* susceptible d'induire la cryptosporidiose.

Selon un mode de réalisation particulier, les souches de *Sarcocystidae* choisies parmi *Toxoplasma spp* ou *Neospora spp* isolées de leur milieu naturel et possédant un effet immunostimulant pour leur utilisation selon la présente invention se trouvent sous une forme galénique choisie parmi le groupe comprenant ou constitué par des suspensions liquides, des dispersions solides ou liquides, des poudres, des pâtes ou des lyophilisats.

La forme galénique est adaptée par l'homme de l'art en fonction du mode d'administration choisi. Tous les modes classiques d'administration peuvent être envisagés : par voie parentérale (intraveineuse, sous-cutanée, intradermique, intramusculaire, intrapéritonéale, et intra-nasale) ou par voie entérale.

Selon un mode de réalisation plus particulier, les souches de *Sarcocystidae* choisies parmi *Toxoplasma spp* ou *Neospora spp* isolées de leur milieu naturel et possédant un effet immunostimulant pour leur utilisation selon la présente invention sont associées à au moins un autre antigène, ou au moins un adjuvant, ou au moins un stabilisant, au ou moins un conservateur ou un mélange d'au moins deux desdits produits permettant d'accroître la réponse immune dudit mammifère.

Par « antigène », on désigne toute protéine naturelle ou recombinante, sous sa forme native ou mutée, issue d'un parasite ou d'un agent pathogène autre que *Toxoplasma spp* ou *Neospora spp* capable d'induire chez un mammifère une réponse immune cellulaire ou humorale. Le but de l'association de la souche mutante de *Toxoplasma spp* ou *Neospora spp* avec un tel antigène est d'amplifier la réponse immune du mammifère et lui conférer ainsi une meilleure protection vis-à-vis d'une infection à apicomplexe.

Par « adjuvant », on désigne toute substance capable de renforcer et prolonger la réponse immunitaire dirigée contre l'antigène ciblé. Le mécanisme mis en jeu afin de rendre la réponse immunitaire plus efficace est dépendant de l'adjuvant utilisé. Les adjuvants sont des substances bien connues de l'homme de l'art parmi lesquelles figurent notamment les sels d'aluminium, le squalène, les saponines, les constituants ou les toxines bactériennes, ou encore certaines protéines (peptone, albumine, caséine).

Par « stabilisant ou conservateurs », on désigne les composés permettant une parfaite conservation des souches de *Toxoplasma spp* ou *Neospora spp* dans leur conditionnement.

Les stabilisants ou conservateurs sont des substances bien connues de l'homme de l'art parmi lesquelles figurent notamment les carbohydrates (sorbitol, mannitol, lactose, saccharose, glucose, dextran, tréhalose), les solvants organiques polaires, tels que le DMSO (diméthylsulfoxyde), les polysorbates.

Par « accroître la réponse immune », on désigne l'activation des différentes voies du système immunitaire. Chez un mammifère possédant un système immunitaire immature ou déficient, il s'agit d'activer la réponse aspécifique par le biais d'un accroissement de la synthèse de cytokines de catégories distinctes comme les interférons ou les interleukines. Parmi ces cytokines on peut citer l'interleukine-12 (IL-12) qui va activer les cellules LT CD4+ ou CD8+ ainsi que les NK. Ces cellules activées vont-elles-même sécréter l'interféron γ (IFN-γ) qui joue un rôle dans les mécanismes immunologiques qui contrôlent la multiplication de nombreux parasites intracellulaires, empêchant ainsi leur propagation dans l'organisme. Chez un mammifère adulte possédant un système immunitaire mature, l'accroissement de la réponse immunitaire passe par une immunité adaptative (prolifération spécifique en réponse aux antigènes étrangers) en plus de la réponse aspécifique.

La présente invention concerne également une méthode pour induire une réponse immune chez un mammifère comprenant une étape d'administration, audit mammifère, de tachyzoïtes de souches de *Sarcocystidae* choisies parmi *Toxoplasma spp* ou *Neospora spp,* isolées de leur milieu naturel et possédant un effet immunostimulant, permettant d'induire une réponse immune.

La présente invention concerne également une méthode pour induire une réponse immune chez un mammifère nouveau-né comprenant une étape d'administration, audit mammifère nouveau-né, de tachyzoïtes de souches de *Sarcocystidae* choisies parmi *Toxoplasma spp* ou *Neospora spp,* isolées de leur milieu naturel et possédant un effet immunostimulant, permettant d'induire une réponse immune.

Par « réponse immune », on désigne les deux phases essentielles constituées par la reconnaissance de l'antigène et la réaction destinée à l'élimination dudit antigène. Lorsqu'un antigène entre pour la première fois dans l'organisme, c'est la réponse immunitaire non spécifique qui est activée. Elle met en jeu des mécanismes non clonaux puisqu'elle ne nécessite pas de clones cellulaires spécifiques. La réaction inflammatoire créée par cette réponse non spécifique va conduire vers une immunité adaptative. Cette réponse met en jeu des cellules reconnaissant spécifiquement l'antigène et conduira ensuite à une expansion clonale. Cette expansion clonale sera à l'origine d'une réponse immunitaire très efficace et d'une réponse mémoire (réponse immunitaire spécifique dès la seconde entrée de l'antigène dans l'organisme).

Selon un mode de réalisation particulier, dans la méthode selon la présente invention, ledit mammifère est un nouveau né.

Selon un mode de réalisation plus particulier, dans la méthode selon la présente invention, ledit mammifère est un être humain ou un animal.

Selon un mode de réalisation encore plus particulier, dans la méthode selon la présente invention, l'animal appartient au groupe comprenant ou constitué des ovins, des caprins, des porcins, des bovins, des équidés, des camélidés, des canidés ou des félidés.

Selon un autre mode de réalisation, dans la méthode selon la présente invention, lesdites souches de *Sarcocystidae* choisies parmi *Toxoplasma spp* ou *Neospora spp* possèdent au moins une adhésine MIC1 et/ou une adhésine MIC3 inactivée par une modification génique qui porte sur l'un au moins des gènes *mic1* et/ou *mic3.*

Selon un autre mode de réalisation particulier, dans la méthode selon la présente invention, lesdites souches de *Sarcocystidae* choisies parmi *Toxoplasma spp* ou *Neospora spp* possèdent les deux adhésines MIC1 et MIC3 inactivées par une modification génique qui porte sur les deux gènes *mic1* et *mic3.*

Selon un autre mode de réalisation plus particulier, dans la méthode selon la présente invention, lesdites souches de *Sarcocystidae* choisies parmi *Toxoplasma spp* ou *Neospora spp* sont respectivement *Toxoplasma gondii* ou *Neospora caninum.*

Selon encore un autre mode de réalisation particulier, dans la méthode selon la présente invention, les souches de *Sarcocystidae* choisies parmi *Toxoplasma spp* ou *Neospora spp* induisent une réponse immune aspécifique caractérisée, notamment par la sécrétion d'IL-12 et/ou d'IFN-γ.

La présente invention concerne également une méthode pour protéger un mammifère contre une parasitose associée à un apicomplexe de la famille des *Cryptosporidiidae* comprenant une étape d'administration, audit mammifère, de tachyzoïtes de souches de *Sarcocystidae* choisies parmi *Toxoplasma spp* ou *Neospora spp,* isolées de leur milieu naturel et possédant un effet immunostimulant, permettant d'induire une protection contre ladite parasitose.

Selon un mode de réalisation plus particulier, dans la méthode selon la présente invention, le mammifère est un nouveau-né.

Selon un mode de réalisation encore plus particulier, dans la méthode selon la présente invention, le mammifère est un être humain ou un animal.

Selon un mode de réalisation particulier, dans la méthode selon la présente invention, l'animal appartient au groupe comprenant ou constitué des ovins, des caprins, des porcins, des bovins, des équidés, des camélidés, des canidés ou des félidés.

Selon un mode de réalisation particulier, dans la méthode selon la présente invention, lesdites souches de *Sarcocystidae* choisies parmi *Toxoplasma spp* ou *Neospora spp* possèdent au moins une adhésine MIC1 et/ou une adhésine MIC3 inactivée par une modification génique portant sur l'un au moins des gènes *mic1* et/ou *mic3.*

Selon un mode de réalisation plus particulier, dans la méthode selon la présente invention, lesdites souches de *Sarcocystidae* choisies parmi *Toxoplasma spp* ou *Neospora spp* possèdent les deux adhésines MIC1 et MIC3 inactivées par une modification génique portant sur les deux gènes *mic1* et *mic3.*

Selon un mode de réalisation plus particulier, dans la méthode selon la présente invention, ladite parasitose associée à un apicomplexe de la famille des *Cryptosporidiidae* est la cryptosporidiose.

Selon un autre mode de réalisation plus particulier, dans la méthode selon la présente invention, l'apicomplexe de la famille des *Cryptosporidiidae* responsable de la cryptosporidiose est au moins un apicomplexe choisi parmi le groupe constitué de *Cryptosporidium parvum, Cryptosporidium bovis, Cryptosporidium andersoni, Cryptosporidium ryanae, Cryptosporidium muris, Cryptosporidium ubiquitum, Cryptosporidium hominis, Cyrptosporidium canis, Cyrptosporidium felis, Cyrptosporidium baileyi, Cyrptosporidium meleagridis ou Cryptosporidium xiaoi.*

Selon un mode de réalisation encore plus particulier, dans la méthode selon la présente invention, l'administration, audit mammifère, desdits tachyzoïtes desdites souches de *Sarcocystidae* choisies parmi *Toxoplasma spp* ou *Neospora spp* est effectuée par voie entérale ou par voie parentérale.

Selon encore un autre mode de réalisation, dans la méthode selon la présente invention, l'administration, audit mammifère, desdits tachyzoïtes desdites souches de *Sarcocystidae* choisies parmi *Toxoplasma spp* ou *Neospora spp* est effectuée avant l'exposition dudit mammifère à l'apicomplexe de la famille des *Cryptosporidiidae* responsable de la cryptosporidiose.

Selon encore un autre mode de réalisation, dans la méthode selon la présente invention, l'administration, audit mammifère, desdits tachyzoïtes desdites souches de *Sarcocystidae* choisies parmi *Toxoplasma spp* ou *Neospora spp* est effectuée pendant l'exposition dudit mammifère à l'apicomplexe de la famille des *Cryptosporidiidae* responsable de la cryptosporidiose.

Selon encore un autre mode de réalisation, dans la méthode selon la présente invention, l'administration, audit mammifère, desdits tachyzoïtes desdites souches de *Sarcocystidae* choisies parmi *Toxoplasma spp* ou *Neospora spp* est effectuée après l'exposition dudit mammifère à l'apicomplexe de la famille des *Cryptosporidiidae* responsable de la cryptosporidiose.

Dans les trois modes de réalisation précités, ledit mammifère peut être un nouveau-né.

Les figures et les exemples suivants sont donnés uniquement à titre d'illustration de l'objet de la présente invention dont ils ne constituent en aucune manière une limitation.

### Description des figures

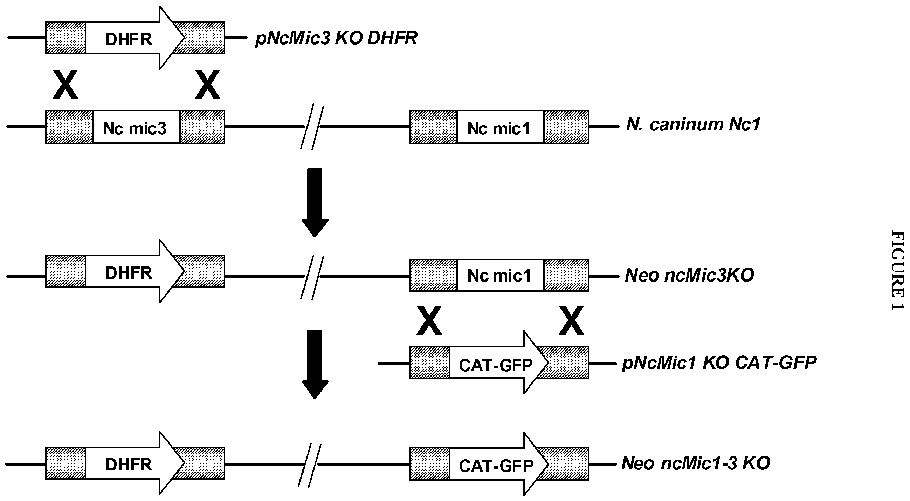

Figure 1 : cette figure illustre les 2 étapes de recombinaison homologue pour obtenir la souche Neo *ncmic1-3* KO. La première étape de recombinaison homologue permet l'intégration du gène codant pour l'enzyme dihydrofolate réductase (DHFR) au locus du gène *ncmic3.* Une sélection avec la pyriméthamine permet d'amplifier la souche simple mutante Neo *ncmic3* KO. La souche Neo *ncmic3* KO ainsi obtenue sert à la seconde étape de recombinaison homologue qui permet l'intégration du gène codant pour la protéine chimérique chloramphénicol-acétyl-transférase/green fluorescent protein (CAT-GFP) au locus du gène *ncmic1.* Une sélection avec le chloramphénicol permet ensuite d'amplifier la souche double mutante Neo *ncmic1-3* KO.

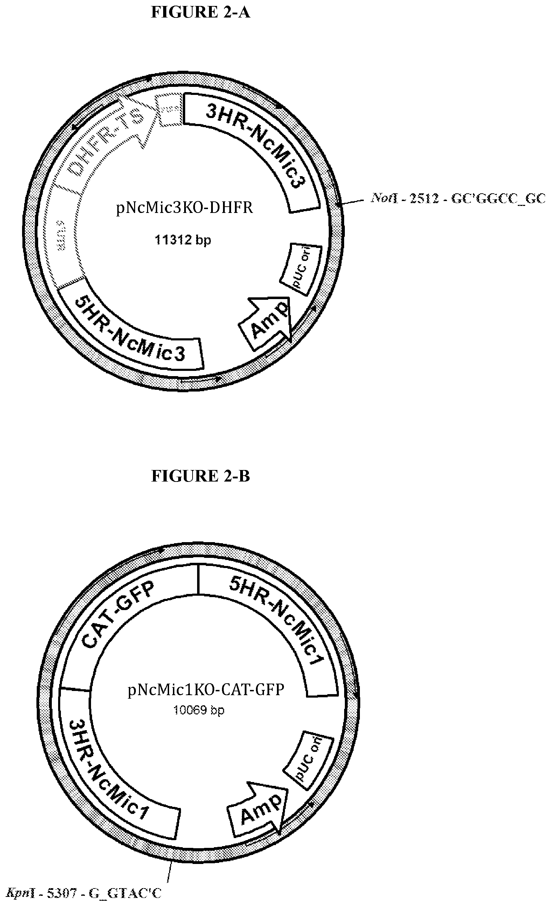

Figure 2-A : cette figure est une représentation schématique du plasmide pNcMic3KO-DHFR. Ce plasmide de 11 312 paires de bases comprend le gène de sélection *DHFR* flanqué par les régions homologues (5HR-NcMic3 et 3HR-NcMic3) des séquences flanquant le gène *ncmic3,* le gène de résistance à l'ampicilline (Amp) ainsi que le site de restriction Not I qui permet sa linéarisation.
Figure 2-B : cette figure est une représentation schématique du plasmide pNcMic1KO-CAT-GFP. Ce plasmide de 10 069 paires de bases comprend le gène de sélection CAT-GFP flanqué par les régions homologues (3HR-NcMic1 et 5HR-NcMic1) des séquences flanquant le gène *ncmic1,* le gène de résistance à l'ampicilline (Amp) ainsi que le site de restriction Kpn I qui permet sa linéarisation.

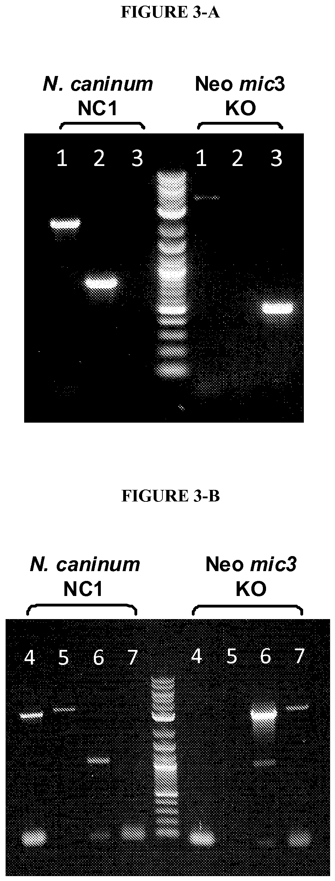

Figure 3-A : cette figure représente les profils électrophorétiques des produits PCR obtenus respectivement chez la souche sauvage NC1 de *N. caninum* et chez la souche mutante Neo *ncmic3* KO, en utilisant les jeux d'amorces des PCR n°1, n°2 ou n°3 du Tableau II qui correspondent aux SEQ ID NO : 5 à SEQ ID NO : 10.
Figure 3-B : cette figure représente les profils électrophorétiques des produits PCR obtenus respectivement chez la souche sauvage NC1 de *N.caninum* et chez la souche mutante Neo *ncmic3* KO, en utilisant les jeux d'amorces des PCR n°4, n°5, n°6 ou n°7 du Tableau II qui correspondent aux SEQ ID NO : 11 à SEQ ID NO : 16.

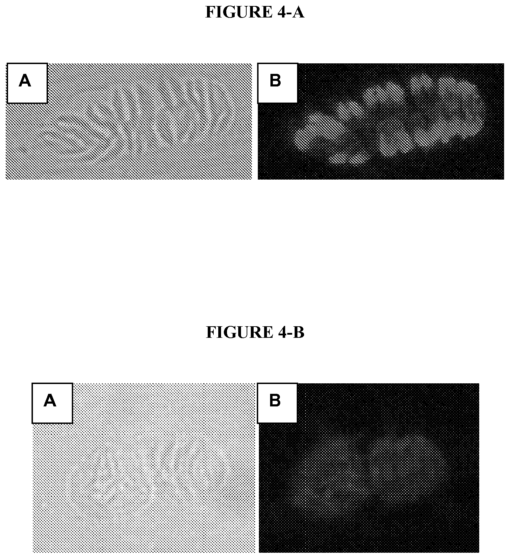

Figure 4-A : cette figure illustre l'analyse de la détection de la protéine NcMIC3 chez la souche sauvage NC1 de *N. caninum* par immunofluorescence, en utilisant un anticorps reconnaissant spécifiquement la protéine NcMIC3. Un même champ microscopique est visualisé en lumière directe (image A) ou en fluorescence (image B).
Figure 4-B : cette figure illustre l'analyse de la détection de la protéine NcMIC3 chez la souche mutante de *N. caninum* Neo *ncmic3* KO par immunofluorescence, en utilisant un anticorps spécifiquement dirigé contre la protéine NcMIC3. Un même champ microscopique est visualisé en lumière directe (image A) ou en fluorescence (image B).

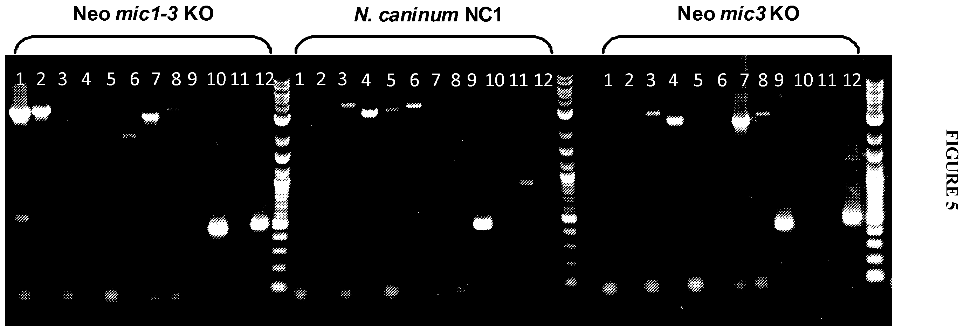

Figure 5 : cette figure représente les profils électrophorétiques des produits PCR obtenus respectivement chez la souche sauvage NC1 de *N. caninum,* chez la souche mutante Neo *ncmic3* KO et chez la souche mutante Neo *ncmic1-3* KO en utilisant les jeux d'amorces des PCR n°1 à n° 12 du Tableau VII qui correspondent aux SEQ ID NO : 7 à 16 et aux SEQ ID NO : 21 à 30.

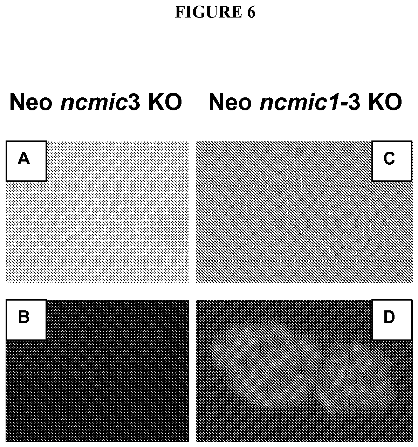

Figure 6 : cette figure illustre l'analyse de la détection de la protéine GFP chez les souches mutantes Neo *ncmic3* KO (images A et B) et Neo *ncmic1-3* KO (image C et D) par immunofluorescence, en utilisant les propriétés fluorescentes de la protéine CAT-GFP. Un même champ microscopique est visualisé en lumière directe (images hautes A et C) ou en fluorescence (images basses B et D).

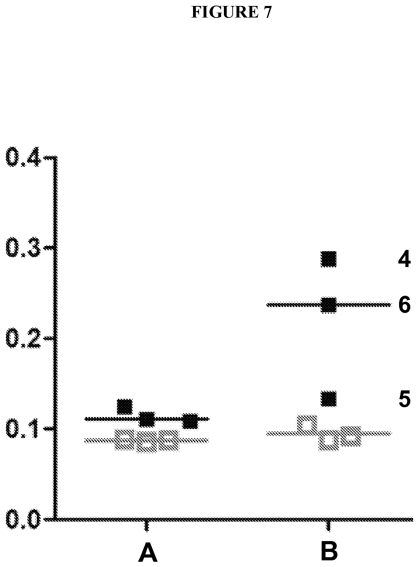

Figure 7 : la figure 7 illustre le dosage des anticorps de type IgM anti-*Toxoplasma gondii* dans le sérum de souriceau. Le dosage des anticorps de type IgM anti-*Toxaplasma gondii* dans le sérum a été réalisé 3 jours (carrés gris vides) ou 9 jours (carrés noirs pleins) après que les souriceaux aient reçu 20 tachyzoïtes de la souche Toxo *mic1-3* KO par voie intrapéritonéale. Des souriceaux n'ayant reçu aucun tachyzoïte de la souche Toxo *micl-3* KO servent de témoins.
   - en abscisse : lots de souriceaux C57BL/6 témoins (A) et inoculés avec la souche Toxo *mic1-3* KO (B)
   - en ordonnée : valeurs d'absorbance mesurées à 405 nm.
   Les trois souriceaux ayant reçu 20 tachyzoïtes de la souche Toxo *micl-3* KO et sacrifiés à 9 jours apparaissent par leur numéro (4, 5, 6) sur la figure 7.

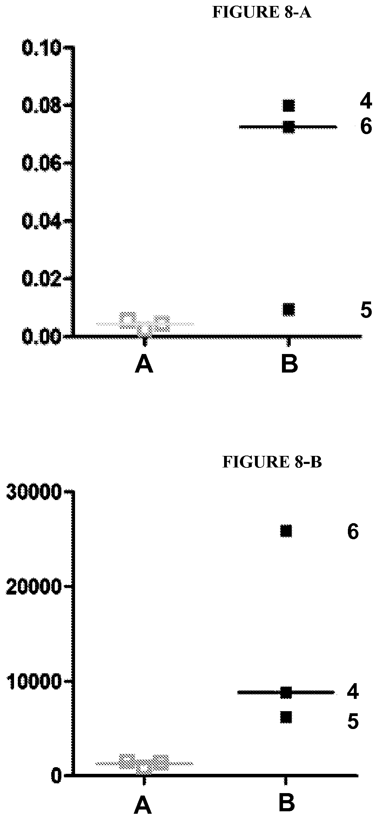

Figure 8-A : la figure 8-A illustre la mesure de l'expression de l'interféron-gamma (IFN-γ) dans l'iléon de souriceau. Le niveau d'expression du gène codant pour l'IFN-γ a été mesuré par PCR quantitative sur les ARN totaux extraits d'un fragment d'iléon à l'aide d'amorces spécifiques (SEQ ID NO : 33 à 34), 9 jours après que les souriceaux aient reçu 20 tachyzoïtes de la souche Toxo *mic1-3* KO par voie intrapéritonéale (carrés noirs pleins). Des souriceaux n'ayant reçu aucun tachyzoïtes de la souche Toxo *mic1-3* KO servent de témoins (carrés gris vides).
   - en abscisse : lots de souriceaux C57BL/6 témoins (A) et inoculés avec la souche Toxo *mic1-3* KO (B)
   - en ordonnée : ratio IFN-γ/HPRT.
   Les trois souriceaux ayant reçu 20 tachyzoïtes de la souche Toxo *mic1-3* KO et sacrifiés à 9 jours apparaissent par leur numéro (4, 5, 6) sur la figure 8-A.
Figure 8-B : la figure 8-B illustre la mesure de l'expression de l'interleukine-12 (sous unité p40) (IL-12p40) dans l'iléon de souriceau. Le niveau d'expression du gène codant pour l'IL-12p40 a été mesuré par PCR quantitative sur les ARN totaux extraits d'un fragment d'iléon à l'aide d'amorces spécifiques (SEQ ID NO : 31 à 32), 9 jours après que les souriceaux aient reçu 20 tachyzoïtes de la souche Toxo *mic1-3* KO par voie intrapéritonéale (carrés noirs pleins). Des souriceaux n'ayant reçu aucun tachyzoïtes de la souche Toxo *mic1-3* KO servent de témoins (carrés gris vides) :
   - en abscisse : lots de souriceaux C57BL/6 témoins (A) et immunostimulés par la souche Toxo *micl-3* KO (B)
   - en ordonnée : ratio IL-12p40/HPRT.
   Les trois souriceaux ayant reçu 20 tachyzoïtes de la souche Toxo *mic1-3* KO et sacrifiés à 9 jours apparaissent par leur numéro (4, 5, 6) sur la figure 8-B.

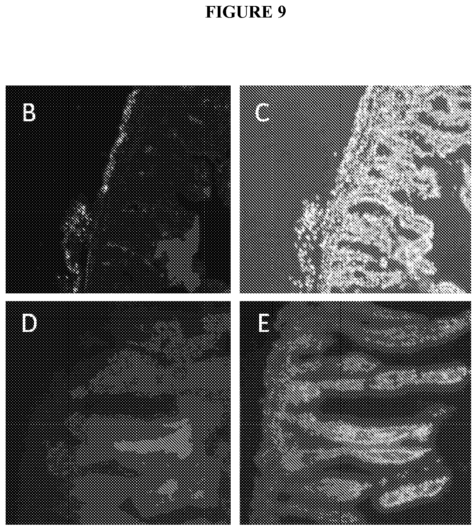

Figure 9 : la figure 9 illustre la détection de la présence de tachyzoïtes de Toxo *micl-3* KO dans l'intestin de souriceau. La détection de la présence de tachyzoïtes de Toxo *mic1-3* KO a été réalisée par immunohistologie sur des coupes d'intestin 9 jours après que les souriceaux ont reçu 20 tachyzoïtes de la souche Toxo *mic1-3* KO par voie intrapéritonéale. L'immunomarquage est réalisé à l'aide d'un anticorps polyclonal de lapin anti-*SAG-1* et d'un anticorps secondaire anti-lapin couplé à l'isothiocyanate de fluoresceine.
   La détection de tachyzoïtes de *Toxoplasma gondii mic1-3* KO est montrée pour les intestins des souriceaux n° 4 (B) et n° 6 (D). Les tachyzoïtes apparaissent sous forme de points blancs dans les muscles intestinaux du souriceau n° 4 (B).
   Les noyaux des cellules des villosités intestinales et des muscles intestinaux des souriceaux n° 4 (C) et n° 6 (E) sont marqués par l'Hoechst et apparaissent sous forme de points blancs.

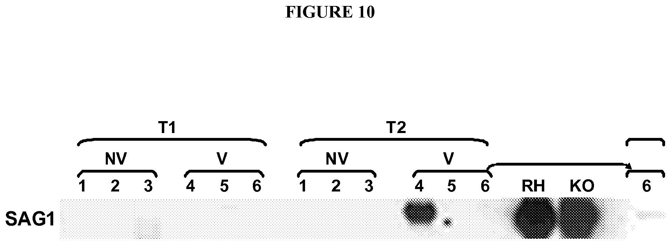

Figure 10: la figure 10 illustre la présence de *Toxoplasma gondii* via l'expression de l'antigène de surface SAG-1 dans l'iléon de souriceau.
   Les ADNc obtenus à partir des ARN totaux extraits d'un fragment d'iléon, ont servi de matrice à l'amplification de SAG-1 par PCR à l'aide d'amorces spécifiques (SEQ ID NO : 37 à 38). Les souriceaux ont été sacrifiés trois jours (T1) ou 9 jours (T2) après avoir reçu 20 tachyzoïtes de la souche Toxo *micl-3* KO par voie intrapéritonéale (V). Des souriceaux n'ayant reçu aucun tachyzoïte de la souche Toxo *mic1-3* KO servent de témoins (NV). Les produits de PCR ont été déposés sur un gel d'agarose.
   SAG-1 a également été amplifié à partir de l'ADN génomique de la souche sauvage de *Toxoplasma gondii* (RH) et à partir de l'ADN génomique de la souche mutée de Toxo *mic1-3* KO (KO). Les produits d'amplification servent de témoins de taille du produit d'amplification de SAG-1.
   Les souriceaux apparaissent par leur numéro (1, 2, 3, 4, 5, 6) sur la figure 10.

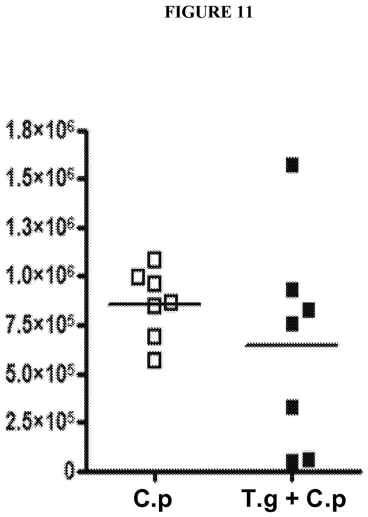

Figure 11: la figure 11 illustre le dénombrement des oocystes de *Cryptosporidium parvum* dans l'intestin de souriceau. Le comptage du nombre d'oocystes de *Cryptosporidium parvum* a été réalisé en cellule de Thoma à partir d'un extrait de broyat d'intestin placé en solution sucrée. Les intestins proviennent :
   - de souriceaux challengés avec 500 000 parasites de *Cryptosporidium parvum* et ayant reçu 20 tachyzoïtes de la souche Toxo *mic1-3* KO par voie intrapéritonéale 3 jours préalablement au challenge par *Cryptosporidium parvum* (carrés noirs pleins), ou
   - de souriceaux challengés avec 500 000 parasites de *Cryptosporidium parvum* et n'ayant reçu aucun tachyzoïte de la souche Toxo *mic1-3* KO (carrés gris vides).
   Les souriceaux sont sacrifiés 6 jours après l'infection par *Cryptosporidium parvum.*
   - en abscisse : lots de souriceaux C57BL/6 infectés par *Cryptosporidium parvum* seuls (C.p) ou par Toxo *mic1-3* KO puis *Cryptosporidium parvum* (T.g + C.p)
   - en ordonnée : nombre total d'oocystes de *Cryptosporidium parvum* dans l'intestin.

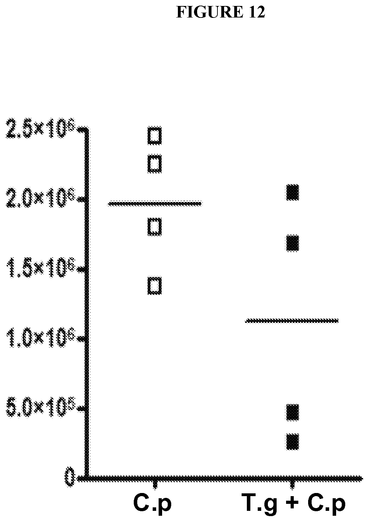

Figure 12: la figure 12 illustre la détection de la présence d'oocystes de *Cryptosporidium parvum* dans l'intestin de souriceau. Le comptage du nombre d'oocystes de *Cryptosporidium parvum* a été réalisé en cellule de Thoma à partir d'un extrait de broyat d'intestin placé en solution sucrée. Les intestins proviennent :
   - de souriceaux challengés avec 1 000 000 parasites de *Cryptosporidium parvum* et ayant reçu 20 000 tachyzoïtes de la souche Toxo *mic1-3* KO par voie orale 3 jours préalablement au challenge par *Cryptosporidium parvum* (carrés noirs pleins), ou
   - de souriceaux challengés avec 1 000 000 parasites de *Cryptosporidium parvum* et n'ayant reçu aucun tachyzoïte de la souche Toxo *mic1-3* KO (carrés gris vides).
   Les souriceaux sont sacrifiés 7 jours après l'infection par *Cryptosporidium parvum.*
   - en abscisse : lots de souriceaux C57BL/6 infectés par *Cryptosporidium parvum* seuls (C.p) ou par Toxo *mic1-3* KO puis *Cryptosporidium parvum* (T.g + C.p)
   - en ordonnée : nombre total d'oocystes de *Cryptosporidium parvum* dans l'intestin.

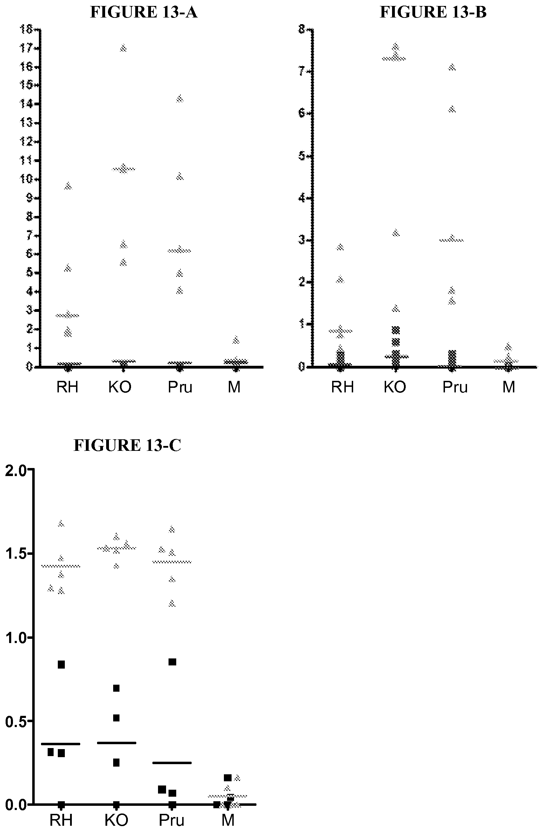

Figure 13-A : la figure 13-A illustre le dosage de l'interleukine-12 (IL-12) dans les cellules mononucléées issues de la rate d'agneau et de mouton adulte. Des échantillons de cellules mononuclées issues de la rate d'agneau (triangles gris) et de mouton adulte (carrés noirs) ont été infectés *in vitro* par trois souches distinctes de *Toxoplasma gondii* : la souche sauvage de type I (RH), la souche mutante de type I Toxo *micl-3* KO (KO) et la souche sauvage de type II (Pru). Des cellules mononuclées issues de la rate d'agneau et de mouton adulte non infectées *in vitro* servent de témoins (M).
   - en abscisse : « RH » : souche sauvage de Toxoplasma gondii de type I, « KO » : souche Toxo *mic 1-3* KO, « Pru » souche sauvage de type II, « M » : cellule de rate cultivées *in vitro* sans stimulant (témoin négatif)
   - en ordonnée : concentration d'IL-12 (UI/ml).
Figure 13-B : la figure 13-B illustre le dosage de l'interleukine-12 (IL-12) dans les cellules mononucléées issues des ganglions mésentériques d'agneau et de mouton adulte. Des échantillons de cellules mononuclées issues des ganglions mésentériques d'agneau (triangles gris) et de mouton adulte (carrés noirs) ont été infectés *in vitro* par trois souches distinctes de *Toxoplasma gondii* : la souche sauvage de type I (RH), la souche mutante de type I Toxo *mic1-3* KO (KO) et la souche sauvage de type II (Pru). Des cellules mononuclées issues des ganglions mésentériques d'agneau et de mouton adulte non infectées *in vitro* servent de témoins (M).
   - en abscisse : « RH » : souche sauvage de Toxoplasma gondii de type I, « KO » : souche Toxo *mic 1-3* KO, « Pru » souche sauvage de type II, « M » : cellule de rate cultivées *in vitro* sans stimulant (témoin négatif)
   - en ordonnée : concentration d'IL-12 (UI/ml).
Figure 13-C : la figure 13-C illustre le dosage de l'interféron gamma (IFNγ) dans les cellules mononucléées issues de la rate d'agneau et de mouton adulte. Des échantillons de cellules mononuclées issues de la rate d'agneau (triangles gris) et de mouton adulte (carrés noirs) ont été infectés *in vitro* par trois souches distinctes de *Toxoplasma gondii* : la souche sauvage de type I (RH), la souche mutante de type I Toxo *mic1-3* KO (KO) et la souche sauvage de type II (Pru). Des cellules mononuclées issues de la rate d'agneau et de mouton adulte non infectées *in vitro* servent de témoins (M).
   - en abscisse : « RH » : souche sauvage de Toxoplasma gondii de type I, «KO»: souche Toxo *mic 1-3* KO, «Pru» souche sauvage de type II, « M » : cellule de rate cultivées *in vitro* sans stimulant (témoin négatif)
   - en ordonnée : concentration d'IFNγ (ng /ml).

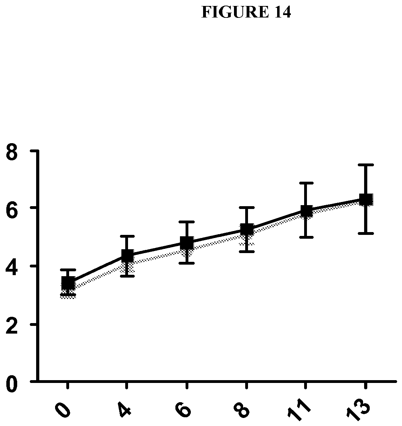

Figure 14 : la figure 14 illustre l'évolution de la masse corporelle des agneaux immunostimulés avec 10⁶ tachyzoïtes Toxo *mic1-3* KO, un jour après leur naissance (lot A - carré noir) en comparaison avec des agneaux témoins non immunostimulés (lot B - cercle gris).
   - en abscisse : durée de temps écoulé après stimulation des agneaux par Toxo *mic1-*3 KO (en jours)
   - en ordonnée : masse corporelle des agneaux (en kilogramme).

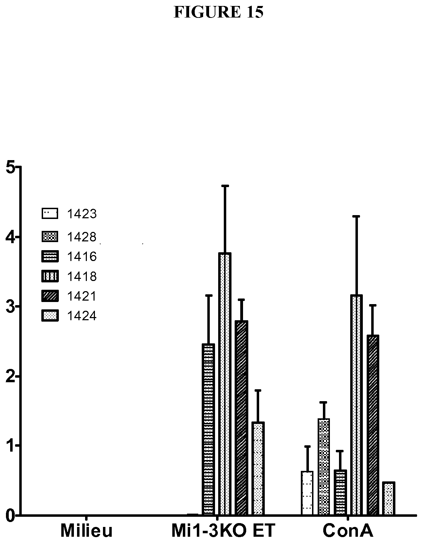

Figure 15 : la figure 15 illustre la production d'IFN-γ après restimulation avec de l'extrait total de la souche Toxo *mic1-3* KO de cellules mononuclées de rate d'agneaux immunostimulés avec 10⁶ tachyzoïtes Toxo *mic1-3* KO, un jour après leur naissance (Agneaux n° 1416 - 1418 - 1421 et 1424) ou d'agneaux témoins (Agneaux 1428 et 1423) et sacrifiés 15 jours après l'immunostimulation,
   - en abscisse : « milieux » : cellules de rate cultivées in vitro sans stimulant (témoin -), « Mic1-3 KO ET: cellules de rate restimulées par de l'extrait total parasitaire de la souche Toxo *mic1-3* KO et « ConA » : cellules de rate stimulées par la concanavaline A (témoin +)]
   - en ordonnée : concentration d'IFN-γ (en ng/ml).

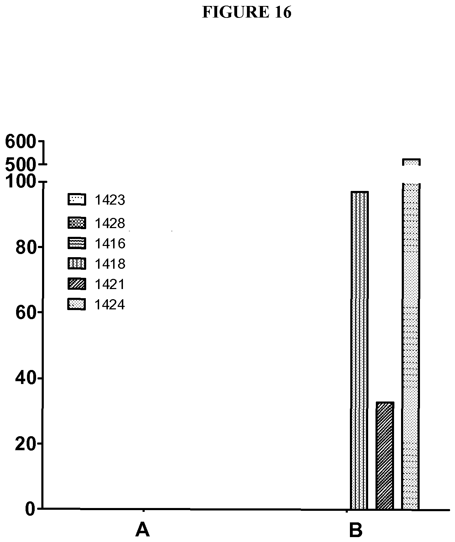

Figure 16 : la figure 16 illustre la production d'IFN-γ produits à partir de culture *ex vivo* de cellules de ganglions sub-iliaques et poplités d'agneaux immunostimulés avec 10⁶ tachyzoïtes Toxo *mic1-3* KO, un jour après leur naissance (Agneaux n° 1416 - 1418 - 1421 et 1424) ou d'agneaux témoins (Agneaux 1428 et 1423) et sacrifiés 15 jours après l'immunostimulation.
   - en abscisse : cellules de ganglions poplités (A) et cellules de ganglions sub-iliaques (B)
   - en ordonnée : concentration d'IFN-γ (en pg/ml).

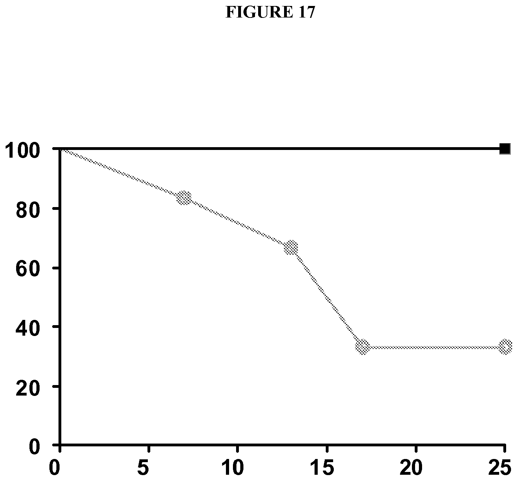

Figure 17 : la figure 17 illustre la survie des agneaux immunostimulés avec 10⁶ tachyzoïtes Toxo *mic1-3* KO, un jour après leur naissance et challengés avec 5.10⁶ oocystes de *C. parvum* (carré noir) et les agneaux témoins uniquement challengés (cercle gris). Les courbes de survie sont de type Kaplan-Meier.
   - en abscisse : durée de temps écoulé après infection des agneaux par *C. parvum* (en jours)
   - en ordonnée : survie des agneaux (en %).

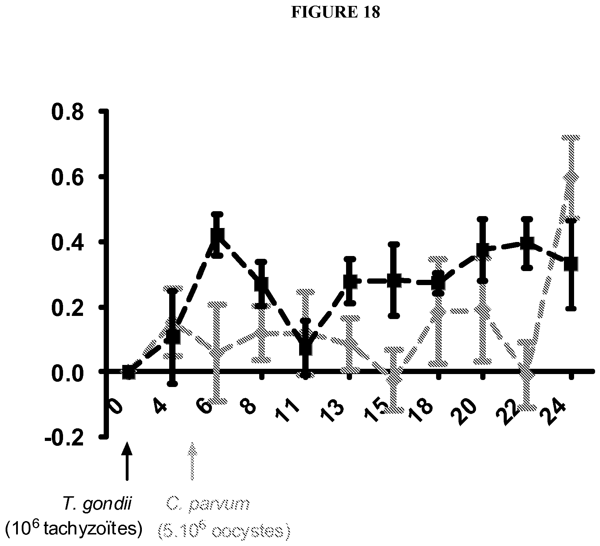

Figure 18 : la figure 18 illustre le gain de poids quotidien des agneaux immunostimulés avec 10⁶ tachyzoïtes Toxo *mic1-3* KO, un jour après leur naissance et challengés avec 5.10⁶ oocystes de *C. parvum* (carré noir) et les agneaux témoins uniquement challengés (losange gris).
   - en abscisse : durée de temps écoulé après stimulation des agneaux par Toxo *mic1-*3 KO (en jours)
   - en ordonnée : variation de la masse des agneaux (en kg).
   Le gain moyen de poids (GMQ) traduit la vitesse d'augmentation en fonction du temps selon la formule : GMQ poids = d Poids/d' Age.

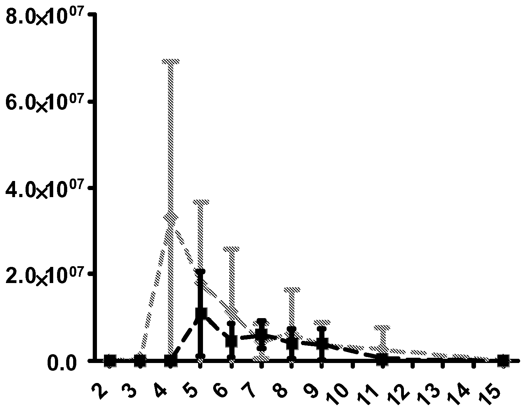

Figure 19 : la figure 19 illustre l'excrétion moyenne d'oocystes *C. parvum* par gramme d'excrément d'agneaux immunostimulés avec 10⁶ tachyzoïtes Toxo *mic1-3* KO, un jour après leur naissance et challengés avec 5.10⁶ oocystes de *C. parvum* (carré noir) et les agneaux témoins uniquement challengés (losange gris).
   - en abscisse : durée de temps écoulé après infection des agneaux par *C. parvum* (en jours)
   - en ordonnée : nombre d'oocystes de *C. parvum* par gramme d'excréments.

### Partie expérimentale

Afin d'élaborer la souche de *N. caninum* invalidée pour les gènes *ncmic1* et *ncmic3,* deux étapes de recombinaison homologue ont été effectuées. La première étape de recombinaison homologue permet l'obtention d'un simple mutant KO (souche Neo *ncmic3* KO). La deuxième étape de recombinaison homologue se fait dans la souche Neo *ncmic3* KO pour obtenir une souche doublement délétée (Neo *ncmicl-3* KO) (Figure 1).

### Exemple 1 : Construction de la souche mutante Neo ncmic3 KO

L'haploïdie du génome de *Neospora caninum* lors de la phase proliférative permet l'invalidation d'un gène en une seule recombinaison homologue.

Tous les tachyzoïtes de la souche NC1 de *Neospora caninum* utilisés ont été produits en fibroblastes humains (HFF) cultivés en milieu minimal de Dulbecco (DMEM) supplémenté par 10% de sérum de veau foetal (FCS), 2 mM glutamine, 50 U/mL de pénicilline et 50 µg/mL de streptomycine. Ils ont été récoltés après lyse mécanique des cellules hôtes et 3 passages en seringue 25G.

### a) Construction du plasmide pNcMic3KO-DHFR

Le plasmide pNcMic3KO-DHFR (Figure 2-A) contient le gène de sélection *DHFR* (dihydrofolate reductase) conférant une résistance à la pyriméthamine. Le gène de sélection *DHFR* est placé sous le contrôle du promoteur de l'α-tubuline de *Toxoplasma gondii* (promoteur *aTUB5*) pour permettre l'expression du gène dans le parasite. L'efficacité de ce promoteur hétérologue a été démontrée préalablement chez *N. caninum.* Cette cassette est encadrée par les régions homologues (5HR-NcMic3 et 3HR-NcMic3) des séquences flanquant le gène *ncmic3.* La cassette de sélection DHFR permet d'effectuer une sélection à la pyriméthamine.

La région 5'UTR du gène *ncmic3* a été amplifiée par PCR à partir de l'ADN génomique de la souche NC1 de *Neospora caninum.* Pour l'amplification, les amorces 5 HR NCmic3 F KpnI et 5 HR NCmic3 R ClaI (SEQ ID NO : 1 et SEQ ID NO : 2) permettent l'amplification de la région 5'UTR du gène *ncmic3* et la création de deux sites de restrictions qui ont été utilisés pour cloner le fragment 5HR en amont de la cassette de sélection DHFR dans le plasmide pT230 DHFR (KpnI en 5' et ClaI en 3' du fragment de PCR).

La région 3'UTR du gène *ncmic3* a été amplifiée par PCR à partir de l'ADN génomique de la souche NC1 de *Neospora caninum.* Pour l'amplification, les amorces 3 HR NCmic3 F XbaI et 3 HR NCmic3 R NotI (SEQ ID NO : 3 et SEQ ID NO : 4) permettent l'amplification de la région 3'UTR du gène *ncmic3* et la création de deux sites de restrictions qui ont été utilisés pour cloner le fragment 3HR en aval de la cassette de sélection DHFR dans le plasmide pT230 5HR-NcMic3-DHFR (XbaI en 5' et NotI en 3' du fragment de PCR). Les séquences des amorces figurent dans le Tableau I ci-dessous.

**Tableau I : Liste des amorces servant à l'intégration des séquences 5'UTR et 3'UTR du gène ncmic3. Les séquences des sites de restrictions sont soulignées.**

| **Nom de l'amorce** | **Séquence 5' → 3'** | **n° de séquence** |
|---|---|---|
| 5 HR NCmic3 F KpnI | CGCGGTACCCATGTGAATATGCTTTAACCGTGAC | SEQ ID NO: 1 |
| 5 HR NCmic3 R ClaI | CGCATCGATGAGCTATAACCCTTGGAAATGACTC | SEQ ID NO: 2 |
| 3 HR NCmic3 F XbaI | CGCTCTAGACATGCTGATGAAGAAGGGAAGT | SEQ ID NO: 3 |
| 3 HR NCmic3 R NotI | CGCGCGGCCGCTCTCTCCTGAAGTCTTCGAGACC | SEQ ID NO: 4 |

### b) Conditions d'électroporation et de sélection

50 µg du plasmide pNcMic3KO-DHFR purifié puis linéarisé par NotI ont été ajoutés à 5x10⁷ tachyzoïtes NC1 de *Neospora caninum* mis en suspension dans le milieu d'électroporation CYTOMIX contenant de l'ATP (3 mM) et du Glutathion (3 mM) (Van den Hoff et al., Nucleic Acid Research, Jun 11; 20(11):2902), et l'électroporation a été réalisée en cuvette d'écart 4 mm, dans un volume de 800 µL sur un appareil BioRad (Paramètres : 2000 V, 50 ohms, 25 µF, avec deux chocs éléctriques).

Après électroporation, les tachyzoïtes ont été déposés sur une monocouche de cellules HFF en culture. Pour la sélection des mutants, le milieu de culture est remplacé et supplémenté par l'agent de sélection (pyriméthamine 2 µM), 24h après l'électroporation. Trois passages en culture sont effectués dans ce milieu.

Après 16 jours de sélection, les parasites résistants sont clonés par dilution limite dans les puits de plaques de 96 puits de cellules HFF. Après amplification, les plages de lyse provoquées par le parasite sont recherchées. Les parasites sont repiqués et leur ADN génomique est extrait pour des analyses PCR. Ces analyses PCR doivent confirmer l'intégration du transgène mais doivent également permettre de différencier les parasites ayant intégré le transgène de manière aléatoire des parasites d'intérêt dont le gène *ncmic3* a été effectivement supprimé par recombinaison homologue.

### c) Analyse PCR

A partir de l'ADN génomique, des PCR ont été réalisées pour :
- étudier la taille du fragment d'ADN amplifié avec un jeu d'amorces de la PCR n°1: HR NCmic3 F (SEQ ID NO: 5) et HR NCmic3 R (SEQ ID NO: 6), présentes sur les séquences homologues. Avec une intégration aléatoire du transgène, deux fragments d'ADN de 2163 pb et de 3824 pb sont amplifiés alors qu'avec recombinaison homologue, seul un fragment de 3824 pb est amplifié. Avec les souches sauvages, seul un fragment de 2163 pb est amplifié.
- vérifier la présence / l'absence du gène *ncmic3* avec le jeu d'amorces de la PCR n°2: ORF NCmic3 F (SEQ ID NO: 7) et ORF NCmic3 R (SEQ ID NO: 8).
- et/ou vérifier la présence / l'absence de la cassette *DHFR* avec le jeu d'amorce de la PCR n°3: ORF DHFR F (SEQ ID NO: 9) et ORF DHFR R (SEQ ID NO: 10).

Les séquences des amorces et la taille des amplicons issus des différentes PCR figurent respectivement dans le Tableau II et le Tableau III ci-dessous.

**Tableau II : Liste des amorces ayant servi aux différentes PCR de validation de la construction de la souche mutante Neo ncmic3 KO.**

| **Nom de l'amorce** | **Séquence 5' → 3'** | **n° de séquence** | **n° de PCR** |
|---|---|---|---|
| HR NCmic3 F | GTCATCGACCGCCGGAACTAGTAGT | SEQ ID NO: 5 | 1 |
| HR NCmic3 R | GCAGAGGTTCTGCGTATCTAACACGG | SEQ ID NO: 6 | 1 |
| ORF NCmic3 F | TTTCCCTTCTAAACACAGTCG | SEQ ID NO: 7 | 2 |
| ORF NCmic3 R | CCTTCAGTGGTTCTCCATGAGT | SEQ ID NO: 8 | 2 |
| ORF DHFR F | CCTTCTCAGACAACGGGGTA | SEQ ID NO: 9 | 3 |
| ORF DHFR R | AGATCTTCACGCCCTTCTCA | SEQ ID NO: 10 | 3 |
| Integ NCmic3 F | GAAAGTGTCAGTGGTAGAGACTGC | SEQ ID NO: 11 | 4 et 6 |
| ORF NCmic3 R2 | CCTTCACTCGAGATCGCGCAAATGAGC | SEQ ID NO: 12 | 4 |
| ORF DHFR R2 | GGACCTCTGTACGAGACATGCCG | SEQ ID NO: 13 | 6 |
| Integ NCmic3 R | TGTTTACAGGTGATCCAGAAAAGG | SEQ ID NO: 14 | 5 et 7 |
| ORF NCmic3 F2 | GAATTTTGGGACAGGGGAAT | SEQ ID NO: 15 | 5 |
| ORF DHFR F2 | GTCTCTCGTTTTCCTCTCTTTTCGG | SEQ ID NO: 16 | 7 |

**Tableau III : Taille des amplicons (en paires de base) des différentes PCR de validation de la construction de la souche mutante Neo ncmic3 KO.**

| **n° de PCR** | **Neo *ncmic3* KO** | ***Neospora caninum* (NC1)** |
|---|---|---|
| 1 | 3824 | 2163 |
| 2 | - | 850 |
| 3 | 504 | - |
| 4 | - | 3127 |
| 5 | - | 3374 |
| 6 | 2890 | - |
| 7 | 3258 | - |

Les profils électrophorétiques des produits PCR sont présentés sur la Figure 3-A. Parmi les clones étudiés, certains clones présentaient une bande spécifique de *DHFR* (PCR 3) mais pas de bande spécifique de *ncmic3* (PCR 2). La PCR n°1 réalisée sur ces clones a mis en évidence une bande de 3824 pb spécifique d'un clone Neo *ncmic3* KO.

De nouvelles analyses PCR ont été effectuées sur ces clones d'intérêt avec de nouveaux jeux d'amorces. Ces PCR, dites d'intégration, permettent de valider le KO génétique en utilisant une amorce présente sur le génome en amont ou en aval des séquences flanquant le gène *ncmic3* et une deuxième amorce présente dans la cassette de sélection (gène *dhfr*) ou dans le gène d'intérêt (*ncmic3*) (Figure 3-B).

Dans la Figure 3-B, les PCR n°4 et n°5 permettent de montrer la présence de *ncmic3* au locus de *ncmic3.* La PCR n°4 est réalisée avec le jeu d'amorce Integ NCmic3 F (SEQ ID NO: 11) et ORF NCmic3 R2 (SEQ ID NO: 12). La PCR n°5 est réalisée avec le jeu d'amorce Integ NCmic3 R (SEQ ID NO: 14) et ORF NCmic3 F2 (SEQ ID NO: 15). On observe la présence de bandes pour la souche sauvage NC1 de *Neospora caninum* et l'absence de ces bandes pour la souche mutante Neo *ncmic3* KO. Dans la Figure 3-B, les PCR n°6 et n°7 permettent de montrer la présence de *DHFR* au locus de *ncmic3.* La PCR n°6 est réalisée avec le jeu d'amorce Integ NCmic3 F (SEQ ID NO: 11) et ORF DHFR R2 (SEQ ID NO: 13). La PCR n°7 est réalisée avec le jeu d'amorce Integ NCmic3 R (SEQ ID NO: 14) et ORF DHFR F2 (SEQ ID NO: 16). On constate l'absence de bandes pour la souche sauvage NC1 de *Neospora caninum* et présence de bandes pour la souche Neo *ncmic3* KO. Il faut noter la présence d'une bande aspécifique pour la PCR n°6 à environ 1000 pb.

L'ensemble des résultats de PCR démontre que la recombinaison homologue a bien eu lieu et que la souche mutante Neo *ncmic3* KO est bien délétée du gène *ncmic3.*

### d) Analyse en immunofluoresence

L'analyse a été réalisée en immunofluorescence. 24h avant l'analyse en immunofluorescence, 5x10⁵ parasites sont déposés dans un puits de p24 contentant un coverslip recouvert d'un tapis cellulaire de cellules HFF.

Les cellules infectées par les parasites sont lavées 2 fois au PBS 1X puis fixées au paraformaldéhyde (3,7% dans du PBS 1X) pendant 30 min. Après 3 lavages au PBS 1X, les cellules sont perméabilisées avec une solution de Triton (0.1% dans du PBS 1X) pendant 5 minutes. Après 3 lavages au PBS 1X, une étape de saturation est effectuée avec une solution de PBS 1X/SVF 10% pendant 30 min. Les cellules sont ensuite incubées avec l'anticorps primaire dilué dans une solution de PBS/SVF 2% pendant 1 heure, lavées 3 fois puis incubées avec l'anticorps secondaire dilué dans une solution de PBS/SVF 2% pendant 1 heure. Après 2 lavages au PBS1X, les coverslips sont montés sur lame avec de l'immumount et observés au microscope à fluorescence.

L'anticorps primaire utilisé est un anticorps qui permet de détecter l'expression de la protéine NcMIC3 dans le parasite (anticorps primaire : anticorps de lapin anti-mic3 et anticorps secondaire commercial : Alexa fluor® 594 chèvre anti-lapin, Life technologies réf. A-11012).

Pour la souche sauvage NC1 de *Neospora caninum,* on observe une fluorescence rouge au pole apical du parasite révélant la présence de la protéine NcMIC3 (Figure 4A), alors que pour la souche mutante Neo *ncmic3* KO, on n'observe pas de fluorescence au pole apical du parasite démontrant l'absence de la protéine NcMIC3 (Figure 4B).

### Exemple 2 : Construction de la souche mutante Neo ncmic1 KO

### a) Construction du plasmide pNc mic1KO-CAT-GFP

Le plasmide pNcMic1KO-CAT-GFP (Figure 2-B) contient une cassette de sélection *CAT-GFP* codant pour une protéine de fusion permettant à la fois la résistance au chloramphénicol (CAT) et une fluorescence verte (GFP : Green Fluorescent Protein). Celle-ci est placée sous le contrôle du promoteur de l'α-tubuline de *Toxoplasma gondii* pour permettre l'expression du gène dans le parasite. De part et d'autre de la cassette, les régions homologues des séquences flanquant le gène *ncmic1* ont été clonées.

La région 3'UTR du gène *ncmic1* a été amplifiée par PCR à partir de l'ADN génomique de la souche NC1 de *Neospora caninum.* Pour l'amplification, les amorces 3 HR NCmic1 F KpnI et 3 HR NCmic1 R HindIII (SEQ ID NO: 17 et SEQ ID NO: 18) permettent l'amplification de la région 3'UTR du gène *ncmic1* et la création de deux sites de restrictions qui ont été utilisés pour cloner le fragment 3HR en amont de la cassette de sélection CAT-GFP dans le plasmide pT230 CAT-GFP (KpnI en 5' et HindIII en 3' du fragment de PCR).

La région 5'UTR du gène *ncmic1* a été amplifiée par PCR à partir de l'ADN génomique de la souche NC1 de *Neospora caninum.* Pour l'amplification, les amorces 5 HR NCmic1 F BamHI et 5 HR NCmic1 R NotI (SEQ ID NO: 19 et SEQ ID NO: 20) permettent l'amplification de la région 5'UTR du *gène ncmic1* et la création de deux sites de restrictions qui ont été utilisés pour cloner le fragment 5HR en aval de la cassette de sélection CAT-GFP dans le plasmide pT230 3HRNcMic1CAT-GFP (BamHI en 5' et NotI en 3' du fragment de PCR). Les séquences des amorces figurent dans le Tableau IV ci-dessous.

**Tableau IV : Liste des amorces servant à l'intégration des séquences 5'UTR et 3'UTR du gène ncmic 1. Les séquences des sites de restrictions sont soulignées.**

| **Nom de l'amorce** | **Séquence 5' → 3'** | **n° de séquence** |
|---|---|---|
| 3 HR NCmic1 F KpnI | CGCGGTACCAGGCAGAAGTAAAGA AGGTTCCTC | SEQ ID NO: 17 |
| 3 HR NCmic1 R HindIII | CGCAAGCTTTGATCACGCAAGAAA AGAAGC | SEQ ID NO: 18 |
| 5 HR NCmic1 F BamHI | CGCGGATCCCATTTGTAGATACGGT TGCACAC | SEQ ID NO: 19 |
| 5 HR NCmic1 R NotI | CGCGCGGCCGCACATTCAGACGGC | SEQ ID NO: 20 |
| | AGAACTCTG | |

### b) Conditions d'électroporation et de sélection

50 µg du plasmide pNcMic1KO-CAT-GFP purifié puis linéarisé par KpnI doivent être ajoutés à 5x10⁷ tachyzoïtes NC1 mis en suspension dans le milieu d'électroporation CYTOMIX contenant de l'ATP (3 mM) et du Glutathion (3 mM) (Van den Hoff et al., Nucleic Acid Research, Jun 11; 20(11):2902), et l'électroporation doit être réalisée en cuvette d'écart 4 mm, dans un volume de 800 µL sur un appareil BioRad (Paramètres : 2000 V, 50 ohms, 25 µF, avec deux chocs électriques).

Après électroporation, les tachyzoïtes seront déposés sur une monocouche de cellules HFF en culture. Pour la sélection des mutants, le milieu de culture sera remplacé et supplémenté par l'agent de sélection (chloramphénicol 50 µM), 24h après l'électroporation. Trois passages en culture doivent être effectués dans ce milieu.

Après 15 jours de sélection, les parasites résistants seront clonés par dilution limite dans les puits de plaques de 96 puits de cellules HFF. Après amplification, les plages de lyse provoquées par le parasite seront recherchées. Les parasites seront repiqués et leur ADN génomique sera extrait pour des analyses PCR.

### c) Analyse PCR

Les séquences des amorces et la taille attendue des amplicons issus des différentes PCR figurent respectivement dans le Tableau V et le Tableau VI ci-dessous.

**Tableau V : Liste des amorces servant aux différentes PCR de validation de la construction des souches mutantes Neo ncmic1 KO.**

| **Nom de l'amorce** | **Séquence 5' → 3'** | **n° de séquence** | **n° de PCR** |
|---|---|---|---|
| Integ NCmic1 F | CCGAGCAAGTTAGCAAGTCC | SEQ ID NO: 21 | 1 et 3 |
| ORF CATGFP R | CCGTTTGGTGGATGTCTTCT | SEQ ID NO: 22 | 1 |
| ORF CATGFP F | GCATCGACTTCAAGGAGGAC | SEQ ID NO: 23 | 2 |
| Integ NCmic1 R | CTTGTCCGTCACATCGTTTG | SEQ ID NO: 24 | 2 et 4 |
| ORF NCmic1 R | TTCTCCAGGCACTCACCTCT | SEQ ID NO: 25 | 3 |
| ORF NCmic1 F | AGCTTCCAACAACGAGAGGA | SEQ ID NO: 26 | 4 |
| ORF NCmic1 F2 | CCCAGGATATCGTTTGTTGC | SEQ ID NO: 27 | 5 |
| ORF NCmic1 R2 | CTTCTGATGCACGGAACTGA | SEQ ID NO: 28 | 5 |
| ORF CATGFP F2 | CCTGAAGTTCATCTGCACCA | SEQ ID NO: 29 | 6 |
| ORFCATGFP R2 | GTAGTGGTTGTCGGGCAGCA | SEQ ID NO: 30 | 6 |

**Tableau VI : Taille des amplicons (en paires de base) des différentes PCR de validation de la construction de la souche mutante Neo ncmic1 KO.**

| **n° de PCR** | **Neo *ncmic1* KO** | ***Neospora caninum* (NC1)** |
|---|---|---|
| 1 | 3359 | - |
| 2 | 3421 | - |
| 3 | - | 3746 |
| 4 | - | 3046 |
| 5 | - | 449 |
| 6 | 472 | - |

### Exemple 3 : Construction de la souche mutante Neo ncmic1-3 KO

### a) Construction du plasmide pNc mic1KO-CAT-GFP

La construction du plasmide pNcMic1KO-CAT-GFP est décrite dans l'exemple 2 (2a).

### b) Conditions d'électroporation et de sélection

50 µg du plasmide pNcMic1KO-CAT-GFP purifié puis linéarisé par KpnI ont été ajoutés à 5x10⁷ tachyzoïtes Neo *ncmic3* KO mis en suspension dans le milieu d'électroporation CYTOMIX contenant de l'ATP (3 mM) et du Glutathion (3 mM) (Van den Hoff et al., Nucleic Acid Research, Jun 11; 20(11):2902), et l'électroporation a été réalisée en cuvette d'écart 4 mm, dans un volume de 800 µL sur un appareil BioRad (Paramètres : 2000 V, 50 ohms, 25 µF, avec deux chocs éléctriques).

Après électroporation, les tachyzoïtes ont été déposés sur une monocouche de cellules HFF en culture. Pour la sélection des mutants, le milieu de culture est remplacé et supplémenté par l'agent de sélection (chloramphénicol 50 µM), 24h après l'électroporation. Trois passages en culture sont effectués dans ce milieu.

Après 15 jours de sélection, les parasites résistants sont clonés par dilution limite dans les puits de plaques de 96 puits de cellules HFF. Après amplification, les plages de lyse provoquées par le parasite sont recherchées. Les parasites sont repiqués et leur ADN génomique est extrait pour des analyses PCR.

### c) Analyse PCR

Les séquences des amorces et la taille des amplicons issus des différentes PCR figurent respectivement dans le Tableau VII et le Tableau VIII ci-dessous.

**Tableau VII : Liste des amorces ayant servi aux différentes PCR de validation de la construction des souches mutantes Neo ncmic3 KO et Neo ncmicl-3 KO.**

| **Nom de l'amorce** | **Séquence 5' → 3'** | **n° de séquence** | **n° de PCR** |
|---|---|---|---|
| Integ NCmic1 F | CCGAGCAAGTTAGCAAGTCC | SEQ ID NO: 21 | 1 et 3 |
| ORF CATGFP R | CCGTTTGGTGGATGTCTTCT | SEQ ID NO: 22 | 1 |
| ORF CATGFP F | GCATCGACTTCAAGGAGGAC | SEQ ID NO: 23 | 2 |
| Integ NCmic1 R | CTTGTCCGTCACATCGTTTG | SEQ ID NO: 24 | 2 et 4 |
| ORF NCmic1 R | TTCTCCAGGCACTCACCTCT | SEQ ID NO: 25 | 3 |
| ORF NCmic1 F | AGCTTCCAACAACGAGAGGA | SEQ ID NO: 26 | 4 |
| Integ NCmic3 F | GAAAGTGTCAGTGGTAGAGACTGC | SEQ ID NO: 11 | 5 et 7 |
| ORF NCmic3 R2 | CCTTCACTCGAGATCGCGCAAATGAGC | SEQ ID NO: 12 | 5 |
| ORF DHFR R2 | GGACCTCTGTACGAGACATGCCG | SEQ ID NO: 13 | 7 |
| Integ NCmic3 R | TGTTTACAGGTGATCCAGAAAAGG | SEQ ID NO: 14 | 6 et 8 |
| ORF NCmic3 F2 | GAATTTTGGGACAGGGGAAT | SEQ ID NO: 15 | 6 |
| ORF DHFR F2 | GTCTCTCGTTTTCCTCTCTTTTCGG | SEQ ID NO: 16 | 8 |
| ORF NCmic1 F2 | CCCAGGATATCGTTTGTTGC | SEQ ID NO: 27 | 9 |
| ORF NCmic1 R2 | CTTCTGATGCACGGAACTGA | SEQ ID NO: 28 | 9 |
| ORF CATGFP F2 | CCTGAAGTTCATCTGCACCA | SEQ ID NO: 29 | 10 |
| ORFCATGFP R2 | GTAGTGGTTGTCGGGCAGCA | SEQ ID NO: 30 | 10 |
| ORF NCmic3 F | TTTCCCTTCTAAACACAGTCG | SEQ ID NO: 7 | 11 |
| ORF NCmic3 R | CCTTCAGTGGTTCTCCATGAGT | SEQ ID NO: 8 | 11 |
| ORF DHFR F | CCTTCTCAGACAACGGGGTA | SEQ ID NO: 9 | 12 |
| ORF DHFR R | AGATCTTCACGCCCTTCTCA | SEQ ID NO: 10 | 12 |

**Tableau VIII : Taille des amplicons (en paires de base) des différentes PCR de validation de la construction des souches mutante Neo ncmic3 KO et Neo ncmic1-3 KO.**

| **n° de PCR** | **Neo *ncmic1-3* KO** | ***Neospora caninum* (NC1)** | **Neo *ncmic3* KO** |
|---|---|---|---|
| 1 | 3359 | - | - |
| 2 | 3421 | - | - |
| 3 | - | 3746 | 3746 |
| 4 | - | 3046 | 3046 |
| 5 | - | 3127 | - |
| 6 | - | 3374 | - |
| 7 | 2890 | - | 2890 |
| 8 | 3258 | - | 3258 |
| 9 | - | 449 | 449 |
| 10 | 472 | - | - |
| 11 | - | 850 | - |
| 12 | 504 | - | 504 |

Dans la Figure 5, la PCR n°1 est réalisée avec le jeu d'amorces Integ NCmic1 F(SEQ ID NO: 21) et ORF CATGFP R (SEQ ID NO: 22). Le PCR 2 est réalisé avec le jeu d'amorces ORF CATGFP F (SEQ ID NO: 23) et Integ NCmic1 R (SEQ ID NO: 24). La PCR n°3 est réalisée avec le jeu d'amorces Integ NCmic1 F (SEQ ID NO: 21) et ORF NCmic1 R (SEQ ID NO: 25). La PCR n°4 est réalisée avec le jeu d'amorces Integ NCmic1 R (SEQ ID NO: 24) et ORF NCmic1 F (SEQ ID NO: 26). La PCR n°5 est réalisée avec le jeu d'amorces Integ NCmic3 F (SEQ ID NO: 11) et ORF NCmic3 R2 (SEQ ID NO: 12). La PCR n°6 est réalisée avec le jeu d'amorces Integ NCmic3 R (SEQ ID NO: 14) et ORF NCmic3 F2 (SEQ ID NO: 15). La PCR n°7 est réalisée avec le jeu d'amorces Integ NCmic3 F (SEQ ID NO: 11) et ORF DHFR R2 (SEQ ID NO: 13). La PCR n°8 est réalisée avec le jeu d'amorces Integ NCmic3 R (SEQ ID NO: 14) et ORF DHFR F2 (SEQ ID NO: 16). La PCR n°9 est réalisée avec le jeu d'amorces ORF NCmic1 F2 (SEQ ID NO: 27) et ORF NCmic1 R2 (SEQ ID NO: 28). La PCR n°10 est réalisée avec le jeu d'amorces ORF CATGFP F2 (SEQ ID NO: 29) et ORF CATGFP R2 (SEQ ID NO: 30). La PCR n°11 est réalisée avec le jeu d'amorces ORF NCmic3 F (SEQ ID NO: 7) et ORF NCmic3 R (SEQ ID NO: 8). La PCR n°12 est réalisée avec le jeu d'amorces ORF DHFR F (SEQ ID NO: 9) et ORF DHFR R (SEQ ID NO: 10).

Les analyses électrophorétiques des produits de PCR montrent que la souche Neo *ncmic1-3* KO ne possède plus les gènes *ncmic1* et *ncmic3* (puits 3, 4, 5, 6, 9 et 11, Figure 5) et possède bien les gènes *dhfr* et *cat-gfp* (puits 1, 2, 7, 8, 10 et 12, Figure 5) validant ainsi l'obtention de la souche Neo *ncmic1-3* KO. L'ensemble des résultats de PCR démontre que la recombinaison homologue a bien eu lieu et que la souche Neo *ncmic1-3* KO est bien délétée des gènes *ncmic1* et *ncmic3.*

### d) Analyse en immunofluoresence

L'analyse en immunofluorescence a uniquement été réalisée par observation directe de la fluorescence du parasite (Figure 6).

Les parasites des deux souches mutantes sont visualisés en lumière directe (images A et C). Un même champ microscopique est visualisé en fluorescence. La fluorescence verte, due à l'expression de la protéine recombinante chimérique CAT-GFP n'est détectée que dans la souche mutante Neo *ncmic1-3* KO (image D) suite à l'insertion de la cassette CAT-GFP. A l'inverse, la souche Neo *ncmic3* KO, qui ne possède pas de cassette CAT-GFP, n'exprime pas la protéine CAT-GFP et par conséquent ne présente pas de fluorescence (image B).

### Exemple 4 : immunostimulation de souriceaux par le mutant Toxo mic1-3 KO

### 1 - Protocole expérimental

### 1.1 - Animaux

L'immunostimulation est réalisée sur des souriceaux C57BL/6 âgés de 3 jours. Ces souriceaux ont été obtenus et sont élevés au sein du Centre INRA de Nouzilly (Indre et Loire, France). Les souriceaux sont maintenus tout au long de l'expérimentation en animalerie de niveau de confinement 2 afin de limiter au mieux le risque de contamination externe.

### 1.2 - Souche de T. gondii

### 1.2.1 - Souche Toxo mic1-3 KO

La souche mutante de *Toxoplasma gondii* dont les gènes codant pour les protéines MIC1 et MIC3 ont été invalidés (appelée souche Toxo *mic1-3* KO) est entretenue par passages successifs sur une lignée de fibroblastes de prépuce humain (HFF) cultivés dans du milieu DMEM supplémenté par 10% de sérum de veau foetal (FCS), 2 mM glutamine, 50 U/mL de pénicilline et 50 µg/mL de streptomycine.

### 1.2.2 - Souche RH

La souche sauvage RH de *Toxoplasma gondii,* dont est dérivée la souche Toxo *mic1-3* KO, est également entretenue par passages successifs sur une lignée de fibroblastes de prépuce humain (HFF) cultivés dans du milieu DMEM supplémenté par 10% de sérum de veau foetal (FCS), 2 mM glutamine, 50 U/mL de pénicilline et 50 µg/mL de streptomycine.

Pour la préparation de l'extrait parasitaire total, les tachyzoïtes de la souche RH sont lavés, soniqués à 60 watt/s, trois fois durant 10 min et centrifugés à 2000 g pendant 30 min à 4°C. Le surnageant est concentré et aliquoté. La concentration est déterminée par un dosage BCA, en utilisant comme standard de la BSA (Bovine Serum Albumin). Les aliquots sont conservés à -20°C.

### 1.3 - Immunostimulation

Des lots de souriceaux C57BL/6 âgés de 3 jours ont été traités comme suit :
- 6 souriceaux (lot A) ont servi de lot témoin non vacciné,
- 6 souriceaux (lot B) ont reçu le mutant Toxo *mic1-3* KO
   A J0, les souriceaux du lot A ont reçu 20 tachyzoïtes de la souche Toxo *mic1-3* KO par voie intrapéritonéale.
   A J3 post-immunostimulation, 3 souriceaux de chaque lot ont été sacrifiés pour l'étude de la réponse immunitaire humorale, de la réponse inflammatoire et de la parasitémie.
   A J9 post-immunostimulation, les 3 souriceaux restants de chaque lot ont été sacrifiés pour l'étude de la réponse immunitaire humorale, de la réponse inflammatoire et de la parasitémie.

### 1.4 - Réponse immunitaire humorale

La réponse immunitaire humorale a été étudiée en évaluant, par ELISA, la cinétique d'apparition des anticorps IgM spécifiques anti-*Toxoplasma gondii* dans le sérum.
Les sera sont prélevés au moment du sacrifice des souriceaux à J3 et à J9 post-infection. Le prélèvement est laissé 10 min à température ambiante pour permettre la formation du caillot. Le sérum est récupéré en centrifugeant les prélèvements à 2000 rpm pendant 10 min à +20°C. Le surnageant est aliquoté dans un tube propre et conservé à -20°C.

L'extrait total de la souche RH de *Toxoplasma gondii,* obtenu comme décrit ci-dessus, est utilisé pour sensibiliser les puits à fond plat de plaques de microtitration (Nunc). 100 µL d'extrait (à une concentration de 10 µg/mL en tampon carbonate à 50 mM et pH = 9,6) sont déposés dans chaque puits. Après une nuit à +4°C, trois lavages sont effectués en tampon PBS additionné de Tween-20 0,05% (PBS-T).

Les sites non spécifiques sont saturés par incubation des plaques pendant 1h30 à 37°C sous atmosphère humide avec du PBS à 4% de BSA.

100 µL de chaque échantillon de sérum dilué en PBS-T (dilution au 1/50) sont déposés et incubés pendant 1h à 37°C sous atmosphère humide.

Après deux séries de trois lavages, 100 µL d'anti-IgM de souris couplé à la phosphatase alcaline (PAL ; Sigma), dilué au 1/5000 en PBS-T sont déposés et incubés pendant 1h30 à 37°C sous atmosphère humide. Deux nouvelles séries de trois lavages sont effectuées. La révélation est effectuée à l'aide de 100 µL de paranitrophénylphosphate (PNPP) à 1 mg/ mL en DEA-HCL.

La lecture se fait après 10 à 20 minutes d'incubation, sur un lecteur de plaques (Wallac 1420 Multilabel counter) à la longueur d'onde ë= 405 nm.

Le seuil de positivité admis a été déterminé en fonction des valeurs d'absorbance (DO) des souriceaux du lot témoin : il est fixé à 0,23 de DO pour une dilution de sérum au 1/50.

### 1.5 - Etude de la réponse inflammatoire

L'analyse de la réponse inflammatoire des souriceaux au niveau de l'intestin a été quantifiée par PCR quantitative (PCRq) par amplification des gènes codant pour l'IL-12 et l'IFN-γ. L'IL-12 est une cytokine produite en réponse à l'intrusion d'un pathogène qui stimule la sécrétion de l'IFN-γ, cytokine produite par les cellules de l'immunité en réponse à une inflammation sur le site de l'infection du pathogène.

Après sacrifice des souriceaux à J3 et J9, l'intestin est prélevé et l'iléon (1cm au dessus du caecum) est utilisé pour l'analyse par PCRq. Le tissu est incubé dans 1 mL de Trizol® (Invitrogen) puis est broyé au thurax, incubé à température ambiante pendant 5 min et centrifugé pendant 10 minutes à 12 000 g. Le surnageant est alors récupéré et mélangé par une dizaine de refoulements successifs à la pipette.

Une fois les complexes nucléoprotéiques dissociés par le Trizol®, l'ARN est isolé de l'ADN et des protéines par le chloroforme. Un millilitre de chloroforme est ajouté au surnageant et le mélange Trizol®/chloroforme est agité fortement pendant 15 secondes, puis incubé à température ambiante et enfin centrifugé à 12 000 g pendant 15 minutes à 4°C. Après la centrifugation, le mélange est séparé en une phase organique (phénol/chloroforme, rose, phase inférieure), une interphase (voile blanc, ADN et débris cellulaire) et une phase aqueuse (phase supérieure) contenant les ARN totaux.

La phase aqueuse contenant les ARN totaux est récupérée et 500 µL d'isopropanol sont ajoutés pour précipiter l'ARN. La solution est agitée, incubée à température ambiante durant 10 min et centrifugée à 12000 g pendant 10 min à 4°C. Le culot obtenu est isolé puis lavé avec 1 mL d'éthanol 75% (éthanol absolu dilué dans de l'eau DEPC 0,1% et conservé à -20°C), agité et centrifugé à 7500 g à 4°C pendant 10 min. Le culot est séché pendant 10 min sous une hotte chimique dans la glace. Enfin l'ARN est repris dans environ 20 µL d'eau DEPC 0,1%.

La qualité de l'extraction de l'ARN est vérifiée par électrophorèse sur gel d'agarose 1% et par le calcul du ratio direct entre l'absorbance à une longueur d'onde de 260 nm et celle à une longueur d'onde de 280 nm. Ce ratio doit être proche de 2. Enfin, le rendement d'extraction de l'ARN est quantifié à l'aide d'un spectrophotomètre.

Deux microgrammes d'ARN sont incubés avec 1 µL d'Oligo dT (Eurogentec 133pmole/µl) dans un volume final de 11 µL à 65°C pendant 10 min, puis 2 min dans la glace. Une fois les oligonucléotides dT fixés sur la queue polyA, la solution d'ARN est incubée avec 2 µL de dNTP (dATP, dTTP, dGTP, dCTP à 20 mM chacun), 4 µL de tampon de transcriptase inverse (5X Eurogentec ; 250 mM Tris-HCL (pH 8,3) ; 375 mM KCl ; 50 mM DTT ; 15 mM MgCl2) et 0,4 µL de MuMLV (25 U/µL ; 50 mM Tric-HCL (pH 8,3) ; 1 mM EDTA, 0,1% Triton X-100, 0,1 M NaCl ; 5 mM DTT ; 50% (v/v) Glycérol) dans un volume final de 20 µL pendant 1H30 à 37°C. La réverse transcriptase est ensuite inhibée à 85°C pendant 10 min.

Dans le cas présent, l'expression des gènes codant pour les protéines IL-12 et IFN-y, exprimés lors de la réponse inflammatoire a été quantifiée.
Pour l'amplification, le couple d'amorces SEQ ID NO : 31 (5'-CTCACATCTGCTGCTCCACAA-3') et SEQ ID NO : 32 (5'-GACGCCATTCCACATGTCACT-3') a été utilisé pour l'IL-12, le couple d'amorce SEQ ID NO : 33 (5'-TCTTCTTGGATATCTGGAGGAA-3') et SEQ ID NO : 34 (5'-AGCTCATTGAATGCTTGGCGCTG-3') a été utilisé pour doser l'IFNγ et le couple d'amorce SEQ ID NO : 35 (5'-GGATACAGGCCAGACTTTGTTG-3') et SEQ ID NO : 36 (5'-GAGGGTAGGCTGGCCTATAG-3') a été utilisé pour doser le gène murin de référence HPRT.

Deux microlitres d'ADNc dilué au 1/10e de la réaction de reverse transcription sont incubés avec 0,3 µL de l'amorce 5' (25 µM) ; 0,3 µL de l'amorce 3' (25 µM) ; 7,5 µL de Mix PCR (BioRad) dans un volume final de 15 µL. Les conditions choisies pour la réaction de PCR sont les suivantes : 1) dénaturation à 95°C pendant 5 minutes, 2) dénaturation à 95°C pendant 10 secondes, 3) appariement et élongation à 62°C pour l'IL-12, l'IFNγ et le HPRT pendant 15 secondes, 4) reprise du cycle à partir de l'étape 2 : 39 fois, 5) Courbe de fusion de 55°C à 95°C pour vérifier la présence au non des dimères.

### 1.6 - Etude de l'état d'infection des souriceaux

Le niveau d'infection des souriceaux a été analysé par trois techniques différentes : 1) dissémination tissulaire des tachyzoïtes sur cellules HFF, 2) immuno-histologie sur coupes d'intestin de souriceaux infectés et 3) PCR à partir d'iléon des souriceaux des lots A et B.

### 1.6.1 - Dissémination tissulaire des tachyzoïtes sur cellules HFF

Les cellules HFF sont déposées en plaque 24 puits une semaine avant le dépôt des organes à raison de 1x10⁴ cellules/puits. Les cellules sont cultivées dans 1 mL de milieu de culture cellulaire DMEM supplémenté par 10% de sérum de veau foetal (FCS), 2 mM glutamine, 50 U/mL de pénicilline et 50 µg/mL de streptomycine.

Après le sacrifice, la rate des souriceaux des lots 1 et 2 est prélevée et broyée dans 2 mL de PBS 1X. Dix microlitres de broyat sont déposés par puits contenant les cellules HFF. Vingt-quatre heures après le dépôt des broyats de rate, les cellules sont lavées au DMEM puis 1 mL de milieu propre est déposé dans chaque puits. Les cellules sont incubées à 37°C, 5% CO₂ jusqu'à détection des plages de lyse révélant la présence de tachyzoïtes Toxo *mic1-3* KO.

### 1.6.2 - Immuno-histologie sur coupes d'intestin de souriceaux infectés

L'intestin des souriceaux sacrifiés est enroulé en escargot (swiss-roll) puis maintenu sous cette forme à l'aide de papier plié autour du swiss-roll et agrafé. Les échantillons sont fixés dans une solution de paraformaldhéhyde 4%, dilué dans du PBS 1X (pH = 7) et mis à incuber durant 8 heures à 4°C. Les tissus sont ensuite lavés en PBS 1X puis incubés à 4°C durant 8h dans du PBS 1X propre. Cette dernière étape est renouvelée une seconde fois. Les tissus sont incubés durant 8 heures à 4°C, dans une solution de sucrose 30% diluée dans du PBS 1X et filtrée. Enfin les tissus sont transférés dans des moules de taille adéquate rempli de milieu d'enrobage OCT. Après 5 minutes d'incubation dans l'OCT, les échantillons sont congelés à l'aide de carboglace et stockés à -80°C.

La découpe des échantillons d'intestin est alors effectuée à l'aide d'un cryostat qui maintient l'échantillon à -20°C. Des coupes histologiques de 7 µm d'épaisseur sont effectuées puis déposées sur lame par force électrostatique. Les lames sont stockées à -80°C.
Les lames de coupes histologiques sont décongelées puis laisser sécher à température ambiante durant 1h. La zone de l'échantillon sur la lame est délimitée avec un stylo à encre hydrophobe « Dakocitamation pen ». Les coupes histologiques d'intestin sont perméabilisées avec 50 µL d'une solution de PBS 1X, Mg²⁺, Ca²⁺ free, Triton X-100 et BSA 1% à température ambiante et en chambre humide durant 10 min. La solution de perméabilisation est retirée par aspiration et les échantillons sont saturés dans 50 µL d'une solution de PBS 1X, Mg²⁺, Ca²⁺ free, Triton X-100 et BSA 10% à température ambiante et en chambre humide durant 1h. Après avoir aspiré la solution de saturation, 50 µL de sérum polyclonal de lapin anti-SAG1 de *Toxoplasma gondii* dilué au 1/100e dans une solution de PBS 1X, Mg²⁺, Ca²⁺ free, Triton X-100 et BSA 1%, sont déposés par échantillon. Les échantillons sont incubés à 4°C, en chambre humide durant 8 h. Deux lavages de 5 minutes en PBS 1X, Mg²⁺, Ca²⁺ free, Triton X-100 et BSA 1% sont réalisés. 50 µL d'anticorps Swine anti-lapin couplé à l'isothiocyanate de fluorescéine dilués au 1/20e dans une solution de 1X, Mg²⁺, Ca²⁺ free, Triton X-100 et BSA 1% sont déposés par échantillon. Les échantillons sont incubés en présence de l'anticorps secondaire couplé, à température ambiante, en chambre humide durant 1h20. Deux lavages de 5 minutes en PBS 1X, Mg²⁺, Ca²⁺ free, Triton X-100 et BSA 1% sont réalisés puis un dernier lavage des échantillons est effectué en eau distillée stérile. Les lames sont séchées puis une goutte de fluoromount G est déposée sur chaque échantillon. Enfin, les échantillons sont montés entre lame et lamelle.

### 1.6.3 - Détection de SAG-1

Une PCR a été effectuée à partir des ADNc obtenus par réaction de reverse transcription (cf. paragraphe 1.5). Le gène SAG-1 spécifique du parasite *Toxoplasma gondii* a été amplifié par PCR avec les amorces SEQ ID NO : 37 (5'-CTGCACCACTTCATTATTTCTTCTG-3') et SEQ ID NO : 38 (5'-ACTCACGCGACACAAGCTG-3').

2 µL d'ADNc sont incubés avec 1 µL d'amorce 5' (10 µM) ; 1 µL d'amorce 3' (10 µM) ; 25 µL de GoTaq®Green Master Mix (2X, Promega) dans un volume final de 50 µL. Les conditions choisies pour la PCR sont les suivantes : 1) dénaturation à 94°C pendant 5 min, 2) dénaturation à 94°C pendant 30 sec, 3) appariement à 60°C pendant 30 sec, 4) élongation à 72°C pendant 1 min, 5) reprise du cycle à partir de l'étape 2 : 34 fois, 6) élongation à 72°C durant 5 min. La présence du parasite *Toxoplasma gondii* au niveau de l'intestin est vérifiée par un fragment amplifié de 1001 paires de bases.

### 2 - Résultats

### 2.1 - Etude de la réponse humorale post-immunostimulation

Les résultats des tests ELISA à J3 et J9 post infection pour les sera des lots de souriceaux témoins et immunostimulés par la souche Toxo *mic1-3* KO sont représentés sur la Figure 7. Les souriceaux du lot témoin (A) n'ont pas développé de réponse humorale à J3 et à J9 post-infection contrairement à 2 souriceaux sur 3 du lot immunostimulé par la souche Toxo *mic1-3* KO (B) qui ont produit des anticorps IgM anti-toxoplasmique à 9 jours post-infection ; il s'agit des souriceaux n° 4 et n° 6.

### 2.2 - Etude de la réponse inflammatoire post-immunostimulation

Les résultats des essais de PCR quantitative de l'expression du gène codant pour l'IFN-γ et le gène codant pour l'IL-12 réalisés à J9 post-immunostimulation sont représentés respectivement sur les Figures 8-A et 8-B. Les souriceaux du lot témoin (A) n'ont pas développé de réponse inflammatoire (sécrétion IL-12 et IFN-γ) à J9 post-infection. Au contraire, 9 jours après l'immunostimulation, les souriceaux n° 4, 5 et 6 présentent une expression en IL-12 significativement supérieure à celle des souriceaux des lots témoins (figure 8-B). De même, deux souriceaux sur 3 du lot immunostimulé par la souche Toxo *mic1-3* KO présentent une expression en IFN-γ significativement supérieure à celle des souriceaux des lots témoins (figure 8-A) ; il s'agit des souriceaux n° 4 et n° 6.

### 2.3 - Etude de l'état d'infection des souriceaux

### 2.3.1 - Dissémination des rates sur cellules HFF

La dissémination des rates sur cellules HFF est illustrée dans le Tableau IX. Aucune plage de lyse dans les cellules HFF infectées avec les rates provenant des souriceaux du lot témoin n'est détectée. Au contraire, les cellules HFF infectées avec les broyats de rate provenant des souriceaux immunostimulés par la souche Toxo *mic1-3* KO présentent des plages de lyse démontrant que des tachyzoïtes sont présents au niveau de la rate.

Par ailleurs, la technique de dissémination tissulaire des tachyzoïtes sur cellules HFF est une technique semi-quantitative qui montre que les souriceaux du lot immunostimulé par la souche Toxo *mic1-3* KO ont des états d'infection différents. Les souriceaux n° 4 et n° 6 semblent être les plus infectés car ils présentent le plus grand nombre de parasites dans la rate, suivi du souriceau n° 5 qui présente beaucoup moins de parasites dans la rate (Tableau IX).

**Tableau IX : Bilan d'analyse au microscope optique de la dissémination des organes sur cellules de fibroblastes Humain (HFF). Les tachyzoïtes observés sont issus des rates des souriceaux immunostimulés par la souche Toxo mic1-3 KO et forment des plages de lyse lorsqu'ils colonisent les cellules HFF.**

| | **Présence de parasites** | **Plages de lyse observées** |
|---|---|---|
| **Souriceau témoin 4** | NON | 0 |
| **Souriceau témoin 5** | NON | 0 |
| **Souriceau témoin 6** | NON | 0 |
| **Souriceau immunostimulé 4** | OUI | >70% des cellules lysées |
| **Souriceau immunostimulé 5** | OUI | 2 |
| **Souriceau immunostimulé 6** | OUI | >70% des cellules lysées |

### 2.3.2 - Immunohistologie

Les coupes d'immunohistologie d'intestins de souriceaux sont illustrées dans la Figure 9.

Pour les souriceaux du lot témoin, aucun tachyzoïte n'a été observé sur les coupes d'immunohistologie.

Les coupes d'intestin, respectivement des souriceaux n° 4 (B) et n° 6 (D) et n°5 (données non montrées), immunomarquées par un anticorps polyclonal de lapin anti-T. *gondii* permettent de visualiser la présence tachyzoïtes de la souche Toxo *mic1-3* KO (points blancs) uniquement pour le souriceau n°4.

### 2.3.3 - PCR

Les résultats de PCR sont représentés par la Figure 10. L'amplification du gène SAG1 de *Toxoplasma gondii* à partir d'échantillon d'ADN issu de broyat d'iléon est représentative de la présence de tachyzoïtes Toxo *mic1-3* KO (fragment amplifié à 1001 pb). Cette technique semi-quantitative montre une absence de bande pour les souriceaux du lot témoin (NV) sacrifié à J3 (T1) ou à J9 (T2).

Pour les souriceaux immunostimulés par la souche Toxo *mic1-3* KO (V), aucune bande correspondant au gène SAG1 n'est détecté 3 jours après l'infection. A J9, deux souriceaux sur 3 présentent une bande d'ADN de 1001 paires de bases témoignant de la présence de tachyzoïtes Toxo *mic1-3* KO. L'intensité des bandes observées entre les souriceaux n° 4 et n° 6 est nettement différente. L'intensité des bandes étant proportionnelle au nombre de parasites présents dans l'intestin, le souriceau n° 4 présente d'avantage de parasites au niveau intestinal que le souriceau n° 6, 9 jours post-infection. Ces résultats confirment l'observation faite par immunohistologie.

### Exemple 5 : Protection contre la cryptosporidiose de souriceaux immunostimulés par voie intrapéritonéale avec le mutant Toxo mic1-3 KO

### 1 - Protocole expérimental

### 1.1 - Animaux

L'immunostimulation est réalisée sur des souriceaux C57BL/6 âgés de 3 jours. Ces souriceaux ont été obtenus et sont élevés au sein du Centre INRA de Nouzilly (Indre et Loire). Les souriceaux sont maintenus tout au long de l'expérimentation en animalerie de niveau de confinement 2 afin de limiter au mieux le risque de contamination externe.

### 1.2 - Cryptosporidium parvum

Les oocystes de *Cryptosporidium parvum* sont obtenus à partir d'excréments de veaux infectés avec 10⁷ oocystes *C. parvum.* Les selles subissent différents traitements jusqu'à l'obtention d'une suspension de parasites purifiés, stériles, capables d'être utilisés en culture cellulaire. La manipulation des oocystes tout au long du traitement s'effectue à 4°C pour éviter que les oocystes s'excystent.

Brièvement, après récupération des selles, ces dernières sont diluées dans de l'eau fraiche puis passées sur un filtre de 100 µm et centrifugées à 1 900 g durant 10 minutes à 4°C. Les culots obtenus contenant les oocystes sont repris dans la solution de Bichromate de Potassium à 2% (Prolabo, réf. 26 776 290, CAS 7778-50-9), puis lavés deux fois à l'eau froide par centrifugation à 1 900 g durant 10 minutes à 4°C pour éliminer le bichromate de Potassium. Après lavage, le culot de coccidies est repris dans un mélange d'eau et d'éther (Ether Ethylique, Carlo Erba, CAS n° 60-29-7) dilué au 1/5, puis de nouveau centrifugé à 1 900 g durant 10 minutes à 4°C. Les phases supérieures contenant les graisses et l'éther sont éliminées et le culot est récupéré et repris dans de l'eau froide après un passage sur un filtre de 20 µm. Deux à trois millilitres de la suspension de parasites obtenue sont déposés sur un gradient de glucose réalisé à partir de la solution de Sheather (saccharose 500g, eau 320 ml, 0,2 g d'azide de sodium (Prolabo, CAS 26628-22-8)). Deux anneaux d'oocystes sont formés après centrifugation du gradient de glucose à 2 000 g durant 20 minutes à 4°C. Les deux anneaux sont récupérés et lavés plusieurs fois dans l'eau froide. Les oocystes purifiés sont ensuite stérilisés. Après centrifugation à 1 900 g durant 10 minutes à 4°C, le culot d'oocystes est incubé durant 15 minutes dans une solution d'eau de javel (Hypochlorite de sodium (Sigma 239305-500ML Titre : 4,5% de chlore actif) diluée à 10% dans de l'eau déminéralisée, puis lavé 3 fois dans du PBS 1X stérile (dilué à partir d'une solution de PBS 10X : Chlorure de sodium, NaCl 80 g /litre eau ; Chlorure de potassium, KCl 2g /litre ; Potassium Dihydrogeno-phosphate, KH₂ PO₄ : 2 g/litre ; Di-sodium Hydrogénophosphate, Na₂HPO₄, 12 H₂O : 29 g/litre). Les oocystes purifiés et stérilisés sont énumérés sur lame (5 µL de solution d'oocystes et 495 µL de vert Malachite), ajustés à une concentration de 2x10⁸ oocystes/ mL, aliquotés dans des tubes de 1,5 mL et conservés à 4°C.

### 1.3 - Souche Toxo mic1-3 KO

La souche mutante de *Toxoplasma gondii* dont les gènes codant pour les protéines MIC1 et MIC3 ont été invalidés (appelée souche Toxo *micl-3* KO) est entretenue par passages successifs sur une lignée de fibroblastes de prépuce humain (HFF) cultivés dans du milieu DMEM supplémenté par 10% de sérum de veau foetal (FCS), 2 mM glutamine, 50 U/mL de pénicilline et 50 µg/mL de streptomycine.

### 1.4 - Immunostimulation

Des lots de souriceaux C57BL/6 âgés de 3 jours ont été traités comme suit :
- 7 souriceaux (lot 1) ont reçu le mutant Toxo *mic1-3* KO
- 7 souriceaux (lot 2) ont servi de lot témoin non vaccinés.
   A J0, les souriceaux du lot 1 ont reçu 20 tachyzoïtes de la souche Toxo *mic1-3* KO par voie intrapéritonéale.
   A J3 post-immunostimulation, les souriceaux du lot 1 et du lot 2 ont été challengés avec 500 000 parasites de *Cryptosporidium parvum,*
   A J9 post-infection, les souriceaux du lot 1 et 2 sont sacrifiés pour évaluer la protection contre *Cryptosporidium parvum.*

### 1.5 - Etude de l'état d'infection des souriceaux

L'état d'infection des souriceaux a été analysé par la technique de dissémination tissulaire des tachyzoïtes sur cellules HFF précédemment décrite. Les cellules HFF sont déposées en plaque 24 puits, une semaine avant le dépôt des organes à raison de 10⁴ cellules par puits. Les cellules sont cultivées dans 1 mL de milieu de culture cellulaire DMEM supplémenté par 10% de sérum de veau foetal (FCS), 2 mM glutamine, 50 U/mL de pénicilline et 50 µg/mL de streptomycine. Après le sacrifice des souriceaux, la rate de chacun des souriceaux est prélevée et broyée dans 2 mL de PBS 1X. Dix microlitres de broyat sont déposés par puits contenant les cellules HFF cultivées dans 1 mL de milieu. Vingt-quatre heures après le dépôt des broyat de rate, les cellules sont lavées en DMEM puis 1 mL de milieu propre est déposé par puits. Les cellules sont laissées en culture jusqu'à détection des plages de lyse révélant la présence de tachyzoïtes Toxo *mic1-3* KO.

### 1.6 - Etude de la protection :

L'analyse de la protection des souriceaux contre *Cryptosporidium parvum* se fait par comptage des oocystes dans l'intestin. Une fois les souriceaux sacrifiés les intestins sont récupérés, pesés, mis dans 1 mL d'eau (4°C) et broyés pendant 20 secondes. Cent microlitres de broyat sont ajoutés à 400 µL de solution sucré à 4°C (500g de sucre en poudre, 320 mL d'eau distillée, azide de Na à 0,02%). Après avoir homogénéisé la solution par pipetage, 20 µL sont déposés sur cellule de THOMA. Avant la numérisation des oocystes, les lames sont gardées au frais pendant 15 minutes pour permettre la montée à la surface des oocystes dans la solution sucrée. Le nombre total d'oocystes dans l'intestin est calculé par la formule suivante : Nbr total d'oocystes de *C. parvum* dans l'intestin = Nbr d'oocystes comptés sur lame de THOMA x facteur de dilution x 10 000 x (1 + poids de l'intestin du souriceau).

### 2 - Résultats

### 2.1 - Etude de l'état d'infection des souriceaux

L'état de l'infection des souriceaux est évalué par la parasitémie pour *Cryptosporidium parvum* (*C. parvum*) et *Toxoplasma gondii* (*T. gondii*)*.* La parasitémie pour *C. parvum* est déterminée par le nombre d'oocystes total de *C. parvum* comptés dans l'intestin du souriceau. La parasitémie pour *T. gondii* est évaluée par dissémination des rates de souriceaux sur des cellules HFF. Les parasitémies pour *C. parvum* et *T. gondii* sont présentées dans le Tableau X.

**Tableau X : Récapitulatif de la parasitémie observée pour C. parvum et pour T. gondii du lot de souriceaux infectés par C. parvum (infectés C. parvum) et du lot de souriceaux inoculés par T. gondii et infectés par C. parvum (inoculés T. gondii + C. parvum) : t, tachyzoïtes ; NR, données non renseignées. Le nombre associé à la parasitémie pour T. gondii correspond au nombre de tachyzoïtes comptés dans 20 µL du surnageant des cellules HFF infectées après dissémination de la rate.**

| Infectés *C*. *parvum* | | | Inoculés *T*. *gondii* + *C. parvum* | | |
|---|---|---|---|---|---|
| **souriceaux** | Nbr d'oocystes total de *C. parvum* | Parasitémie *T.gondii* | **souriceaux** | Nbr d'oocystes total de *C. parvum* | Parasitémie *T.gondii* |
| **1** | 862500 | NON | **1** | 935000 | PEU (6,6 10⁵ t.) |
| **2** | 966500 | NON | **2** | 53000 | OUI (NR) |
| **3** | 1083000 | NON | **3** | 55000 | OUI (210⁶ t.) |
| **4** | 565000 | NON | **4** | 1568000 | TRES PEU (2 10⁵ t.) |
| **5** | 684000 | NON | **5** | 832500 | TRES PEU (1,2 10⁵ t.) |
| **6** | 847500 | NON | **6** | 754000 | TRES PEU (1,6 10⁵ t.) |
| **7** | 994500 | NON | **7** | 330000 | OUI (1,24 10⁶ t.) |

Les souriceaux infectés uniquement par *C. parvum* présentent un nombre d'oocystes total de *C. parvum* dans l'intestin qui varie entre 5x10⁵ et 1x10⁶ oocystes.

Les souriceaux immunostimulés par la souche Toxo *micl-3* KO puis infectés par *C. parvum* présentent une parasitémie T. *gondii* variable d'un souriceau à l'autre. Pour les souriceaux n° 2, 3 et 7, qui possèdent la parasitémie *T. gondii* la plus importante, le nombre d'oocystes total de *C. parvum* dans l'intestin varie de 5x10⁴ à 3,3x10⁵ oocystes. Ces résultats démontrent la corrélation existant entre la parasitémie à Toxo *mic1-3* KO et la diminution de l'infection à *C. parvum.*

### 2.2 - Etude de la protection

L'état de protection des souriceaux est représenté sur la Figure 11.

Dans le lot des souriceaux vaccinés par voie intrapéritonéale avec le mutant Toxo *micl-3* KO, 3 souriceaux sur 7 présentent un nombre total d'oocystes de *C. parvum* dans l'intestin significativement diminué d'environ 62% à 94%. Ces trois souriceaux sont les souriceaux n° 2, n° 3 et n° 7 ayant démontré une parasitémie à *T. gondii* la plus élévée de tous les souriceaux de ce groupe.

### Exemple 6 : Protection contre la cryptosporidiose de souriceaux immunostimulés par voie orale avec le mutant Toxo mic1-3 KO

### 1 - Protocole expérimental

### 1.1 - Animaux

L'immunostimulation est réalisée sur des souriceaux C57BL/6 âgés de 3 jours. Ces souriceaux ont été obtenus et sont élevés au sein du Centre INRA de Nouzilly (Indre et Loire). Les souriceaux sont maintenus tout au long de l'expérimentation en animalerie de niveau de confinement 2 afin de limiter au mieux le risque de contamination externe.

### 1.2 - Cryptosporidium parvum

Les oocystes de *Cryptosporidium parvum* sont obtenus à partir d'excréments de veaux infectés avec 10⁷ oocystes *C. parvum.* Les selles subissent différents traitements jusqu'à l'obtention d'une suspension de parasites purifiés, stériles, capables d'être utilisés en culture cellulaire. La manipulation des oocystes tout au long du traitement s'effectue à 4°C pour éviter que les oocystes s'excystent.

Brièvement, après récupération des selles, ces dernières sont diluées dans de l'eau fraiche puis passées sur un filtre de 100 µm et centrifugées à 1 900 g durant 10 minutes à 4°C. Les culots obtenus contenant les oocystes sont repris dans la solution de Bichromate de Potassium à 2% (Prolabo réf. 26 776 290, CAS 7778-50-9), puis lavés deux fois à l'eau froide par centrifugation à 1 900 g durant 10 minutes à 4°C pour éliminer le bichromate de Potassium. Après lavage, le culot de coccidies est repris dans un mélange d'eau et d'éther (Ether Ethylique, Carlo Erba CAS n° 60-29-7) dilué au 1/5, puis de nouveau centrifugé à 1 900 g durant 10 minutes à 4°C. Les phases supérieures contenant les graisses et l'éther sont éliminées et le culot est récupéré et repris dans de l'eau froide après un passage sur un filtre de 20 µm. Deux à trois millilitres de la suspension de parasites obtenue sont déposés sur un gradient de glucose réalisé à partir de la solution de Sheather (saccharose 500g, eau 320 ml, 0,2 g d'azide de sodium (Prolabo, CAS 26628-22-8)). Deux anneaux d'oocystes sont formés après centrifugation du gradient de glucose à 2 000 g durant 20 minutes à 4°C. Les deux anneaux sont récupérés et lavés plusieurs fois dans l'eau froide. Les oocystes purifiés sont ensuite stérilisés. Après centrifugation à 1 900 g durant 10 minutes à 4°C, le culot d'oocystes est incubé durant 15 minutes dans une solution d'eau de javel (Hypochlorite de sodium, Sigma 239305-500ML Titre : 4,5% de chlore actif) diluée à 10% dans de l'eau déminéralisée puis lavé 3 fois dans du PBS 1X stérile (dilué à partir d'une solution de PBS 10X : Chlorure de sodium, NaCl 80 g /litre eau ; Chlorure de potassium, KCl 2g /litre ; Potassium Dihydrogeno-phosphate, KH₂ PO₄ : 2 g/litre ; Di-sodium Hydrogénophosphate, Na₂HPO₄, 12 H₂O : 29 g/litre). Les oocystes purifiés et stérilisés sont énumérés sur lame (5 µL de solution d'oocystes et 495 µL de vert Malachite), ajustés à une concentration de 2x10⁸ oocystes/ mL, aliquotés dans des tubes de 1,5 mL et conservés à 4°C.

### 1.3 - Souche Toxo mic1-3 KO

La souche mutante de *Toxoplasma gondii* dont les gènes codant pour les protéines MIC1 et MIC3 ont été invalidés (appelée souche Toxo *micl-3* KO) est entretenue par passages successifs sur une lignée de fibroblastes de prépuce humain (HFF) cultivés dans du milieu DMEM supplémenté par 10% de sérum de veau foetal (FCS), 2 mM glutamine, 50 U/mL de pénicilline et 50 µg/mL de streptomycine.

### 1.4 - Immunostimulation

Des lots de souriceaux C57BL6 âgés de 3 jours ont été traités comme suit :
- 4 souriceaux (lot 1) ont reçu le mutant Toxo *mic1-3* KO
- 4 souriceaux (lot 2) ont servi de lot témoin non traités.
   A J0, les souriceaux du lot 1 ont reçu 20 000 tachyzoïtes de la souche Toxo *mic1-3* KO par voie orale.
   A J3 post-immunostimulation, les souriceaux du lot 1 et du lot 2 ont été challengés avec 1 000 000 parasites de *Cryptosporidium parvum,*
   A J10 post-immunostimulation, les souriceaux du lot 1 et 2 sont sacrifiés pour évaluer la protection contre *Cryptosporidium parvum.*

### 1.5 - Etude de l'état d'infection des souriceaux

L'état d'infection des souriceaux a été analysé par la technique de dissémination tissulaire des tachyzoïtes sur cellules HFF précédemment décrite. Les cellules HFF sont déposées en plaque 24 puits, une semaine avant le dépôt des organes à raison de 10⁴ cellules par puits. Les cellules sont cultivées dans 1 mL de milieu de culture cellulaire DMEM supplémenté par 10% de sérum de veau foetal (FCS), 2 mM glutamine, 50 U/mL de pénicilline et 50 µg/mL de streptomycine. Après le sacrifice des souriceaux, la rate de chacun des souriceaux est prélevée et broyée dans 2 mL de PBS 1X. Dix microlitres de broyat sont déposés par puits contenant les cellules HFF cultivées dans 1 mL de milieu. Vingt-quatre heures après le dépôt des broyats de rate, les cellules sont lavées en DMEM puis 1 mL de milieu propre est déposé par puits. Les cellules sont laissées en culture jusqu'à détection des plages de lyse révélant la présence de tachyzoïtes Toxo *mic1-3* KO.

### 1.6 - Etude de la protection

L'analyse de la protection des souriceaux contre *Cryptosporidium parvum* se fait par comptage des oocystes dans l'intestin. Une fois les souriceaux sacrifiés les intestins sont récupérés, pesés, mis dans 1 mL d'eau (4°C) et broyés pendant 20 secondes. Cent microlitres de broyat sont ajoutés à 400µl de solution sucré à 4°C (500g de saccharose, 320 mL d'eau distillée, azide de Na à 0,02%). Après avoir homogénéisé la solution par pipetage, 20 µL sont déposés sur lame de THOMA. Avant la numérisation des oocystes, les lames sont gardées au frais pendant 15 minutes pour permettre la montée à la surface des oocystes dans la solution sucrée. Le nombre total d'oocystes dans l'intestin est calculé par la formule suivante : Nbr total d'oocystes de *C. parvum* dans l'intestin = Nbr d'oocystes comptés sur lame de THOMA x facteur de dilution x 10 000 x (1 + poids de l'intestin du souriceau).

### 2 - Résultats

### 2.1 - Etude de l'état d'infection des souriceaux

### Dissémination des rates sur cellules HFF

La dissémination des rates sur cellules HFF est illustrée dans le tableau XI.

**Tableau XI: Récapitulatif de la parasitémie observée pour C. parvum et pour T. gondii du lot de souriceaux infectés par C. parvum (infectés C. parvum) et du lot de souriceaux inoculés par Toxo mic1-3 KO et infectés par C. parvum (inoculés T. gondii + C. parvum).**

| Inoculés *C*. *parvum* | | | Inoculés *T*. *gondii* + *C*. *parvum* | | |
|---|---|---|---|---|---|
| **souriceaux** | Nbr d'oocystes total de *C. parvum* | Parasitémie *T.gondii* | **souriceaux** | Nbr d'oocystes total de *C. parvum* | Parasitémie *T.gondii* |
| **1** | 2240000 | NON | **1** | 260000 | OUI |
| **2** | 1794000 | NON | **2** | 476000 | OUI |
| **3** | 1370000 | NON | **3** | 2046000 | PEU |
| **4** | 2448000 | NON | **4** | 1687500 | PEU |

Les souriceaux infectés uniquement par *C. parvum* présentent un nombre d'oocystes total de *C. parvum* dans l'intestin qui varie entre 1,4x10⁶ et 2,4x106 oocystes.

Les souriceaux immunostimulés par Toxo *micl-3* KO puis infectés par *C. parvum* présentent une parasitémie *T. gondii* estimée de manière semi- quantitative. Pour les souriceaux n° 1 et 2, qui possèdent une forte parasitémie *T. gondii,* le nombre d'oocystes total de *C. parvum* dans l'intestin varie de 2,6x10⁵ à 4,8x10⁵ oocystes. Ces résultats démontrent la corrélation existant entre la parasitémie à *T. gondii mic1-3* KO et la diminution de l'infection à *C. parvum.*

### 2.2 - Etude de la protection

L'état de protection des souriceaux est représenté en Figure 12.

Dans le lot des souriceaux vaccinés par voie orale avec le mutant Toxo *mic1-3* KO, deux souriceaux sur 4 présentent un nombre total d'oocystes de *C. parvum* dans l'intestin significativement diminué d'environ 75% et 87%. Il s'agit des souriceaux n° 1 et n° 2.

### Exemple 7 : Induction de sécrétion d'IL-12 et d'IFN-γ de splénocytes et induction de sécrétion d'IL-12 de cellules MLN provenant de moutons adultes ou nouveaux-nés après stimulation avec le mutant Toxo mic1-3 KO

### 1 - Protocole expérimental

### 1.1 - Animaux

Les ganglions mésentériques et les rates de sujets nouveau-nés utilisés au cours de cette expérience proviennent d'agneaux de race Ile de France âgés de 6-12 jours. Jusqu'au sacrifice, les agneaux ont été maintenus avec leurs mères en bergerie étanche (INRA - Nouzilly) afin de limiter les risques de contamination naturelle. Ils ont été anesthésiés par électronarcose puis euthanasiés pour collecter les différents organes. Seuls le personnel animalier et les expérimentateurs, équipés de vêtements internes à la bergerie, peuvent pénétrer dans les bâtiments afin d'éviter la contamination du milieu et ne quittent la zone étanche qu'après s'être douchés. Les ustensiles utilisés et le matériel biologique ne sortent qu'après être passés dans un bain désinfectant et le lisier est incinéré.

Les ganglions mésentériques et les rates de sujets adultes utilisés au cours de cette expérience proviennent de moutons adultes de race Ile de France âgés de 1 à 3 ans.

### 1.2 - Souches de Toxoplasma gondii

Les souches sauvages RH et Pru et la souche mutante Toxo *mic1-3* KO, sont entretenues par passages successifs sur une lignée de fibroblastes de prépuce humain (HFF) cultivés dans du milieu DMEM supplémenté par 10% de sérum de veau foetal (FCS), 2 mM glutamine, 50 U/mL de pénicilline et 50 µg/mL de streptomycine..

### 1.3 - Isolement des cellules d'intérêt

### 1.3.1 - Cellules des ganglions mésentériques

Les ganglions mésentériques sont prélevés le plus rapidement possible après l'euthanasie des animaux et transférés dans un tube de prélèvement stérile contenant du milieu de culture (HBSS supplémenté par 2% sérum de veau foetal (SVF) et 1% pénicilline/streptomycine (P/S)). Le tube de prélèvement contenant l'échantillon est conservé dans la glace jusqu'au laboratoire de recherche.

Chaque ganglion est dégraissé à l'aide de pinces, ciseaux ou scalpel préalablement stérilisés. Le ganglion est ensuite déposé sur une toile de nylon stérile (autoclavée) de 3 cm x 3 cm placée au fond d'une boite de Pétri stérile contenant 10 mL de milieu (HBSS ; 2% SVF ; 1% P/S). Le ganglion est écrasé et dilacéré à l'aide d'un piston d'une seringue de 5 mL pour libérer les cellules contenues dans le ganglion. Le milieu ainsi enrichi en cellules est filtré à l'aide d'une toile de nylon de 60 µm stérile positionnée au dessus d'un tube de 50 mL pour récupérer le milieu enrichi en cellules filtrées. Ces dernières étapes sont renouvelées deux fois comme suit : 10 mL de milieu sont ajoutés sur les fragments de ganglions restants. Les fragments de ganglions sont écrasés, dilacérés et filtrés comme décrit précédemment. Le tube de 50 mL de récupération du milieu enrichis en cellule et filtré est centrifugé à 1600 g durant 15 min à 4°C. Un lavage est effectué avec 50 mL de milieu (HBSS ; 2% SVF ; 1%P/S) par une centrifugation de 10 min à 400 g à 4°C. Le culot ainsi obtenu est resuspendu dans un volume final de 90 mL de milieu (HBSS ; 2% SVF ; 1% P/S) à température ambiante dans deux tubes de 50 mL.

Les 90 mL de suspension cellulaire sont divisés en 3 pour être purifiés en gradient Ficoll-Hypaque. 30 mL sont déposés avec précaution par tube (3 tubes au total) contenant 15 mL de Ficoll-Hypaque. Les 3 tubes de Ficoll-Hypaque enrichis en suspension cellulaire sont centrifugés à 1500 g sans frein (décélération 2, accélération 5), 30 minutes, à température ambiante. Pour chaque tube de Ficoll-Hypaque on obtient une phase supérieure composée de débris cellulaires, un anneau composé des cellules mononucléées positionné en interphase entre les débris cellulaires et la phase de Ficoll et enfin une phase inférieure composée de globule rouge du sang. Les 3 anneaux (20 mL / tubes) sont récupérés et divisés dans deux tubes de 50 mL et laver avec un volume final de 50 mL de milieu (HBSS ; 2% SVF ; 1% P/S) à 700 g pendant 10 min. Les culots de cellules mononuclées obtenus sont poolés. Le culot final est lavé avec 50 mL de milieu (HBSS ; 2% SVF ; 1% P/S) par centrifugation à 400 g durant 10 min. Le culot est repris dans 5 mL de RPMI, 10% SVF, 1% P/S, 5.10⁻⁵M de béta-mercaptoéthanol. Les cellules mononucléées sont conservées dans la glace, une partie des cellules est utilisée pour la numération et l'observation de la viabilité au bleu Trypan (échantillon dilué au 1/100). Une fois énumérées, les cellules sont réparties en plaque 96 puits à raison de 3.10⁵ cellules/puits.

### 1.3.2 - Isolement des splénocytes

La rate est prélevée le plus rapidement possible après l'euthanasie et transférés dans un tube de prélèvement stérile contenant du milieu de culture (HBSS ; 2% SVF ; 1% P/S). Le tube de prélèvement contenant l'échantillon est conservé dans la glace jusqu'au laboratoire de recherche.

Chaque rate est dégraissée à l'aide de pinces, ciseaux ou scalpel préalablement stérilisés. La rate est ensuite déposée sur une grille métallique (autoclavée) placée au fond d'une boite de pétri stérile contenant 10 mL de milieu (HBSS ; 2% SVF ; 1% P/S). La rate est écrasée et dilacérée à l'aide d'un piston d'une seringue de 5 mL pour libérer les cellules contenues dans la rate. Le milieu ainsi enrichi en cellules est déposé dans un tube de 50 mL. Ces dernières étapes sont renouvelées deux fois comme suit : 5 mL de milieu sont ajoutés sur les fragments de rate. Les fragments de rate sont écrasés, dilacérés comme décrit précédemment. Le tube de 50 mL de récupération du milieu enrichi en cellules est mis à sédimenter pendant 5 min à 4°C. Le surnageant est filtré sur une toile de nylon de 60µm au dessus d'un tube 50 mL. Dix millilitres de milieu sont ajoutés sur les sédiments restants et le second surnageant est récupéré de la même manière. Le tube de 50 mL de récupération des deux surnageants est centrifugé durant 30 sec à 400 g à 4°C. Le surnageant est récupéré et filtré sur une toile de nylon de 60 µm stérile au dessus d'un tube de 50 mL. Ce dernier tube est centrifugé 10 min à 400g à 4°C. Le culot cellulaire ainsi obtenu est resuspendu dans un volume final de 50 mL de milieu (HBSS ; 2% SVF ; 1% P/S). Un lavage est effectué par centrifugation durant 10 min à 400 g à 4°C. Le culot est ensuite repris dans un volume finale de 120 mL dans 3 tubes de 50 mL de milieu (HBSS ; 2% SVF ; 1% P/S) à température ambiante.

Les 120 mL de suspension cellulaire sont divisés en 3 pour être purifiés en gradient Ficoll-Hypaque. 30 mL sont déposés avec précaution par tube (4 tubes au total) contenant 15 mL de Ficoll-Hypaque. Les 4 tubes de Ficoll-Hypaque enrichis en suspension cellulaire sont centrifugés à 1500 g sans frein (décélération 2, accélération 5), 30 minutes à température ambiante. Pour chaque tube de Ficoll-Hypaque on obtient une phase supérieure composée de débris cellulaire, un anneau composé des cellules mononucléées positionné en interphase entre les débris cellulaire et la phase de Ficoll et enfin une phase inférieure composée de globule rouge du sang. Les 4 anneaux (20 mL/tubes) sont récupérés et divisés dans trois tubes de 50 mL et laver avec un volume final de 50 mL de milieu (HBSS ; 2% SVF ; 1% P/S) à 700 g pendant 10 min. Les culots de cellules mononuclées obtenus sont poolés. Le culot final est lavé avec 50 mL de milieu (HBSS ; 2% SVF ; 1%P/S) par centrifugation à 400g durant 10 min. Le culot est repris dans 5 mL de RPMI, 10% SVF, 1% P/S, 5.10⁻⁵M de béta-mercaptoéthanol. Les cellules mononucléées sont conservées dans la glace, une partie des cellules est utilisée pour la numération et l'observation de la viabilité au bleu trypan (échantillon dilué au 1/100). Une fois énumérées, les cellules sont réparties en plaque 96 puits à raison de 3.10⁵ cellules/puits.

### 1.4 - Stimulation des cellules mononucléées des ganglions mésentériques et des splénocytes d'agneau

Une fois énumérées les cellules sont réparties en plaque 96 puits à raison de 3.10⁵ cellules/puits. Les tachyzoïtes sont également énumérés en lame de Malassey, culotés et repris dans du milieu RPMI, 10% SVF, 1% P/S. Les solutions des tachyzoïtes (RH ou KO) sont ajustées pour obtenir une concentration de 3.10⁶ tachyzoïtes/mL. Les conditions de stimulation sont les suivantes, 3 cellules mononuclées sont stimulées par un tachyzoïte. Parasites, cellules mononuclées de ganglion et cellules mononuclées de rate sont cultivés à 37°C dans une étuve 5% CO₂ et 95 % d'humidité.

24h post stimulation les surnageants des cellules mononucléées de ganglion et les surnageants des cellules mononucléées de rate sont prélevés pour le dosage de l'interleukine-12 et de l'IFN-γ par la technique ELISA.

### 1.5 - Dosage de l'interleukine-12 par la technique ELISA

La technique employée pour le dosage de l'interleukine-12 est un ELISA de type sandwich sur plaque 96 puits. L'anticorps (anti-IL-12) immobilisé sur une plaque réagit spécifiquement avec l'IL-12 présent dans l'échantillon à tester. La quantité antigène-anticorps est mesurée dans un second temps après sa réaction avec un anticorps de même spécificité couplé à une enzyme.

Toutes les dilutions des différents réactifs se fait en PBS 1X, 0,05% Tween 20 et 1% de BSA hormis pour l'anticorps de capture dont la dilution se fait en PBS 1X.

50 µL par puits d'anticorps de capture dilué au 1/500e (Mouse anti-bovine interleukine-12 clone CC 301, Serotec MCA1782EL, Concentration initiale : 1000 µg / mL) sont déposés dans une plaque ELISA (plaque ELISA Nunc maxisorp 442404) puis incubés une nuit à 4°C. Trois lavages sont réalisés en tampon PBS 1X additionné de Tween 20 0,05% (PBS-T). Les sites non spécifiques sont saturés par incubation des plaques pendant 1 heure à température ambiante avec 200 µL d'une solution PBS 1X ; 0,05% Tween 20, 1% BSA. Trois lavages sont effectués en tampon PBS 1X additionné de Tween 20 à 0,05% (PBS-T).

La gamme est réalisée comme suit : 50 µL de la gamme étalon de rov IL-12 sont déposés à différentes concentration : 16U/mL ; 8U/mL ; 4U/mL ; 2U/mL ; 1U/mL ; 0,5U/mL ; 0,25U/mL ; 0,125U/mL ; 0,0625U/mL ; 0,03125U/mL. En parallèle, les échantillons (surnageant de cellules mononuclées stimulées, dilué ½) sont déposés à raison de 50 µL par puits. Echantillons et gamme étalon sont incubés 1h à température ambiante. 4 lavages sont effectués en tampon PBS 1X additionné de Tween 20 à 0,05% (PBS-T).

50 µl d'anticorps de détection biotinylés (Mouse anti-bovine interleukine-12: biotine clone CC 326 (Serotec MCA2173B) Concentration initiale : 500 µg/ml) dilué au 1/500 sont déposés/puits, pour une durée d'incubation d'une heure à température ambiante. Une série de 4 lavages est réalisée en tampon PBS 1X additionné de Tween 20 à 0,05% (PBS-T).

La révélation de l'IL-12 se fait par une réaction d'affinité biotine-ExtrAvidine® suivie d'une réaction colorimétrique entre la peroxydase et son substrat : 50 µL d'avidine modifiée couplée à une peroxydase (ExtrAvidine-Peroxydase conjugate® (Sigma E2886) diluée au 1/2000ème sont déposés par puits et incubés 20 minutes à température ambiante. Une série de 4 lavages est réalisée en tampon PBS 1X additionné de Tween 20 à 0,05% (PBS-T). La révélation est réalisée à l'aide de 50 µL/puits de substrat de peroxydase provenant d'une solution A et B mélangés volume à volume (TMB Peroxydase substrat Eurobio KPL Solution A 50-76-01 et solution B 50-65-00). La solution de substrat est incubée durant 15 minutes à température ambiante. 50 µL de solution d'arrêt (acide phosphorique 1M sigma 43,080-1, CAS 7664-38-2) sont ajoutés par puits sous hotte chimique.

La lecture des plaques se fait à la longueur d'onde λ=450nm. La courbe étalon est déterminée par la fonction suivante : valeur de la DO en fonction de la concentration de l'IL-12 recombinante. Les valeurs de DO des échantillons testés sont converties en concentration grâce à la courbe étalon. Pour être pris en compte les échantillons doivent donner des DO se trouvant dans la partie linéaire de la courbe étalon.

### 1.6 - Dosage de l'interféron gamma par la technique ELISA

La technique employée pour le dosage de l'interféron est un ELISA, comparable au dosage de l'interleukine-12 décrit dans le paragraphe précédent, de type sandwich sur plaque 96 puits. L'anticorps (anti-IFNγ) immobilisé sur une plaque réagit spécifiquement avec l'IFNγ présent dans l'échantillon à tester. La quantité antigène-anticorps est mesurée dans un second temps après sa réaction avec un anticorps de même spécificité à une enzyme.

Toutes les dilutions des différents réactifs se fait en PBS 1X, 0,05% Tween 20 et 1% de BSA hormis pour l'anticorps de capture dont la dilution se fait en PBS 1X.

50 µl par puits d'anticorps de capture dilué au 1/500^{e} (Mouse anti-bovine interféron gamma clone CC 330, Serotec MCA2112, Concentration initiale : 1000 µg / mL) sont déposés dans une plaque ELISA (plaque ELISA Nunc maxisorp 442404) puis incubés une nuit à 4°C. Trois lavages sont réalisés en tampon PBS 1X additionné de Tween 20 0,05% (PBS-T). Les sites non spécifiques sont saturés par incubation des plaques pendant 1 heure à température ambiante avec 200 µL d'une solution PBS 1X ; 0,05% Tween 20, 1% BSA. Trois lavages sont effectués en tampon PBS 1X additionné de Tween 20 à 0,05% (PBS-T).

La gamme est réalisée comme suit : 50 µL de la gamme étalon de Recombinant bovin IFNγ (Perbio Endogen robIFNGγ, concentration initiale : 30µg/ml) sont déposés à différentes concentration : 4ng/mL ; 2ng/mL ; 1ng/mL ; 0,5ng/mL ; 0,25ng/mL ; 0,125ng/mL ; 0,0625ng/mL ; 0,03125ng/mL ; 0,015ng/ml). En parallèle, les échantillons (surnageant de cellules mononuclées stimulées, dilué ½) sont déposés à raison de 50 µL par puits. Echantillons et gamme étalon sont incubés 1h à température ambiante. 4 lavages sont effectués en tampon PBS 1X additionné de Tween 20 à 0,05% (PBS-T).

50 µL d'anticorps de détection biotinylés (Mouse anti-bovine interferon gamma: biotine clone CC 302 (Serotec MCA1783B) Concentration initiale : 500 µg/ml) dilué au 1/500 sont déposés par puits, pour une durée d'incubation d'une heure à température ambiante. Une série de 4 lavages est réalisée en tampon PBS 1X additionné de Tween 20 à 0,05% (PBS-T).

La révélation de l'IFNγ se fait par une réaction d'affinité biotine-ExtrAvidine® suivie d'une réaction colorimétrique entre la peroxydase et son substrat : 50 µL d'avidine modifiée couplée à une peroxydase (ExtrAvidine-Peroxydase conjugate® (Sigma E2886) diluée au 1/2000ème sont déposés par puits et incubés 20 minutes à température ambiante. Une série de 4 lavages est réalisée en tampon PBS 1X additionné de Tween 20 à 0,05% (PBS-T). La révélation est réalisée à l'aide de 50 µL par puits de substrat de peroxydase provenant d'une solution A et B mélangés volume à volume (TMB Peroxydase substrat Eurobio KPL Solution A 50-76-01 et solution B 50-65-00). La solution de substrat est incubée durant 15 minutes à température ambiante. 50 µL de solution d'arrêt (acide phosphorique 1M sigma 43,080-1, CAS 7664-38-2) sont ajoutés par puits sous hotte chimique.

La lecture des plaques se fait à la longueur d'onde λ=450nm. La courbe étalon est déterminée par la fonction suivante : valeur de la DO en fonction de la concentration de l'IFNγ recombinante. Les valeurs de DO des échantillons testés sont converties en concentration grâce à la courbe étalon. Pour être pris en compte les échantillons doivent donner des DO se trouvant dans la partie linéaire de la courbe étalon.

### 2 - Résultats

Le taux d'IL-12 et d'IFN-γ sécrétés par les cellules mononucléées de rates et le taux d'IL-12 secrétée par les cellules mononucléées de ganglions mésentériques (mesenteric lymph node, MLN) isolées à partir d'agneaux âgés de 6 à 12 jours et de moutons adultes âgés de 1 à 3 ans et stimulées avec différentes souches du parasite *Toxoplasma gondii* sont représentés dans les Figures 13-A, 13-B et 13-C.

Chez les moutons âgés de 1 à 3 ans, la production d'IL-12 par les cellules mononucléées issues de la rate ou des ganglions mésentériques est supérieure lorsque celles-ci ont été stimulées *in vitro* par la souche mutante Toxo *mic1-3* KO ou par les souches sauvages Pru et RH de *T. gondii.* Cette observation est plus marquée dans le cas des cellules provenant des ganglions mésentériques de mouton adultes.

Chez les agneaux âgés de 6 à 12 jours, la production d'IL-12 par les cellules mononucléées issues de la rate ou des ganglions mésentériques est supérieure lorsque celles-ci ont été stimulés *in vitro* par du parasite *T. gondii.* La production d'IL-12 est même supérieure lorsque les cellules mononuclées ont été stimulées *in vitro* par la souche mutante Toxo *mic1-3* KO.

Chez les moutons âgés de 1 à 3 ans, la production d'IFNγ par les cellules mononucléées issues de la rate ne présente pas de différence significative lorsque celles-ci ont été stimulées *in vitro* par la souche mutante *mic1-3* KO ou par les souches sauvages Pru et RH de *T. gondii.*

Chez les agneaux âgés de 6 à 12 jours, la production d'IFNγ par les cellules mononucléées issues de la rate ne présente pas de différence significative lorsque celles-ci ont été stimulées *in vitro* par la souche mutante *mic1-3* KO ou par les souches sauvages Pru et RH de *T. gondii.*

La production d'IL-12 et d'IFN-γ par les cellules mononucléées issues d'agneau est très supérieure à celle observée pour les cellules mononucléées issues des moutons adultes.

### Exemple 8 : immunostimulation d'agneaux nouveau-nés par le mutant Toxoplasma gondii Toxo mic1-3 KO

### 1 - Protocole expérimental

### 1.1 - Animaux

L'immunostimulation est réalisée sur des agneaux nouveau-nés âgés de 1 jour. Après ingestion du collostrum, les agneaux ont été isolés de leurs mères en bergerie étanche (INRA - Nouzilly) afin de limiter les risques de contamination naturelle. Ils ont été anesthésiés par électronarcose puis euthanasiés pour collecter les différents organes. Seuls le personnel animalier et les expérimentateurs, équipés de vêtements internes à la bergerie, peuvent pénétrer dans les bâtiments afin d'éviter la contamination du milieu et ne quittent la zone étanche qu'après s'être douchés.

### 1.2 - Souche Toxo mic1-3 KO

La souche mutante de *Toxoplasma gondii* dont les gènes codant pour les protéines MIC1 et MIC3 ont été invalidés (appelée souche Toxo *micl-3* KO) est entretenue par passages successifs sur une lignée de fibroblastes de prépuce humain (HFF) cultivés dans du milieu DMEM supplémenté par 10% de sérum de veau foetal (FCS), 2 mM glutamine, 50 U/mL de pénicilline et 50 µg/mL de streptomycine.

### 1.3 - Immunostimulation

Six agneaux âgés d'un jour ont été traités comme suit :
- 4 agneaux (lot A) ont reçu le mutant Toxo *mic1-3* KO
- 2 agneaux (lot B) ont servi de lot témoin non vacciné.
   A J1, les agneaux du lot A ont reçu 10⁶ tachyzoïtes de la souche Toxo *mic1-3* KO par voie sous-cutanée. Après immunostimulation, les températures et les poids des agneaux sont relevés quotidiennement.
   A J15 post-immunostimulation, les agneaux sont euthanasiés pour l'étude de la réponse immunitaire et de la parasitémie.

### 1.4 - Isolement des cellules d'intérêt

### 1.4.1 - Isolement des splénocytes

La rate est prélevée le plus rapidement possible après l'euthanasie et transférée dans un tube de prélèvement stérile contenant du milieu de culture (HBSS ; 2% SVF ; 1% P/S). Le tube de prélèvement contenant l'échantillon est conservé dans la glace jusqu'au laboratoire de recherche.

Chaque rate est dégraissée à l'aide de pinces, ciseaux ou scalpel préalablement stérilisés. La rate est ensuite déposée sur une grille métallique (autoclavée) placée au fond d'une boite de pétri stérile contenant 10 mL de milieu (HBSS ; 2% SVF ; 1% P/S). La rate est écrasée et dilacérée à l'aide d'un piston d'une seringue de 5 mL pour libérer les cellules contenues dans la rate. Le milieu ainsi enrichi en cellules est déposé dans un tube de 50 mL. Ces dernières étapes sont renouvelées deux fois comme suit : 5 mL de milieu sont ajoutés sur les fragments de rate. Les fragments de rate sont écrasés, dilacérés comme décrit précédemment. Le tube de 50 mL de récupération du milieu enrichi en cellule est mis à sédimenter pendant 5 min à 4°C. Le surnageant est filtré sur une toile de nylon de 60µm au dessus d'un tube 50 mL. Dix millilitres de milieu sont ajoutés sur les sédiments restants et le second surnageant est récupéré de la même manière. Le tube de 50 mL de récupération des deux surnageants est centrifugé durant 30 sec à 400 g à 4°C. Le surnageant est récupéré et filtré sur une toile de nylon de 60 µm stérile au dessus d'un tube de 50 mL. Ce dernier tube est centrifugé 10 min à 400g à 4°C. Le culot cellulaire ainsi obtenu est resuspendu dans un volume final de 50 mL de milieu (HBSS ; 2% SVF ; 1% P/S). Un lavage est effectué par centrifugation durant 10 min à 400 g à 4°C. Le culot est ensuite repris dans un volume finale de 120 mL dans 3 tubes de 50 mL de milieu (HBSS ; 2% SVF ; 1% P/S) à température ambiante.

Les 120 mL de suspension cellulaire sont divisés en 3 pour être purifiés en gradient Ficoll-Hypaque. 30 mL sont déposés avec précaution par tube (4 tubes au total) contenant 15 mL de Ficoll-Hypaque. Les 4 tubes de Ficoll-Hypaque enrichi en suspension cellulaire sont centrifugés à 1500 g sans frein (décélération 2, accélération 5), 30 minutes à température ambiante. Pour chaque tube de Ficoll-Hypaque on obtient une phase supérieure composée de débris cellulaire, un anneau composé des cellules mononucléées positionné en interphase entre les débris cellulaire et la phase de Ficoll et enfin une phase inférieure composée de globule rouge du sang. Les 4 anneaux (20 mL/tubes) sont récupérés et divisés dans trois tubes de 50 mL et laver avec un volume final de 50 mL de milieu (HBSS ; 2% SVF ; 1% P/S) à 700 g pendant 10 min. Les culots de cellules mononuclées obtenus sont poolés. Le culot final est lavé avec 50 mL de milieu (HBSS ; 2% SVF ; 1%P/S) par centrifugation à 400 g durant 10 min. Le culot est repris dans 5 mL de RPMI, 10% SVF, 1% P/S, 5.10⁻⁵M de béta-mercaptoéthanol. Les cellules mononucléées sont conservées dans la glace, une partie des cellules est utilisée pour la numération et l'observation de la viabilité au bleu trypan (échantillon dilué au 1/100). Une fois énumérées les cellules sont réparties en plaque 96 puits à raison de 3.10⁵ cellules/puits.

### 1.4.2 - Isolement des cellules ganglionnaires

Les ganglions poplités et subiliaques, situés à proximité du site d'injection, sont prélevés le plus rapidement possible après l'euthanasie des animaux et transférés dans un tube de prélèvement stérile contenant du milieu de culture (HBSS supplémenté par 2% sérum de veau foetal (SVF) et 1% pénicilline/streptomycine (P/S)). Le tube de prélèvement contenant l'échantillon est conservé dans la glace jusqu'au laboratoire de recherche.

Chaque ganglion est dégraissé à l'aide de pinces, ciseaux ou scalpel préalablement stérilisés. Le ganglion est ensuite déposé sur une toile de nylon stérile (autoclavée) de 3 cm x 3 cm placée au fond d'une boite de pétrie stérile contenant 10 ml de milieu (HBSS ; 2% SVF ; 1% P/S). Le ganglion est écrasé et dilacéré à l'aide d'un piston d'une seringue de 5 mL pour libérer les cellules contenues dans le ganglion. Le milieu ainsi enrichi en cellules est filtré à l'aide d'une toile de nylon de 60 µm stérile positionnée au dessus d'un tube de 50 mL pour récupérer le milieu enrichi en cellules filtrées. Ces dernières étapes sont renouvelées deux fois comme suit : 10 mL de milieu sont ajoutés sur les fragments de ganglions restants. Les fragments de ganglions sont écrasés, dilacérés et filtrés comme décrit précédemment. Le tube de 50 mL de récupération du milieu enrichi en cellule et filtré est centrifugé à 1600 g durant 15 min à 4°C. Un lavage est effectué avec 50 mL de milieu (HBSS ; 2% SVF ; 1%P/S) par une centrifugation de 10 min à 400 g à 4°C. Le culot est repris dans 5 mL de RPMI, 10% SVF, 1% P/S, 5.10⁻⁵M de béta-mercaptoéthanol. Le culot est conservé dans la glace, une partie des cellules est utilisée pour la numération et l'observation de la viabilité au bleu Trypan (échantillon dilué au 1/100). Une fois énuméré les cellules sont réparties en plaque 96 puits à raison de 3.10⁵ cellules/puits.

### 1.5 - Etude de la réponse inflammatoire post-immunostimulation

### 1.5.1- A partir des cellules de rate

Une fois les cellules mononuclées de la rate isolées par gradient de Ficoll Histopaque et distribuées en plaque 96 puits, à raison de 3.10⁵ cellules par puits, ces dernières sont stimulées *in vitro* avec 30µg/ml d'extrait total parasitaire de la souche Toxo *micl-3* KO. Pour établir un témoin positif d'expérimentation, les cellules sont stimulées avec de la concanavaline A. Inversement pour établir un témoin négatif d'expérimentation, les cellules sont cultivées en milieu sans stimulant. Les surnageants sont prélevés au bout de 24 heures post -stimulation *in vitro*

La technique employée pour le dosage de l'IFNγ est un ELISA de type sandwich sur plaque 96 puits. L'anticorps (anti-IFNγ) immobilisé sur une plaque réagit spécifiquement avec l'IFNγ présent dans l'échantillon à tester. La quantité antigène-anticorps est mesurée dans un second temps après sa réaction avec un anticorps de même spécificité couplé à une enzyme.

Toutes les dilutions des différents réactifs se fait en PBS 1X, 0,05% Tween 20 et 1% de BSA, hormis pour l'anticorps de capture dont la dilution se fait en PBS1X

50 µL par puits d'anticorps de capture dilué au 1/500e (Mouse anti-bovine IFNg clone CC 330, Serotec MCA2112, Concentration initiale : 1000 µg / mL) sont déposés dans une plaque ELISA (plaque ELISA Nunc maxisorp 442404) puis incubés une nuit à 4°C. Trois lavages sont réalisés en tampon PBS 1X additionné de Tween 20 0,05% (PBS-T). Les sites non spécifiques sont saturés par incubation des plaques pendant 1 heure à température ambiante avec 200 µL d'une solution PBS 1X ; 0,05% Tween 20, 1% BSA. Trois lavages sont effectués en tampon PBS 1X additionné de Tween 20 à 0,05% (PBS-T).

La gamme est réalisée comme suit : 50 µL de la gamme étalon de rboIFNγ sont déposés à différentes concentration : 4ng/mL ; 2ng/mL ; 1ng/mL ; 0,5ng/mL ; 0,25ng/mL ; 0,125ng/mL ; 0,0625ng/mL ; 0,03125ng/mL ; 0,015ng/mL. En parallèle, les échantillons (surnageant de cellules mononuclées stimulées) sont déposés à raison de 50 µL par puits. Echantillons et gamme étalon sont incubés 1h à température ambiante. 4 lavages sont effectués en tampon PBS 1X additionné de Tween 20 à 0,05% (PBS-T).

50 µL d'anticorps de détection biotinylés (Mouse anti-bovine IFNg: biotine clone CC 302 (Serotec MCA1783B) Concentration initiale : 500 µg/ml) dilué au 1/500 sont déposés/puits, pour une durée d'incubation d'une heure à température ambiante. Une série de 4 lavages est réalisée en tampon PBS 1X additionné de Tween 20 à 0,05% (PBS-T).

La révélation de l'IFNγ se fait par une réaction d'affinité biotine-ExtrAvidine® suivie d'une réaction colorimétrique entre la peroxydase et son substrat : 50 µL d'une solution d'avidine modifiée couplée à une peroxydase (ExtrAvidine-Peroxydase conjugate® (Sigma E2886) diluée au 1/2000ème sont déposés par puits et incubés 20 minutes à température ambiante. Une série de 4 lavages est réalisée en tampon PBS 1X additionné de Tween 20 à 0,05% (PBS-T). La révélation est réalisée à l'aide de 50 µL/puits de substrat de peroxydase provenant d'une solution A et B mélangés volume à volume (TMB Peroxydase substrat Eurobio KPL Solution A 50-76-01 et solution B 50-65-00). La solution de substrat est incubée durant 15 minutes à température ambiante. 50 µL de solution d'arrêt (acide phosphorique 1M sigma 43,080-1, CAS 7664-38-2) sont ajoutés/ puits sous hotte chimique.

La lecture des plaques se fait à la longueur d'onde λ=450nm. La courbe étalon est déterminée par la fonction suivante : valeur de la DO en fonction de la concentration de l'IFNγ recombinante. Les valeurs de DO des échantillons testés sont converties en concentration grâce à la courbe étalon. Pour être pris en compte les échantillons doivent donner des DO se trouvant dans la partie linéaire de la courbe étalon.

### 1.5.2 - A partir des cellules ganglionnaires

Les cellules ganglionnaires réparties en plaque 96 puits à raison de 3.10⁵ cellules par puits sont cultivées sans aucun stimulant afin de détecter l'expression des cytokines ex-vivo. Les surnageants des cellules ganglionnaires sont prélevés au bout de 24 heures. L'IFNγ est dosé par la technique ELISA comme décrit précédemment.

### 2 - Résultats

### 2.1 - Déroulement de l'expérimentation

Après ingestion du colostrum, les agneaux sont séparés de leur mère et nourris avec un système de louve. Les 4 agneaux du lot A ont été immunostimulés avec 10⁶ tachyzoïtes de la souche Toxo *mic1-3* KO, fraichement produite. Après inoculation de la souche Toxo *micl-3* KO, aucun signe clinique sévère n'a été constaté et les agneaux immunostimulés par la souche Toxo *mic1-3* KO présentent une courbe de poids similaire à la prise de poids des agneaux témoins (Figure 14).

Les agneaux du lot A et du lot B ont été euthanasiés 15 jours après l'immunostimulation.

### 2.2 - Etude de la réponse inflammatoire post-immunostimulation

Les résultats des dosages de l'IFN-γ produit à partir des splénocytes restimulés avec de l'extrait total de *T. gondii* et à partir des cellules des ganglions subiliaques et poplités sont présentés Figures 15 et 16.

Les agneaux du lot témoin (1423 et 1428) n'ont pas développé de réponse inflammatoire (sécrétion IFN-γ) ni au niveau de la rate, ni au niveau des ganglions subiliaques et poplités.

Au contraire, 15 jours après l'immunostimulation, les cellules des ganglions subiliaques de 3 agneaux sur 4, produisent de l'IFN-γ. Ces ganglions sont situés en aval du site d'injection, contrairement aux ganglions poplités, situés en amont et pour lesquels nous ne détectons pas de production d'IFN-γ.

La réponse inflammatoire des agneaux immunostimulés par la souche Toxo *mic1-*3 KO est confirmée après restimulation des splénocytes par de l'extrait total parasitaire de la souche Toxo *mic1-3* KO puisque pour les 4 agneaux, nous induisons une production d'IFN-γ. Les splénocytes sont également stimulés avec la concanavaline A, une protéine de la famille des lectines, connue pour être un activateur polyclonal des lymphocytes T et qui sert de témoin positif de stimulation des cellules immunitaires.

### Exemple 9 : Protection contre la cryptosporidiose d'agneaux immunostimulés par voie sous-cutanée avec le mutant Toxo mic1-3 KO

### 1 - Protocole expérimental

### 1.1 - Animaux

L'immunostimulation est réalisée sur des agneaux nouveau-nés âgés de 1 jour. Après ingestion du colostrum, les agneaux ont été isolés de leurs mères en bergerie étanche (INRA - Nouzilly) afin de limiter les risques de contamination naturelle.

A l'issue de l'expérimentation, les agneaux ont été anesthésiés par électronarcose puis euthanasiés pour collecter les différents organes. Seuls le personnel animalier et les expérimentateurs, équipés de vêtements internes à la bergerie, peuvent pénétrer dans les bâtiments afin d'éviter la contamination du milieu et ne quittent la zone étanche qu'après s'être douchés.

### 1.2 - Cryptosporidium parvum

Les oocystes de *Cryptosporidium parvum* sont obtenus à partir d'excréments de veaux infectés avec 10⁷ oocystes *C. parvum.* Les selles subissent différents traitements jusqu'à l'obtention d'une suspension de parasites purifiés, stériles, capables d'être utilisés en culture cellulaire. La manipulation des oocystes tout au long du traitement s'effectue à 4°C pour éviter que les oocystes s'excystent.

Brièvement, après récupération des selles, ces dernières sont diluées dans de l'eau fraiche puis passées sur un filtre de 100 µm et centrifugées à 1 900 g durant 10 minutes à 4°C. Les culots obtenus contenant les oocystes sont repris dans la solution de Bichromate de Potassium à 2% (Prolabo, réf. 26 776 290, CAS 7778-50-9), puis lavés deux fois à l'eau froide par centrifugation à 1 900 g durant 10 minutes à 4°C pour éliminer le bichromate de Potassium. Après lavage, le culot de coccidies est repris dans un mélange d'eau et d'éther (Ether Ethylique, Carlo Erba, CAS n° 60-29-7) dilué au 1/5, puis de nouveau centrifugé à 1 900 g durant 10 minutes à 4°C. Les phases supérieures contenant les graisses et l'éther sont éliminées et le culot est récupéré et repris dans de l'eau froide après un passage sur un filtre de 20 µm. Deux à trois millilitres de la suspension de parasites obtenue sont déposés sur un gradient de glucose réalisé à partir de la solution de Sheather (saccharose 500g, eau 320 ml, 0,2 g d'azide de sodium (Prolabo, CAS 26628-22-8)). Deux anneaux d'oocystes sont formés après centrifugation du gradient de glucose à 2 000 g durant 20 minutes à 4°C. Les deux anneaux sont récupérés et lavés plusieurs fois dans l'eau froide. Les oocystes purifiés sont ensuite stérilisés. Après centrifugation à 1 900 g durant 10 minutes à 4°C, le culot d'oocyste est incubé durant 15 minutes dans une solution d'eau de javel (Hypochlorite de sodium (Sigma 239305-500ML Titre : 4,5% de chlore actif) diluée à 10% dans de l'eau déminéralisée, puis lavé 3 fois dans du PBS 1X stérile (dilué à partir d'une solution de PBS 10X : Chlorure de sodium, NaCl 80 g /litre eau ; Chlorure de potassium, KCl 2g /litre ; Potassium Dihydrogeno-phosphate, KH₂ PO₄ : 2 g/litre ; Di-sodium Hydrogénophosphate, Na₂HPO₄, 12 H₂O : 29 g/litre). Les oocystes purifiés et stérilisés sont énumérés sur lame (5 µL de solution d'oocystes et 495 µL de vert Malachite), ajustés à une concentration de 2x10⁸ oocystes/ mL, aliquotés dans des tubes de 1,5 mL et conservés à 4°C.

### 1.3 - Souche Toxo mic1-3 KO

La souche mutante de *Toxoplasma gondii* dont les gènes codant pour les protéines TgMIC1 et TgMIC3 ont été invalidés (appelée souche Toxo *micl-3* KO) est entretenue par passages successifs sur une lignée de fibroblastes de prépuce humain (HFF) cultivés dans du milieu DMEM supplémenté par 10% de sérum de veau foetal (FCS), 2 mM glutamine, 50 U/mL de pénicilline et 50 µg/mL de streptomycine.

### 1.4 - Immunostimulation

Deux lots d'agneaux mâles âgés de 1 jour ont été traités comme suit :
- 8 agneaux (lot A) ont servi de lot témoin non vaccinés,
- 8 agneaux (lot B) ont reçu le mutant Toxo *mic1-3* KO.
   A J1, les agneaux du lot B ont reçu 10⁶ tachyzoïtes de la souche Toxo *mic1-3* KO par voie intrapéritonéale.
   A J7 post-infection, les agneaux du lot A et du lot B ont été challengés avec 5.10⁶ oocystes de *Cryptosporidium parvum* et les agneaux ont été sacrifiés à J31.

### 1.5 - Etude de la protection

Pour analyser la protection générée par l'immunostimulation avec la souche Toxo *mic1-3* KO, 3 paramètres ont été étudiés :

### • Survie - Mortalité

Après challenge avec *Cryptosporidium parvum,* les agneaux ont été suivis quotidiennement pendant une durée de 25 jours (jusqu'à J31).

### • La prise de poids :

Les agneaux du lot A et du lot B ont été pesés tous les 2-3 jours afin d'évaluer leur prise de poids.

### • L'excrétion d'oocystes :

Les fèces des agneaux ont été récupérées quotidiennement de J9 à J22 et sont conservées à +4°C. Après pesée, 26 mg de matière fécale sont dilués dans 750 µL d'eau. Une solution de 4mL de sucrose est ajoutée. Après homogénéisation, 20 µL sont déposés sur lame de Thoma et les oocystes sont dénombrés. Le nombre d'oocystes excrétés est ensuite calculé avec la formule suivante: N= n x 10 000 x 20 (N : nombre d'oocystes par g ou par ml, n = nombre d'oocystes comptés sur la cellule, 20 = dilution 1/4 x 1/5).

### 2 - Résultats

### 2.1 - Déroulement de l'expérimentation

Après ingestion du colostrum, les agneaux sont séparés de leur mère et nourris avec un système de louve. 3 agneaux du lot B et 1 agneau du lot A n'ont pas réussi à se nourrir convenablement et ont été euthanasiés. Les euthanasies étant survenues avant le challenge infectieux, ces animaux ont été statistiquement sortis du protocole.

### 2.1 - Etude de la protection

### • Survie - Mortalité

Consécutivement à l'infection à *C. parvum,* quatre agneaux essentiellement du lot contrôle (lot A) ont été euthanasiés contrairement au lot immuno-stimulé (lot B) dans lequel aucun agneau n'est mort à la suite de l'infection à *C. parvum (**Figure 17**).*

### • Prise de poids

Le gain de masse quotidien (GMQ) des agneaux du lot A et du lot B sont présentés Figure 18. Nous constatons un GMQ significativement plus faible pour le lot A uniquement challengé par *C. parvum* que pour le lot B immunostimulé avec la souche Toxo *mic1-3* KO puis challengé par *C. parvum.*

### • Excrétion des oocystes

L'analyse de la charge parasitaire dans les excréments des agneaux montre un décalage du pic d'excrétion d'un jour pour le lot immuno-stimulé avec la souche Toxo *mic1-3* KO, avec une baisse de 70% du pic d'excrétion moyen observé à 5 jours post-infection *C. parvum* comparé au pic d'excrétion moyen pour le lot contrôle uniquement challengé par *C. parvum* observé à 4 jours post-infection par *C. parvum* (Figure 19).

## Revendications

1. Souches de *Toxoplasma gondii* possédant les deux adhésines MIC-1 et MIC-3 inactivées par une modification génique portant sur les deux gènes *mic-1* et *mic-3* pour leur utilisation dans la prévention chez un mammifère nouveau-né, d'une pathologie associée à un apicomplexe de la famille des *Cryptosporidiidae,* ladite pathologie étant la cryptosporidiose, et ledit apicomplexe de la famille des *Cryptosporidiidae* responsable de la cryptosporidiose étant *Cryptosporidium parvum.*

2. Souches de *Toxoplasma gondii* possédant les deux adhésines MIC-1 et MIC-3 inactivées par une modification génique portant sur les deux gènes *mic-1* et *mic-3* pour leur utilisation selon la revendication 1, dans laquelle ledit mammifère est un être humain ou un animal.

3. Souches de *Toxoplasma gondii* possédant les deux adhésines MIC-1 et MIC-3 inactivées par une modification génique portant sur les deux gènes *mic-1* et *mic-3* pour leur utilisation selon la revendication 2, dans laquelle ledit animal appartient au groupe comprenant ou constitué des ovins, des caprins, des porcins, des bovins, des équidés, des camélidés, des canidés ou des félidés.

4. Souches de *Toxoplasma gondii* possédant les deux adhésines MIC-1 et MIC-3 inactivées par une modification génique portant sur les deux gènes *mic-1* et *mic-3* pour leur utilisation selon l'une des revendications 1 à 3, dans laquelle lesdites souches de *Toxoplasma gondii* sont administrées au mammifère à raison de 20 à 10⁹ tachyzoïtes.

5. Souches de *Toxoplasma gondii* possédant les deux adhésines MIC-1 et MIC-3 inactivées par une modification génique portant sur les deux gènes *mic-1* et *mic-3* pour leur utilisation selon l'une quelconque des revendications 1 à 4, dans laquelle lesdites souches se trouvent sous une forme galénique choisie parmi le groupe comprenant ou constitué par des suspensions liquides, des dispersions solides ou liquides, des poudres, des pâtes ou des lyophilisats.

6. Souches de *Toxoplasma gondii* possédant les deux adhésines MIC-1 et MIC-3 inactivées par une modification génique portant sur les deux gènes *mic-1* et *mic-3* pour leur utilisation selon l'une quelconque des revendications 1 à 5, dans laquelle lesdites souches sont associées à au moins un autre antigène, ou au moins un adjuvant, ou au moins un stabilisant, ou au moins un conservateur ou un mélange d'au moins deux desdits produits permettant d'accroitre la réponse immune dudit mammifère.

## Patentansprüche

1. Stämme von *Toxoplasma gondii,* welche die beiden Adhäsine MIC-1 und MIC-3 besitzen, die durch eine genetische Modifikation betreffend die beiden Gene *mic-1* und *mic-3* inaktiviert wurden, zu ihrer Verwendung bei der Prävention einer Pathologie bei neugeborenen Säugern, die mit einer Apicomplexa der Familie der *Cryptosporidiidae* assoziiert ist, wobei die Pathologie Cryptosporidiose ist, und die Apicomplexa der Familie der *Cryptosporidiidae,* die für die Cryptosporidiose verantwortlich ist, *Cryptosporidium parvum* ist.

2. Stämme von *Toxoplasma gondii,* welche die beiden Adhäsine MIC-1 und MIC-3 besitzen, die durch eine genetische Modifikation betreffend die beiden Gene *mic-1* und *mic-3* inaktiviert wurden, zu ihrer Verwendung nach Anspruch 1, wobei der Säuger ein Mensch oder ein Tier ist.

3. Stämme von *Toxoplasma gondii,* welche die beiden Adhäsine MIC-1 und MIC-3 besitzen, die durch eine genetische Modifikation betreffend die beiden Gene *mic-1* und *mic-3* inaktiviert wurden, zu ihrer Verwendung nach Anspruch 2, wobei das Tier zu einer Gruppe gehört, die umfasst oder besteht aus Schafen, Ziegen, Schweinen, Rindern, Equiden, Kameliden, Canidae oder Felidae.

4. Stämme von *Toxoplasma gondii,* welche die beiden Adhäsine MIC-1 und MIC-3 besitzen, die durch eine genetische Modifikation betreffend die beiden Gene *mic-1* und *mic-3* inaktiviert wurden, zu ihrer Verwendung nach einem der Ansprüche 1 bis 3, wobei die Stämme von *Toxoplasma gondii* dem Säuger in einer Menge von 20 bis 10⁹ Tachyzoiten verabreicht werden.

5. Stämme von *Toxoplasma gondii,* welche die beiden Adhäsine MIC-1 und MIC-3 besitzen, die durch eine genetische Modifikation betreffend die beiden Gene *mic-1* und *mic-3* inaktiviert wurden, zu ihrer Verwendung nach einem der Ansprüche 1 bis 4, wobei sich die Stämme in einer galenischen Form befinden, die ausgewählt ist aus der Gruppe umfassend oder bestehend aus flüssigen Suspensionen, festen oder flüssigen Dispersionen, Pulvern, Pasten oder Lyophilisaten.

6. Stämme von *Toxoplasma gondii,* welche die beiden Adhäsine MIC-1 und MIC-3 besitzen, die durch eine genetische Modifikation betreffend die beiden Gene *mic-1* und *mic-3* inaktiviert wurden, zu ihrer Verwendung nach einem der Ansprüche 1 bis 5, wobei die Stämme mit mindestens einem Antigen oder mit mindestens einem Adjuvans oder mit mindestens einem Stabilisator oder mit mindestens einem Konservierungsmittel oder in Mischung mit mindestens zwei der Produkte assoziert sind, die es gestatten, die Immunantwort des Säugers zu erhöhen.

## Claims

1. Strains of *Toxoplasma gondii* having the two adhesins MIC-1 and MIC-3 inactivated by a genetic modification relating to the two *mic-1* and *mic-3* genes, for the use thereof in the prevention in a neonate mammal, of a pathology associated with an apicomplexan of the family *Cryptosporidiidae,* said pathology being cryptosporidiosis, and said apicomplexan of the family *Cryptosporidiidae* responsible for the cryptosporidiosis being *Cryptosporidium parvum.*

2. Strains of *Toxoplasma gondii* having the two adhesins MIC-1 and MIC-3 inactivated by a genetic modification relating to the two *mic-1* and *mic-3* genes, for their use according to claim 1, wherein said mammal is a human being or an animal.

3. Strains of *Toxoplasma gondii* having the two adhesins MIC-1 and MIC-3 inactivated by a genetic modification relating to the two *mic-1* and *mic-3* genes, for their use according to claim 2, in which said animal belongs to the group comprising or constituted by ovines, caprins, porcines, bovines, equines, camelids, canids or felids.

4. Strains of *Toxoplasma gondii* having the two adhesins MIC-1 and MIC-3 inactivated by a genetic modification relating to the two *mic-1* and *mic-3* genes, for their use according to any one of claims 1 to 3, in which said strains of *Toxoplasma gondii* are administered to the mammal at a rate from 20 to 10⁹ tachyzoites.

5. Strains of *Toxoplasma gondii* having the two adhesins MIC-1 and MIC-3 inactivated by a genetic modification relating to the two *mic-1* and *mic-3* genes, for their use according to any one of claims 1 to 4, in which said strains are in a galenic form selected from the group comprising or constituted by liquid suspensions, solid or liquid dispersions, powders, pastes or lyophilizates.

6. Strains of *Toxoplasma gondii* having the two adhesins MIC-1 and MIC-3 inactivated by a genetic modification relating to the two *mic-1* and *mic-3* genes, for their use according to any one of claims 1 to 5, in which said strains are associated with at least one other antigen, or at least one adjuvant, or at least one stabilizer, or at least one preservative or a mixture of at least two of said products enabling to increase the immune response of said mammal.
